# EUROPEAN PATENT APPLICATION

(11) **EP 4 834 889 A1**
(43) Date of publication of application: **04.11.2026**
(21) Application number: 24911511.4
(22) Date of filing: 27.12.2024
(51) Int. Cl.: C07D 295/088, C07D 295/104, C07D 295/13, A61K 45/00, A61K 9/127, A61K 47/16

(54) **ASYMMETRIC CATIONIC LIPID HAVING BRANCHED PIPERAZINE RING**

(30) Priority: 29.12.2023 CN 202311855275; 30.04.2024 CN 202410539911
(71) Applicant: Xiamen Sinopeg Biotech Co., Ltd., Xiamen, Fujian 361100 (CN)
(72) Inventor: WEI, Guohua, Xiamen, Fujian 361100 (CN); LIN, Sheng, Xiamen, Fujian 361100 (CN); WANG, Linlin, Xiamen, Fujian 361100 (CN); WANG, Ailan, Xiamen, Fujian 361100 (CN); LIN, Minggui, Xiamen, Fujian 361100 (CN); WENG, Wengui, Xiamen, Fujian 361100 (CN); LIU, Chao, Xiamen, Fujian 361100 (CN); ZHU, Qi, Xiamen, Fujian 361100 (CN)
(74) Representative: Weickmann & Weickmann PartmbB
(86) International application number: PCT/CN2024/143287
(87) International publication number: WO 2025/140595

(57) **Abstract**

The present application relates to the field of drug delivery, and provides a piperazine-ring-branched, asymmetric cationic lipid having a structure as shown in Formula (1), wherein the definitions of the symbols are consistent with those described herein. The cationic lipid of the present application incorporates biodegradable groups at appropriate positions, wherein the presence of degradable groups enables the LNP pharmaceutical compositions prepared from the cationic lipids to degrade timely within endosomes and exhibit low cytotoxicity, which addresses the issue in the prior art where LNP pharmaceutical compositions prepared from non-degradable or poorly degradable cationic lipids accumulate in endosomes, acidify the endosomal environment, and hinder the endosomal escape of drugs (e.g., mRNA), thereby preventing the delivered drugs from exerting their full effects. The piperazine-containing cationic lipid is prepared using multifunctional piperazine-based raw materials, resulting in a simple preparation process, lower cost, and greener production. The LNP-mRNA compositions prepared from it exhibit low toxicity, high biocompatibility, and high cellular transfection efficiency.

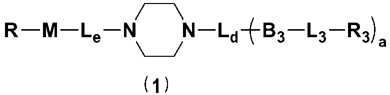

## Description

### TECHNICAL FIELD

The present application relates to the field of drug delivery, specifically relates to a cationic lipid as a pharmaceutical carrier, especially relates to a piperazine-ring-branched, asymmetric cationic lipid, as well as a lipid composition comprising the cationic lipid, a lipid pharmaceutical composition, and formulations and applications thereof.

### BACKGROUND

Nucleic acid drugs refer to a category of oligoribonucleotides (RNA) or oligodeoxyribonucleotides (DNA) with various functions, which exert their effects at the genetic level, include but are not limited to, siRNA, miRNA, antisense nucleic acids, aptamers, DNA, messenger RNA (mRNA), and the like. Wherein, mRNA is a type of single-stranded ribonucleic acid that is transcribed from one strand of DNA, carries genetic information, and can guide protein synthesis. In recent years, due to the advantages of mRNA as a drug or vaccine, such as its ability to be translated in the cytoplasm, simple production process, rapid synthesis, low cost, and suitability for large-scale production, mRNA has been widely applied in the research and development of therapies for various diseases, especially in the field of vaccines. However, mRNA has some drawbacks, such as its susceptibility to degradation, instability, and difficulty in cellular uptake. These limitations can be overcome by optimizing its chemical structure and selecting appropriate delivery systems. Currently, mRNA delivery systems are one of the most critical technologies that limit the development of mRNA-based drugs.

mRNA delivery systems mainly include viral vectors and non-viral vectors. Viral vectors carry the risk of integrating into the host genome and may elicit humoral or cellular immune responses of varying degrees, which limits their clinical use. Non-viral vectors mainly consist of liposomes, lipid nanoparticles (LNPs), inorganic nanoparticles, polymers, and proteins. Lipid nanoparticles are the most extensively studied and highly focused delivery systems at present.

Lipid nanoparticles are typically composed of cationic lipids, neutral lipids, sterol lipids, and PEGylated lipids, which are combined under appropriate conditions. Wherein, cationic lipids bind to nucleic acids through electrostatic interactions, and the cationic lipid component significantly influences the delivery performance of LNPs. Established cationic lipids in current applications include MC3, ALC-0315, SM-102, etc. However, mRNA delivery via LNP technology still faces many challenges. Different research systems may have different optimal cationic lipids and/or lipid formulation ratios. Therefore, there is still a need in this field for improved cationic lipids suitable for routine therapeutic uses.

### SUMMARY

To resolve the aforementioned problem, the present application provides a piperazine-ring-branched, asymmetric cationic lipid and its preparation method thereof, a lipid composition containing the cationic lipid, a lipid pharmaceutical composition containing the lipid composition and formulation thereof, and a liposome or LNP containing the lipid composition. Especially, the LNP-nucleic acid pharmaceutical composition containing the lipid composition and its formulations have the advantages of high delivery efficiency, safety, low toxicity, and high biocompatibility, which can improve the therapeutic and/or preventive effects of drugs.

The above-described purposes of this application can be realized via the embodiments below:
In one embodiment of the present application, provided herein is a cationic lipid:
A cationic lipid, wherein the structure is represented by the general formula (1): 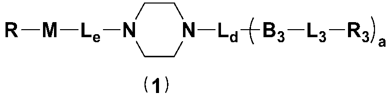
or a salt, tautomer, stereoisomer, deuterated derivative, or solvate thereof;
wherein, L_{d} is a linking bond, and a is 1;
Lₑ is a C₁₋₁₂ alkylene group;
M is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
R is 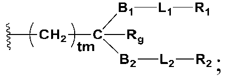
wherein, tm is an integer from 0 to 6; B₁, B₂, and B₃ are each independently a C₁₋₂₀ alkylene group; L₁, L₂, and L₃ are each independently selected from the group consisting of -CH(OH)-, -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, and -NHC(=O)S-, wherein s is 1, 2, or 3; R_{g} is H or a C₁₋₆ alkyl group; R₁, R₂, and R₃ are each independently a linear or branched C₅₋₄₀ hydrocarbon group.

The present application also provides a lipid composition, embodied as follows:
A lipid composition comprising a cationic lipid with the structure represented by the formula (1).

The present application also provides a lipid pharmaceutical composition, embodied as follows:
A lipid pharmaceutical composition comprising a lipid composition and a drug; wherein, the lipid composition contains a cationic lipid with the structure represented by the formula (1); the drug is selected from the group consisting of a nucleic acid drug, a gene vaccine, an anti-tumor drug, a small molecule drug, a peptide drug, and a protein drug.

The present application also provides a formulation of lipid pharmaceutical composition, embodied as follows:
A formulation of lipid pharmaceutical composition comprising the aforementioned lipid pharmaceutical composition and a pharmaceutically acceptable diluent or excipient.

The present application also provides a liposome or LNP, embodied as follows:
A liposome or LNP comprising a lipid composition, wherein the lipid composition contains a cationic lipid with the structure represented by formula (1).

### Compared with the prior art, the present application brings the following beneficial effects:

The asymmetric cationic lipids of the present application contain a piperazine ring branch, with 3 to 4 saturated or unsaturated hydrophobic aliphatic tail chains, enriching the variety of cationic lipid compounds and providing more selectable cationic lipids for the delivery of nucleic acid drugs, gene vaccines, small molecule drugs, peptide and protein drugs, etc.

The cationic lipids of the present application introduce a piperazine ring structure into the hydrophilic head group and contain multiple ionizable tertiary amines, which can enhance their interaction with nucleic acids, thereby enable higher nucleic acid loading.

The piperazine-ring-branched asymmetric cationic lipids of the present application introduce biodegradable groups into the hydrophobic hydrocarbon tails. The presence of these biodegradable groups allows the LNP-drug compositions prepared from the cationic lipid to degrade timely within endosomes. This addresses the issue in the prior art where LNP pharmaceutical compositions prepared from non-degradable or poorly degradable cationic lipids accumulate in endosomes, acidify the endosomal environment, and hinder the endosomal escape of drugs (e.g., mRNA), thereby preventing the delivered drugs from exerting their full effects. Moreover, under the action of intracellular enzymes, the biodegradable groups enable the cationic lipids to degrade into non-toxic metabolites, offering the advantages of high safety and low cytotoxicity.

The novel ionizable lipids provided by the present application is positively charged in acidic environments and nearly uncharged in neutral environments. In an acidic buffer system, these lipids can complex with negatively charged nucleic acid drugs through electrostatic interactions. Once entering the neutral environment of the body, the lipid-nucleic acid drug compositions (such as lipid nanoparticles) exhibit electrical neutrality, which effectively prevents the adsorption of plasma proteins onto the lipid-nucleic acid drug compositions, thereby achieving higher delivery efficiency and safety.

The cationic lipids containing a piperazine ring of the present application are prepared from heterofunctionalized piperazine-containing starting materials. The preparation process is simple, cost-effective, and more environmentally friendly. The LNP-mRNA compositions prepared from these lipids exhibit low toxicity, high biocompatibility, and high cellular transfection efficiency.

### BRIEF DESCRIPTION OF THE DRAWINGS

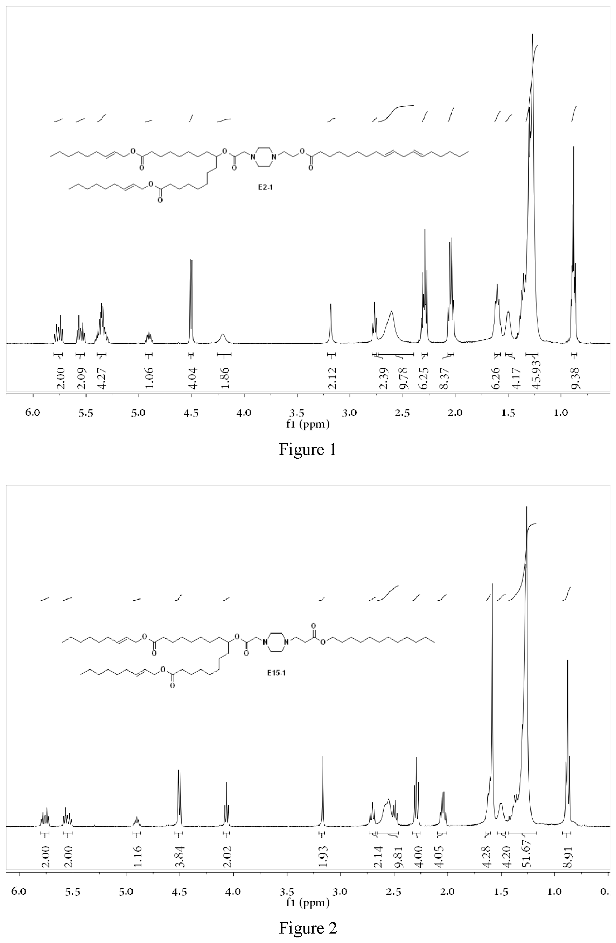

Figure 1 is the ¹H NMR spectrum of cationic lipid E2-1 prepared in Example 2.1.
Figure 2 is the ¹H NMR spectrum of cationic lipid E15-1 prepared in Example 15.1.

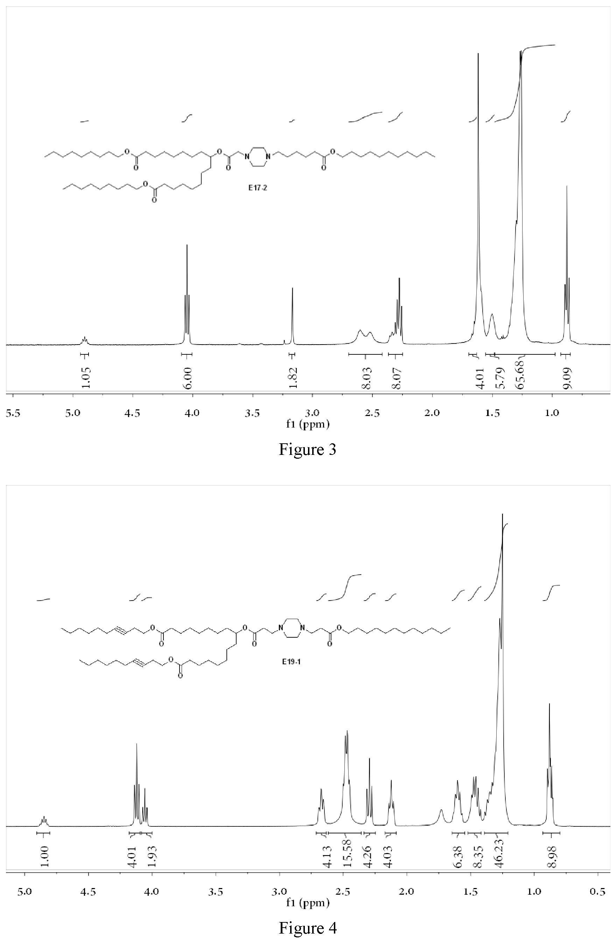

Figure 3 is the ¹H NMR spectrum of cationic lipid E17-2 prepared in Example 17.2.
Figure 4 is the ¹H NMR spectrum of cationic lipid E19-1 prepared in Example 19.

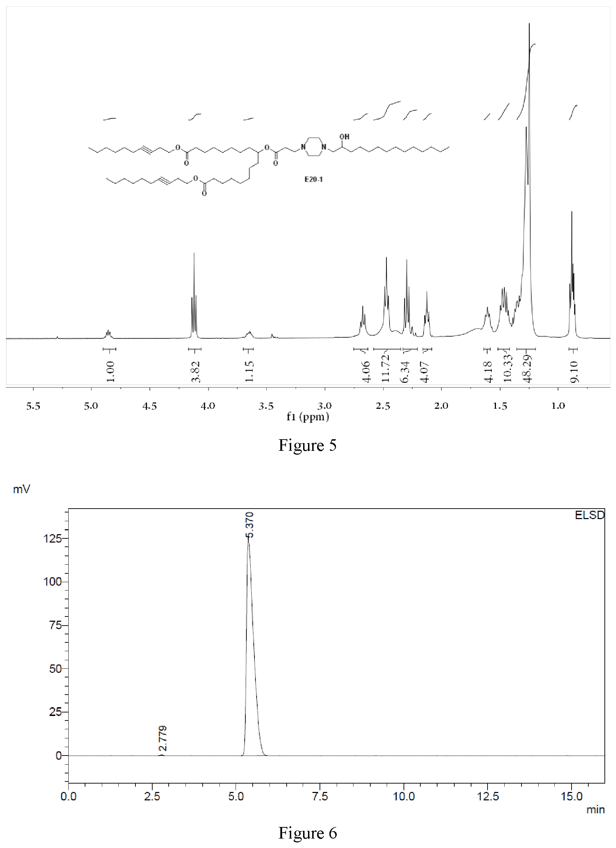

Figure 5 is the ¹H NMR spectrum of cationic lipid E20-1 prepared in Example 20.
Figure 6 is the high-performance liquid chromatography (HPLC) test results of cationic lipid E20-1 prepared in Example 20.
Figure 7 is the mass spectrometry (MS) of cationic lipid E20-1 prepared in Example 20.
Figure 8 is the cytotoxicity test results of LNP-mRNA pharmaceutical composition L-24 prepared in Example 31.
Figure 9 is the imaging results after mouse injection of LNP-mRNA pharmaceutical composition L-24 prepared in Example 31.

### DETAILED DESCRIPTION

### Description of Terms

In the present application, the terms have the following meaning, unless otherwise described.

In the present application, unless otherwise specified, when the structure involved has isomers, it can refer to any one of the isomers. For example, when cis- and trans- isomers are present, it can refer to either a cis isomer or a trans isomer; when E/Z isomers exist, the structure can be either an (E)-structure or a (Z)-structure; and when optical activity is present, it can be either a levorotatory form or a dextrorotatory form.

In the present application, a numerical interval may be indicated by a hyphen (e.g., 0-6), a tilde (e.g., 0~6), or "to" (e.g., 0 to 6, 1 to 6). In the present application, an integer interval represents a group consisting of all integers within the range, including both endpoints, unless otherwise specified. For example, the integer interval 0-6 represents the group consisting of 0, 1, 2, 3, 4, 5, and 6. The numerical interval in the present application includes, but is not limited to, ranges represented by integers, non-integers, percentages, and fractions, and includes both endpoints, unless otherwise specified.

In the present application, "about" and "approximately" generally indicate a range of ±10% for numerical values, and in some cases may be expanded to ±15%, but not more than ±20%, based on the preset values. For example, when the molar percentage of steroid lipid in the total lipids is about 40%, it is generally assumed to include cases where the molar percentage of steroid lipids ranges from 30% to 50%.

In the present application, "pharmaceutically acceptable salt" refers to a salt of this application compounds that is recognized for use in animals, and more specifically in humans. This includes salts formed from the compounds represented by formula (1) with inorganic or organic acids. Such as disclosed in S. M. Berge et al., "Pharmaceutical Salts," J. Pharm. Sci. 1977, 66, 1-19. Wherein, inorganic acids include hydrochloric acid, hydrobromic acid, hydriodic acid, sulfuric acid, phosphoric acid, or nitric acid, and the like; organic acids include formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2-(4-hydroxybenzoyl)-benzoic acid, camphoric acid, cinnamic acid, cyclopentanepropionic acid, d-glucoheptonic acid, 3-hydroxy-2-naphthoic acid, nicotinic acid, pamoic acid, pectinic acid, 3-phenylpropionic acid, picric acid, pivalic acid, 2-hydroxyethanesulfonic acid, itaconic acid, aminosulfonic acid, trifluoromethanesulfonic acid, dodecyl sulfate, ethanesulfonic acid, benzenesulfonic acid, p-toluenesulfonic acid, methanesulfonic acid, 2-naphthalenesulfonic acid, naphthalene-1,5-disulfonic acid, camphorsulfonic acid, citric acid, tartaric acid, stearic acid, lactic acid, oxalic acid, malonic acid, succinic acid, malic acid, adipic acid, alginate, maleic acid, fumaric acid, d-glucuronic acid, mandelic acid, ascorbic acid, gluconic acid, glycerophosphoric acid, aspartic acid, sulfosalicylic acid, and the like. For example, HCl (or hydrochloric acid), HBr (or hydrobromic acid solution), methanesulfonic acid, sulfuric acid, tartaric acid or fumaric acid may be used to form a pharmaceutically acceptable salt with the compounds represented by the formula (1).

In the present application, the "solvate" refers to a composition formed by the compound of formula (1) or its pharmaceutically acceptable salt with a solvent (e.g., ethanol or water). It should be understood that any solvate of the compound of formula (1) used in the treatment of diseases or conditions may provide different properties (including pharmacokinetic properties), but once absorbed into the subject, the compound of formula (1) is obtained, thereby covering the use of the compound of formula (1) and any solvate thereof. It should be further understood that the compound of formula (1) or its pharmaceutically acceptable salt may be isolated in the form of a solvate, and therefore any of the said solvates are included within the scope of this invention. For example, the compound of formula (1) or its pharmaceutically acceptable salt may exist in both non-solvated form and solvated form with a pharmaceutically acceptable solvent (such as water, ethanol, etc.).

In the present application, unless otherwise specified, the term "include", "contain", "comprise", and similar expressions shall be interpreted as "including but not limited to" in the specification and claims, in an open and inclusive manner.

In the present application, when two or more objects are "each independently preferably" selected from multiple levels of preferable options, the objects are not necessarily selected from preferable options of the same level. It is allowed that one is selected from a wider range of options while another one is selected from a narrower range of options. It is also allowed that one is selected from the maximum range of options while another is selected from any allowable options. It is also allowed that the objects are selected from preferable options of the same level.

In the present application and unless otherwise specified, a divalent linking group, e.g., a hydrocarbylene group, an alkylene group, an arylene group, an amide bond, and the like, can use either one of the two linking ends to connect to another group. For example, when an amide bond serves as a divalent linking group between C-CH₂CH₂- and -CH₂-D, both C-CH₂CH₂-C(=O)NH-CH₂-D and C-CH₂CH₂-NHC(=O)-CH₂-D are allowable.

In the present application, when distinguishing the terminus from the substituents of a linking group becomes questionable, " 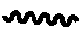 " is used to indicate the connection location between the linking group and the other groups. For example, in structural formulas 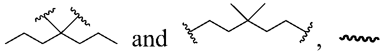 is used to indicate the connection locations between the divalent linking groups and the other groups; the two structural formulas mentioned above represent -CH(CH₂CH₂CH₃)₂- and -CH₂CH₂CH(CH₃)₂-CH₂CH₂-, respectively.

In the present application, a numerical interval written in the subscript of "C" indicates the number of carbon atoms of a group. For example, C₁₋₁₂ indicates "having 1 to 12 carbon atoms"; C₁₋₃₀ indicates "having 1 to 30 carbon atoms". The "substituted C₁₋₁₂ alkyl group" refers to a C₁₋₁₂ alkyl group with one or more hydrogen atoms being replaced. The "C₁₋₁₂ substituted alkyl group" refers to an alkyl group with one or more hydrogen atoms being replaced, with 1 to 12 carbon atoms remaining. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁, and C₁₂ alkylene groups. In the present application and unless otherwise specified, a subscript being a numerical interval indicates that the subscript can be any integer within the interval which includes two endpoints.

In the present application, the term "group" may be referred to as "bond" without changing the meaning. For example, an ether group (-O-) can also be termed an ether bond, and an ester group (-OC(=O)- or -C(=O)O-) can also be termed an ester bond.

In the present application, the "carbon chain length" between two groups refers to the minimum number of carbon atoms involved in the pathway from one group to another, excluding the groups themselves. For example, in the structures -OC(=O)CH₂CH₂OC(=O)- and -OC(=O)CH(CH₃)CH₂OC(=O)-, the carbon chain lengths between the two ester bonds are both 2 (also referred to as C2); the carbon chain length between the two ester bonds of -OC(=O)CH₂CH₂OCH₂CH₂OC(=O)- is C4 (also referred to as C4).

In the present application, heteroatoms are not particularly limited, including but not limited to O, S, N, P, Si, F, Cl, Br, I, B, etc.

In the present application, the heteroatom used for substitution is referred to as a "substituent atom", and the group used for substitution is referred to as a "substituent group".

In the present application, the term "substituted" indicates that at least one hydrogen atom of the described group (e.g., aliphatic hydrocarbon, hydrocarbon, alkyl, or alkylene group) is replaced by a bond connected to a non-hydrogen atom. The non-hydrogen atom include but are not limited to halogen atoms such as F, Cl, Br, and I, oxo group (=O), hydroxyl group (-OH), hydrocarbonoxy groups (-OR_{d}; wherein, R_{d} is a C₁₋₁₂ alkyl group), carboxyl group (-COOH), amine groups (-NR_{c}R_{c}; wherein, both R_{c} are each independently H or a C₁₋₁₂ alkyl group), C₁₋₁₂ alkyl groups, and cycloalkyl groups. In some embodiments, the substituent is a C₁₋₁₂ alkyl group. In other embodiments, the substituent is a cycloalkyl group. In other embodiments, the substituent is a halogen atom, such as fluorine atom. In other embodiments, the substituent is an oxo group. In other embodiments, the substituent is a hydroxyl group. In other embodiments, the substituent is an alkoxy group. In other embodiments, the substituent is a carboxyl group. In other embodiments, the substituent is an amine group.

In the present application, "optional" or "optionally" (e.g., optionally substituted) means that the event of the situation described thereafter may or may not occur, and the description includes instances in which the event or situation occurs as well as instances in which the event or situation does not occur. For example, "optionally substituted hydrocarbon groups" means that the hydrocarbon groups may or may not be substituted, and the description includes both substituted and unsubstituted hydrocarbon groups.

In the present application, a compound or a group can be substituted and heteroatom-incorporated simultaneously, e.g., by replacing a hydrogen atom with a nitrophenyl group, or by replacing -CH₂-CH₂-CH₂- with -CH₂-S-CH(CH₃)-. In the present application, a "linking bond" is only for connecting and does not contain any atoms. When a group is defined as including a linking bond, the group is optionally absent.

In the present application, "each independently at each occurrence" not only means that different groups can be each independently selected from the definitions, but also means that the same group at different positions can be independently selected from the definitions. For example, in -NR^{c}C(=O)NR^{c}-, both R^{c} can be the same or different, which are each independently a hydrogen atom or a C₁₋₁₂ alkyl group.

In the present application, a "group" contains at least one atom, referring to the radical formed by a compound losing one or more atoms. Relative to a compound, the remaining group formed by the removal of other groups is also referred to as the "residue". The valence of groups is not particularly limited, and examples include a monovalent group, a divalent group, a trivalent group, a tetravalent group, ..., a hectovalent group, etc. Wherein, groups with valence being equal to or greater than two are collectively defined as linking groups. A linking group may also contain only one atom, such as ether group or thioether group.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of the type of hydrocarbon groups. Hydrocarbons that do not contain phenyl rings or hydrocarbyl-substituted phenyl rings are defined as aliphatic hydrocarbons. Hydrocarbons that contain at least one phenyl ring or hydrocarbyl-substituted phenyl ring are defined as aromatic hydrocarbons. An aromatic hydrocarbon may contain aliphatic hydrocarbon structures, such as toluene, diphenylmethane, 2,3-dihydroindene, etc.

In the present application, hydrocarbons are classified into saturated hydrocarbons and unsaturated hydrocarbons in terms of the degree of saturation. All aromatic hydrocarbons are unsaturated hydrocarbons. Saturated aliphatic hydrocarbons are also referred to as alkanes. There is no specific limit to the degree of unsaturation of unsaturated aliphatic hydrocarbons. For example, unsaturated aliphatic hydrocarbons may include, but are not limited to, alkenes (containing carbon-carbon double bonds), alkynes (containing carbon-carbon triple bonds), and dienes (containing conjugated carbon-carbon double bonds), etc. When the aliphatic moieties of aromatic hydrocarbons are saturated, the aromatic hydrocarbons are also termed aralkanes, such as toluene.

In the present application, the structures of hydrocarbons are not particularly limited and may include, for example, linear structures without pendant groups, branched structures with pendant groups, ring-containing structures, dendritic structures, comb-like structures, and hyperbranched structures, etc. Unless otherwise specified, preferable structures include linear structures without pendant groups, branched structures with pendant groups, and ring-containing structures, corresponding to linear hydrocarbons, branched hydrocarbons and cyclic hydrocarbons, respectively. Wherein, hydrocarbons that contain no rings are termed open-chain hydrocarbons, including but not limited to linear structures without pendant groups, and branched structures with pendant groups. Open-chain hydrocarbons belong to aliphatic hydrocarbons. Therefore, linear hydrocarbons are also referred to as linear aliphatic hydrocarbons. Branched hydrocarbons are also referred to as branched aliphatic hydrocarbons.

In the present application, compounds in which any carbon atom in a hydrocarbon is substituted by a heteroatom are collectively referred to as heterohydrocarbons.

In the present application, "hydrocarbon group" refers to a residue formed by removing at least one hydrogen atom from a hydrocarbon molecule. Depending on the number of hydrogen atoms removed, hydrocarbon groups can be classified into monovalent hydrocarbon groups (with one hydrogen atom removed), divalent hydrocarbon groups (with two hydrogen atoms removed, also referred to as hydrocarbylene groups), trivalent hydrocarbon groups (with three hydrogen atoms removed), and the like. Accordingly, when n hydrogen atoms are lost, the valence of the formed hydrocarbon group is n. Unless otherwise specified, hydrocarbon groups in the present application specifically refer to monovalent hydrocarbon groups. Unless otherwise expressly stated in this specification, a hydrocarbon group is optionally substituted.

In the present application, the source of hydrocarbon group is not particularly limited and may include, for example, from aliphatic hydrocarbons or aromatic hydrocarbons, from saturated hydrocarbons or unsaturated hydrocarbons, from linear hydrocarbons, branched hydrocarbons or cyclic hydrocarbons, and from hydrocarbons or heterohydrocarbons, etc. According to the degree of saturation, hydrocarbon groups can be derived from alkanes, alkenes, alkynes, dienes, etc. With respect to cyclic hydrocarbons, hydrocarbon groups can be derived from alicyclic hydrocarbons or aromatic hydrocarbons, or from monocyclic hydrocarbons or polycyclic hydrocarbons. With respect to heterocyclic hydrocarbons, hydrocarbon groups can be derived from aliphatic heterocyclic hydrocarbons or aromatic heterocyclic hydrocarbons.

In the present application, "aliphatic hydrocarbon group" refers to the residue formed by removing at least one hydrogen atom from an aliphatic hydrocarbon molecule. Unless otherwise specified, aliphatic hydrocarbon groups refer to monovalent hydrocarbon groups in the present application. Aliphatic hydrocarbon groups include both saturated and unsaturated aliphatic hydrocarbon groups. Unless otherwise expressly stated in this specification, aliphatic hydrocarbon groups are optionally substituted.

In the present application, "alkyl group" refers to a hydrocarbon group obtained from an alkane by removing a hydrogen atom at any position. Unless otherwise specified, the alkyl group can be linear or branched, and substituted or unsubstituted. Specifically, a propyl group refers to either a 1-propyl group or an isopropyl group, and a propylene group refers to a 1,3-propylene group, a 1,2-propylene group, or an isopropylidene group. Unless otherwise specified in this specification, alkyl groups are optionally substituted.

In the present application, "unsaturated hydrocarbon group" refers to the hydrocarbon group obtained from an unsaturated hydrocarbon by removing hydrogen atoms. The unsaturated hydrocarbon obtained by removing hydrogen atoms from unsaturated carbon atoms can be classified into alkenyl groups, alkynyl groups, dienyl groups, and the like.

In the present application, "alkenyl group" refers to a linear or branched-chain hydrocarbon group containing at least one carbon-carbon double bond, formed by removing hydrogen atoms from any position of an alkene. For example, "C₂₋₁₅ alkenyl group" refers to a linear or branched alkenyl group containing 2 to 15 carbon atoms and at least one carbon-carbon double bond, i.e., one, two, three, four, or more carbon-carbon double bonds. Unless otherwise specified, the alkenyl groups include substituted and unsubstituted alkenyl groups in the present application. Unless otherwise expressly stated in this specification, alkenyl groups are optionally substituted.

In the present application, "alkynyl group" refers to a linear or branched-chain hydrocarbon group containing at least one carbon-carbon triple bond, formed by losing hydrogen atoms from any position of an alkyne. For example, "C₂₋₁₅ alkynyl group" refers to a linear or branched alkynyl group containing 2 to 15 carbon atoms and at least one carbon-carbon triple bond. An alkynyl group can contain one, two, three, four, or more carbon-carbon triple bonds. Unless otherwise specified, alkynyl groups include substituted and unsubstituted alkynyl groups in the present application. Unless otherwise expressly stated in this specification, alkynyl groups are optionally substituted.

In the present application, "hydrocarbylene group" or "hydrocarbylene chain" refers to a linear or branched divalent hydrocarbon chain that connects the remainder of a molecule to free radicals, consisting only of carbon and hydrogen, which is saturated or unsaturated. Examples include, a hydrocarbylene group with one to twenty-four carbon atoms (a C₁₋₂₄ hydrocarbylene group), a hydrocarbylene group with one to twelve carbon atoms (a C₁₋₁₂ hydrocarbylene group), and specifically, a methylene group, an ethylene group, a propylene group, an *n*-butylene group, a vinylidene group, a propenylene group, an *n*-butenylene group, a propynylene group, an *n*-butynylene group, etc. Unless otherwise explicitly stated in this specification, hydrocarbylene groups are optionally substituted.

In the present application, an "alkylene group" is also a divalent alkyl group, including open-chain alkylene groups and divalent cycloalkyl groups. The open-chain alkylene group refers to a divalent alkyl group without a cyclic structure, and the divalent cycloalkyl group refers to a divalent alkyl group containing a cyclic structure. Unless otherwise expressly stated in this specification, alkylene groups are optionally substituted.

In the present application, the aliphatic hydrocarbon derivative is preferably an ether-derivatized aliphatic hydrocarbon, more preferably an aliphatic hydrocarbon derivative containing 1 to 2 ether bonds, and most preferably an aliphatic hydrocarbon derivative containing 2 ether bonds.

In the present application, "molecular weight" represents the mass of a compound, and "average molecular weight" represents the mass of a compound component of a general formula in macroscopic matter. Unless otherwise specified, the molecular weight of polymers refers to the "average molecular weight", which generally refers to the "number average molecular weight" (Mₙ). The number average molecular weight can be used to describe the molecular weight of either polydisperse blocks or substances, or monodisperse blocks or substances. Unless otherwise specified, the measuring unit of "molecular weight" and "average molecular weight" is Dalton (Da). Polymers can be characterized by the "degree of polymerization", which indicates the number of repeating units (e.g., oxyethylene units of PEG, EO-units) in a molecule. Accordingly, "average degree of polymerization", "number average degree of polymerization" or "number of EO units" represents the average value or the number average value of the number of repeating units, and unless otherwise specified, each refers to the number average degree of polymerization.

In the present application, with respect to percentages, "about" generally refers to a range of ±0.5%.

In the present application, the terms "stable" and "degradable" are a pair of opposing concepts. Detailed examples of stable groups and degradable groups are given in paragraphs [0134] to [0145] of CN113402405A.

In the present application, "hydroxyl protecting group" includes all the groups that can be used as common hydroxyl protecting groups. A hydroxyl protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a *t*-butyldimethylsilyl group, an allyl group, an acetal group, and a ketal group. Removal of the acetyl group is generally carried out under basic conditions, most commonly by the ammonolysis with NH₃/MeOH or by the methanol anion-catalyzed methanolysis. The benzyl group can be easily removed via palladium-catalyzed hydrogenolysis in a neutral solution at room temperature, or via a reduction reaction by metallic sodium in ethanol or liquid ammonia. The triphenylmethyl group is generally removed via catalytic hydrogenolysis. The trimethylsilyl group is generally removed using reagents containing fluoride ions (e.g., tetrabutylammonium fluoride/anhydrous THF, etc.). The *t*-butyldimethylsilyl ether is relatively stable and can withstand ester hydrolysis conditions with alcoholic potassium hydroxide and mild reduction conditions (e.g., Zn/CH₃OH and the like), which can be removed by fluoride ions (e.g., Bu₄N⁺F⁻) in THF solution or by aqueous acetic acid at room temperature.

In the present application, "carboxyl protecting group" refers to the protecting group which can be transformed into a carboxyl group via the hydrolysis or the deprotection reaction of a carboxyl protecting group itself. A carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me) and an ethyl group (Et).

In the present application, "protected carboxyl group" refers to the group protected by an appropriate carboxyl protecting group, preferably selected from the group consisting of a methoxycarbonyl group, an ethoxycarbonyl group, t-butoxycarbonyl group, and a benzyloxycarbonyl group. The carboxyl protecting groups can be removed through hydrolysis catalyzed by acids or bases, or through pyrolysis reactions occasionally. For example, t-butyl groups can be removed under mild acidic conditions, and benzyl groups can be removed by hydrogenolysis. The reagent used for the removal of carboxyl protecting groups is selected from the group consisting of TFA, H₂O, LiOH, NaOH, KOH, MeOH, EtOH and combinations thereof, preferably selected from the group consisting of the combination of TFA and H₂O, the combination of LiOH and MeOH, and the combination of LiOH and EtOH. A protected carboxyl group can undergo deprotection to produce the corresponding free acid, wherein the deprotection is conducted in the presence of a base, and the base forms a pharmaceutically acceptable salt with the free acid produced via the deprotection.

In the present application, "amino protecting group" includes all the groups which are used as amino protecting groups generally, such as an aryl C₁₋₆ alkyl group, a C₁₋₆ alkoxy group, a C₁₋₆ alkyl group, a C₁₋₆ alkoxycarbonyl group, an aryloxycarbonyl group, a C₁₋₆ alkylsulfonyl group, an arylsulfonyl group, a methylsilyl group, etc. An amino protecting group is preferably selected from the group consisting of a t-butoxycarbonyl group (Boc), a p-methoxybenzyloxycarbonyl group (Moz), and a 9-fluorenylmethyloxycarbonyl (Fmoc). The reagent used for the removal of amino protecting groups is selected from the group consisting of TFA, H₂O, LiOH, MeOH, EtOH, and combinations thereof, preferably selected from the group consisting of the combination of TFA and H₂O, the combination of LiOH and MeOH, and the combination of LiOH and EtOH. The Boc protecting group can be removed using TFA or HCl/EA, preferably TFA. The Fmoc protecting group can be removed using the N,N-dimethylformamide (DMF) solution containing 20% piperidine.

In the present application, "cationic lipid" refers to a lipid having a positive charge or being ionizable. Wherein, "cation" refers to the corresponding structure bearing a positive charge, either permanently, or non-permanently but in response to certain conditions (such as pH). Therefore, the cations include permanent cations and cationizable substances. Permanent cations refer to the corresponding compounds, groups, or atoms that bear positive charges under conditions of any pH value or hydrogen ion activity of their environment. Typically, a positive charge is generated by the presence of quaternary nitrogen atom. When a compound carries multiple such positive charges, it can be termed a permanent cation. A cationizable substance refers to a compound, group, or atom that is positively charged at a lower pH and uncharged at a higher pH of its environment. Moreover, in non-aqueous environments where pH cannot be determined, a cationizable compound, group, or atom is positively charged at high hydrogen ion concentration and uncharged at low concentration or activity of hydrogen ions. It depends on the individual properties of the cationizable or polycationizable compound, in particular the pKa of the corresponding cationizable group or atom, at which pH or hydrogen ion concentration said compound is charged or uncharged. In the diluted aqueous environment, the Henderson-Hasselbalch equation can be used to estimate the fraction of positively charged cationizable compounds, groups, or atoms, which is well-known to those skilled in the art. For example, in some embodiments, if a compound or moiety is cationizable, then, preferably, it is positively charged at a pH of about 1 to 9, preferably 4 to 9, 5 to 8, or even 6 to 8, more preferably equal to or below 9, equal to or below 8, equal to or below 7, and most preferably at physiological pH (e.g., about 7.3 to 7.4), i.e., under physiological conditions, especially under physiological conditions of cells in vivo. In other embodiments, it is preferable that the cationizable compound or moiety is predominantly neutral at physiological pH (e.g., about 7.0 to 7.4), but becomes positively charged at lower pH values. In some embodiments, pKa of the cationizable compound or moiety is preferably about 5 to about 7.

In the present application, LNPs, cationic peptides, proteins, polysaccharides, lipids and polymers are uncharged, having a neutral charge or being respectively electrically neutral under physiological conditions, especially under the physiological conditions of cells in vivo. Cationic peptides or proteins preferably contain a larger amount of cationic amino acids, such as a greater amount of Arg, His, Lys, or Orn compared to other amino acid residues (especially more cationic amino acids than anionic amino acid residues such as Asp or Glu), or contain components formed primarily by cationic amino acid residues. The term "cationic" can also refer to "polycationic" components/compounds, and can also refer to cationic lipids that are capable of being positively charged. Examples include, cationic lipids contain one or more amine groups with positive charges, and preferably cationic lipids that are ionizable to exist in either a positively charged or neutral form depending on pH. The ionization of cationic lipids affects the surface charge of LNPs at different pH conditions. This charge state can affect plasma protein absorption, blood clearance, and tissue distribution, as well as the ability to form non-bilayer structures that are essential for the intracellular delivery of nucleic acids.

In the present application, "PEGylated lipid" refers to the molecules containing both lipid and PEG moieties.

In the present application, "neutral lipid" refers to any lipid substance that is uncharged or exists in the form of neutral zwitterion at the chosen pH, preferably phospholipid. The neutral lipid can be synthetic or natural.

In the present application, "steroid lipid" refers to a steroid or steroid analog.

In the present application, a variant form refers to a structure that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present application, "variant form of reactive group" refers to a reactive form that retains its reactivity (remains a reactive group) after undergoing at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc.; alternatively, it may refer to a non-reactive form of protected reactive group.

In the present application, "micro-modification" refers to a chemical modification process that can be completed through simple chemical reactions. The simple chemical reactions mainly include deprotection, salt complexation and decomplexation, ionization, protonation, deprotonation, and transformation of leaving groups. The "micro-modified form" corresponds to "micro-modification" and refers to the structural form that can form the target reactive group after undergoing simple chemical reactions such as deprotection, salt complexation and decomplexation, ionization, protonation, deprotonation, and transformation of leaving groups. For example, the transformation of a leaving group can involve the conversion from an ester form to an acyl chloride form.

In the present application, "N/P ratio" refers to molar ratio of nitrogen atoms in cationic lipids to phosphates in nucleic acids.

In the present application, "nucleic acid" refers to DNA, RNA, or their modified form.

In the present application, "RNA" refers to ribonucleic acid that may be naturally or non-naturally occurring. For example, RNA may include modified and/or non-naturally occurring components such as one or more nucleobases, nucleosides, nucleotides, or linkers. An RNA may include a cap structure, a chain-terminating nucleoside, a stem-loop, a polyA sequence, and/or a polyadenylation signal. An RNA can be a nucleotide sequence encoding a specific polypeptide, or can be a messenger RNA (mRNA). Translation of mRNA encoding a particular polypeptide, for example, in vivo translation of an mRNA inside a mammalian cell, may produce the encoded polypeptide. RNAs may be selected from the group consisting of small interfering RNA (siRNA), asymmetrical interfering RNA (aiRNA), microRNA (miRNA), dicer-substrate RNA (dsRNA), small hairpin RNA (shRNA), messenger RNA (mRNA), single guide RNA (sgRNA), self-amplifying RNA (saRNA), circular RNA (circRNA), Cas9 mRNA, and mixtures thereof.

In the present application, FLuc mRNA can express luciferase protein, which emits bioluminescence in the presence of luciferin substrate, so FLuc is commonly used in mammalian cell culture to measure gene expression and cell viability.

In the present application, methods for determining the expression level of target genes include but are not limited to dot blots, northern blots, in situ hybridization, ELISA, immunoprecipitation, enzymatic function, and phenotypic assays.

In the present application, "transfection" refers to the introduction of a species (e.g., an RNA) into cells. Transfection may occur, such as, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the present application, the antigen may be a translation product of the provided nucleic acid molecule (preferably mRNA as defined herein). In this context, fragments, variants, and derivatives of peptides and proteins containing at least one epitope are also understood as antigens.

In the present application, "delivery" refers to delivering an entity to the target, for example, delivering drugs and/or therapeutic agents and/or prophylactic agents to subjects, wherein the subjects are tissues and/or cells of humans and/or other animals.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including oils from petroleum, animals, vegetables, or synthesis, e.g., peanut oil, soybean oil, mineral oil, sesame oil, etc. When the pharmaceutical composition is administered intravenously, water is an exemplary carrier. Physiological saline, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral preparations can contain standard carriers, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and water for hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present application, the pharmaceutical compositions can act systemically and/or locally. For this purpose, they can be administered via appropriate routes, such as by injection (e.g., intravenous, intra-arterial, subcutaneous, intraperitoneal, intramuscular, including infusion) or transdermal administration; or be administered by oral, buccal, nasal, transmucosal, topical, ophthalmic formulations, or inhalation. For these routes of administration, the pharmaceutical compositions of this invention can be provided in suitable dosage forms. These dosage forms include, but are not limited to, tablets, capsules, lozenges, troches, powders, sprays, creams, ointments, suppositories, gels, pastes, lotions, soft ointments, aqueous suspensions, injectable solutions, elixirs, and syrups.

In the present application, "vaccines" are preventive or therapeutic materials that provide at least one antigen or antigenic function. Antigen or antigenic function can stimulate the body's adaptive immune system to provide an adaptive immune response.

In the present application, "treatment" refers to the management and care of patients to combat diseases, obstacles, or symptoms, intended to delay the progression of diseases, obstacles, or conditions, reduce or alleviate symptoms and complications, and/or cure or eliminate diseases, disorders, or conditions. The patients to be treated are preferably mammals, particularly humans.

### Detailed Description of the Application:

### 1. Cationic Lipids

An embodiment of the present application:
A cationic lipid, wherein the structure is represented by the general formula (1): 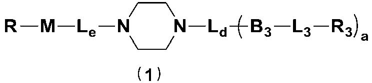
or a salt, tautomer, stereoisomer, deuterated derivative, or solvate thereof;
wherein, L_{d} is a linking bond, and a is 1;
Lₑ is a C₁₋₁₂ alkylene group;
M is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
R is 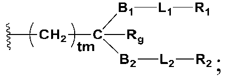
wherein, tm is an integer from 0 to 6; B₁, B₂, and B₃ are each independently a C₁₋₂₀ alkylene group; L₁, L₂, and L₃ are each independently selected from the group consisting of -CH(OH)-, -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, and -NHC(=O)S-, wherein s is 1, 2, or 3; R_{g} is H or a C₁₋₆ alkyl group; R₁, R₂, and R₃ are each independently a linear or branched C₅₋₄₀ hydrocarbon group.

### 1.1. B₁, B₂, B₃, Lₑ

In the present application, B₁, B₂, and B₃ are each independently selected from the group consisting of a methylene group, an ethylene group, a propylene group, a butylene group, a pentylene group, a hexylene group, and a heptylene group.

In a specific embodiment of the present application, Lₑ is preferably a C₃₋₁₂ alkylene group.

In a specific embodiment of the present application, B₃ is preferably a C₃₋₂₀ alkylene group.

In a specific embodiment of the present application, Lₑ is preferably a C₃₋₁₂ alkylene group and B₃ is a C₃₋₂₀ alkylene group.

### 1.2. L₁, L₂, L₃

In a specific embodiment of the present application, the L₁, L₂, and L₃ are each independently selected from the group consisting of -CH(OH)-, -OC(=O)-, -C(=O)O-, -C(=O)NH-, -NHC(=O)-, -NHC(=O)O-, and -OC(=O)NH-.

### 1.3. R₁, R₂, R₃

In the present application, R₁, R₂, and R₃ are each independently a linear or branched C₅₋₄₀ hydrocarbon group, preferably an alkyl group, an alkenyl group, or an alkynyl group.

In the present application, the linear C₅₋₄₀ hydrocarbon group is a C₅₋₄₀ linear alkyl group, a C₅₋₄₀ linear alkenyl group, or a C₅₋₃₀ linear alkynyl group; preferably each independently a C₅₋₂₅ linear alkyl group, a C₅₋₂₅ linear alkenyl group, or a C₅₋₂₅ linear alkynyl group; the linear C₅₋₄₀ hydrocarbon group is more preferably selected from the group consisting of the following structures:

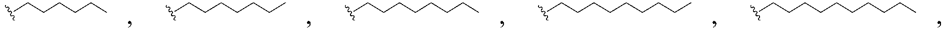

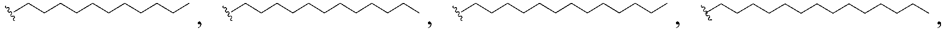

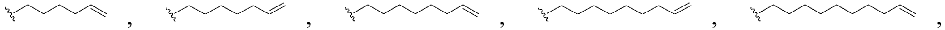

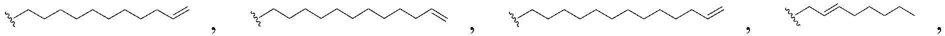

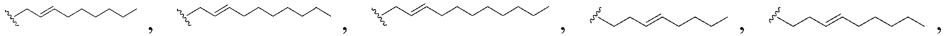

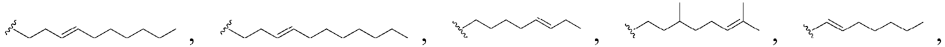

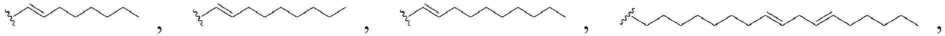

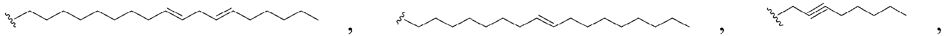

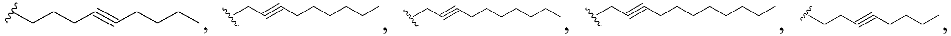

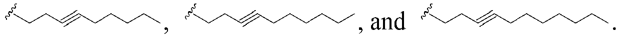

In a specific embodiment of the present application, the branched C₅₋₄₀ hydrocarbon group is a C₅₋₄₀ branched alkyl group, a C₅₋₄₀ branched alkenyl group, or a C₅₋₄₀ branched alkynyl group, each independently represented by 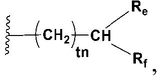 wherein, tn is an integer from 0 to 12; Rₑ and R_{f} are each independently selected from the group consisting of a C₁₋₁₅ alkyl group, a C₂₋₂₀ alkenyl group, and a C₂₋₁₅ alkynyl group; more preferably, Rₑ and R_{f} are each independently selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a pentenyl group, a hexenyl group, a heptenyl group, an octenyl group, a nonenyl group, a decenyl group, a octadecane-6,9-dienyl group, and substituted forms thereof; the branched C₅₋₄₀ hydrocarbon group is most preferably selected from the group consisting of the following structures: 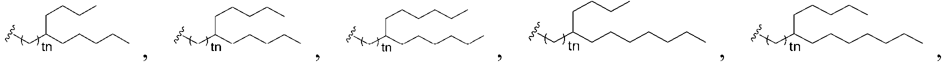 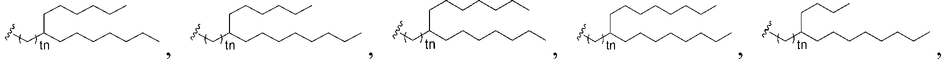 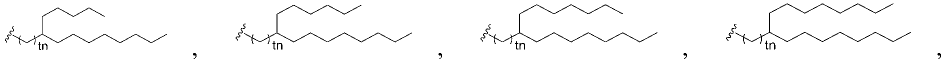 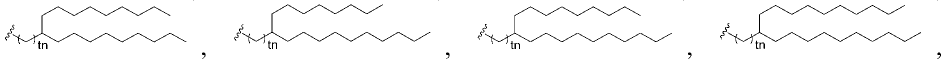 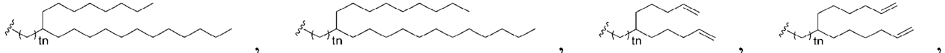 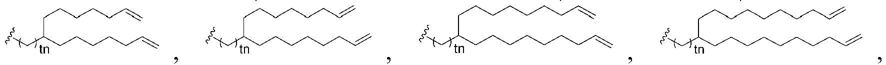 and

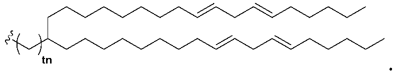

In a specific embodiment of the present application, R₁, R₂, and R₃ are preferably selected from any of the following cases:
Case (1): R₁, R₂, and R₃ are all linear C₅₋₄₀ hydrocarbon groups;
Case (2): R₁, R₂, and R₃ are all branched C₅₋₄₀ hydrocarbon groups;
Case (3): any one of R₁, R₂, and R₃ is a linear C₅₋₄₀ hydrocarbon group, and the other two are branched C₅₋₄₀ hydrocarbon groups;
Case (4): any two of R₁, R₂, and R₃ are linear C₅₋₄₀ hydrocarbon groups, and the other is a branched C₅₋₄₀ hydrocarbon group; preferably, R₁ and R₂ are linear C₅₋₄₀ hydrocarbon groups, and R₃ is branched C_{S-40} hydrocarbon group.

### 1.4. Fragment -B₃-L₃-R₃

In a specific embodiment of the present application, the fragments -B₃-L₃-R₃ is independently selected from the group consisting of the following structures:

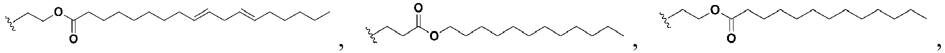

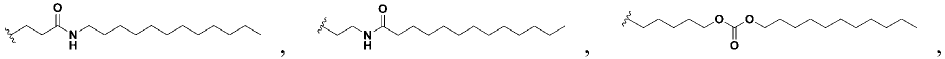

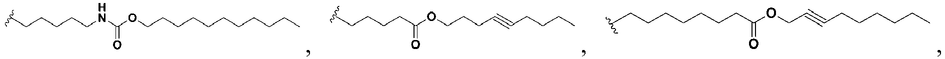

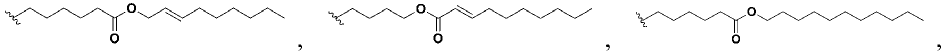

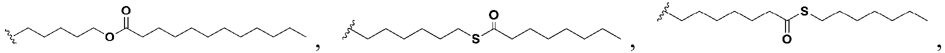

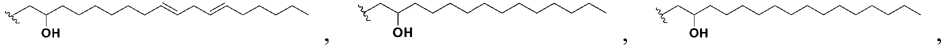

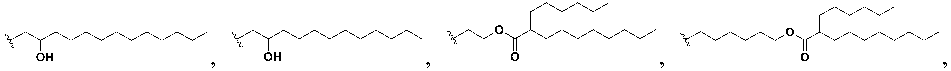

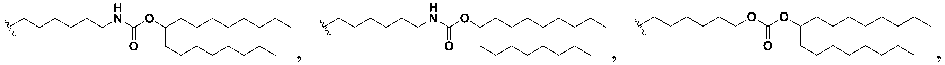

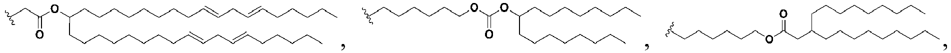

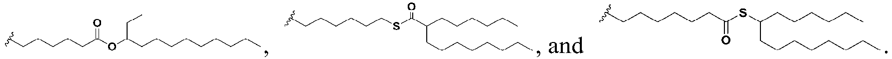

### 1.5. R

In a specific embodiment of the present application, the R is independently selected from the group consisting of the following structures:

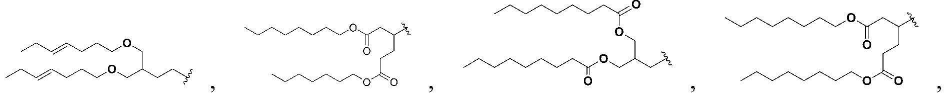

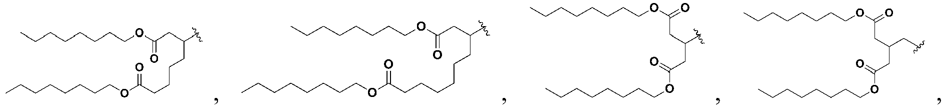

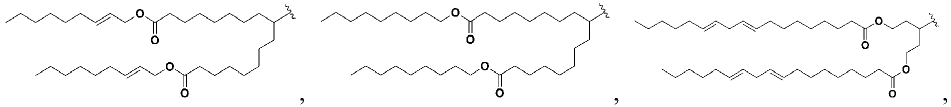

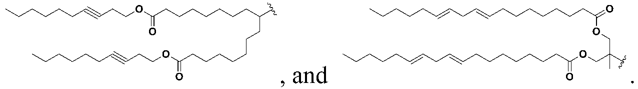

### 1.6. Examples of general formulas of structures

In a specific embodiment of the present application, the M is selected from the group consisting of -OC(=O)-, -C(=O)O-, -OC(=O)O-, -NHC(=O)-, -C(=O)NH-, -OC(=O)NH-, and -NHC(=O)O-; the definitions of other symbols are the same as those in formula (1).

In a specific embodiment of the present application, the aforementioned cationic lipid preferably satisfies any one of the following general formulas: 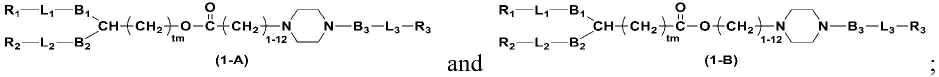 more preferably, the cationic lipid satisfies any one of the following general formulas: 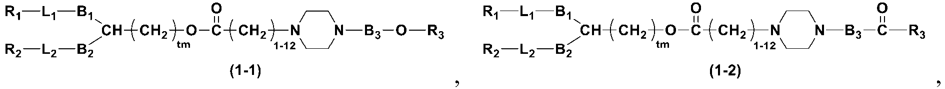 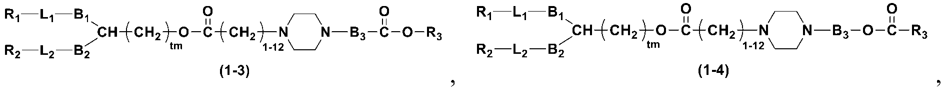 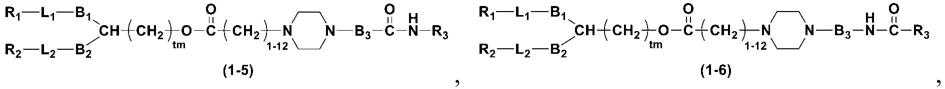 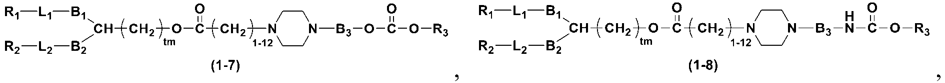 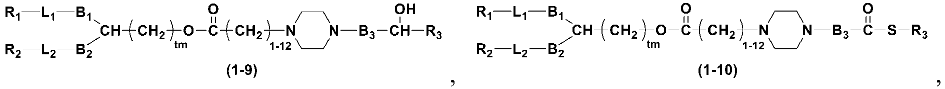 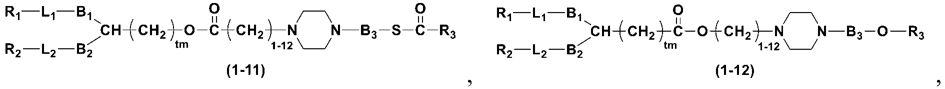 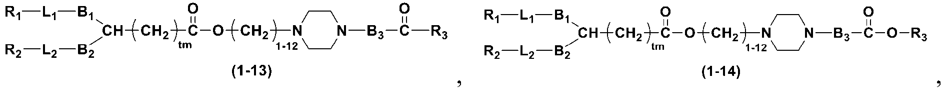 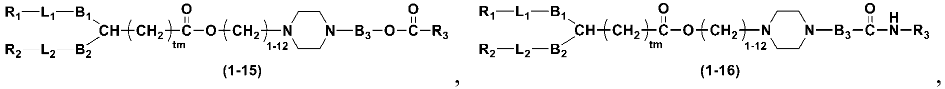 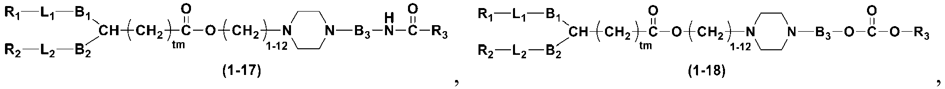 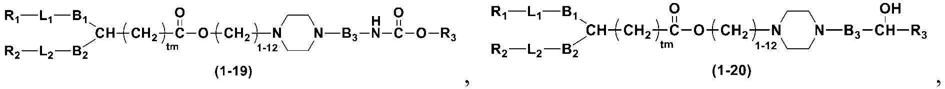 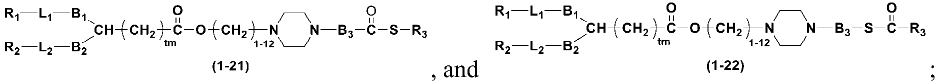 preferably, in the aforementioned general formulas (1-1) to (1-22), B₁ and B₂ are the same, and each a C₁₋₁₂ alkylene group; L₁ and L₂ are the same and each selected from the group consisting of -OC(=O)-, -C(=O)O-, -NHC(=O)-, -C(=O)NH-, and -OC(=O)O-; R₁ and R₂ are the same, and each a linear C₅₋₄₀ hydrocarbon group; R₃ is a linear C₅₋₄₀ hydrocarbon group.

### 1.7. Examples of specific structures

In a specific embodiment of the present application, the cationic lipid is selected from the group consisting of the following structures: 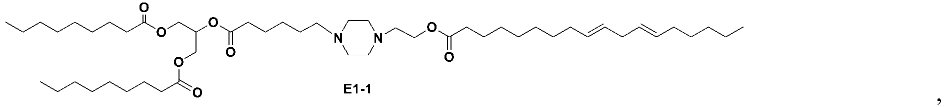 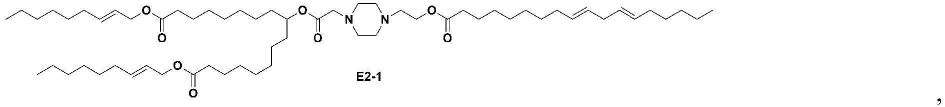 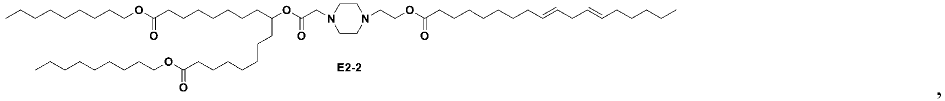 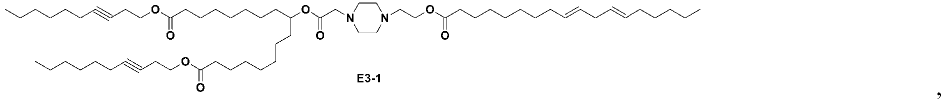 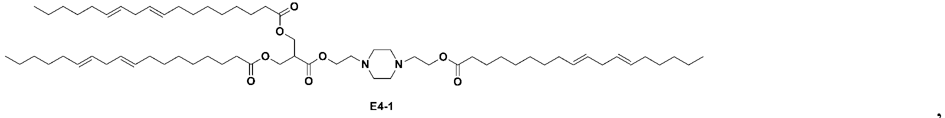 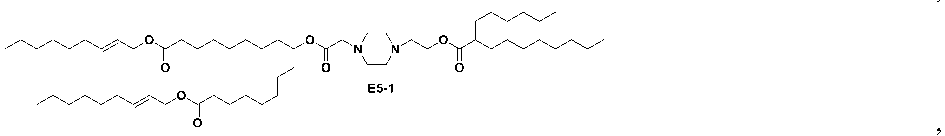 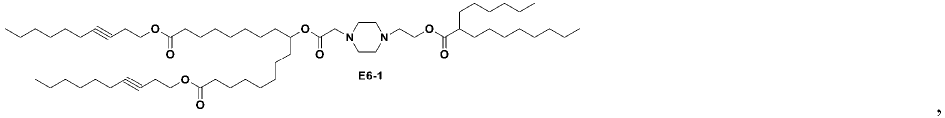 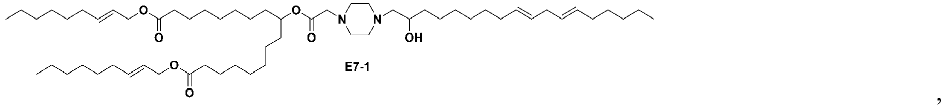 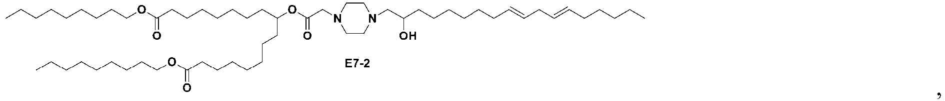 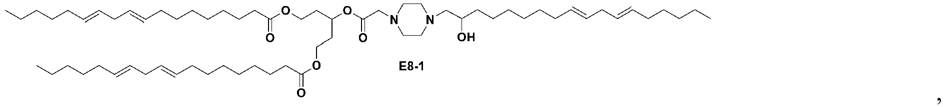 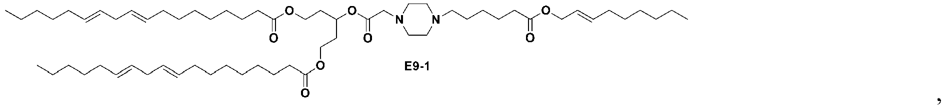 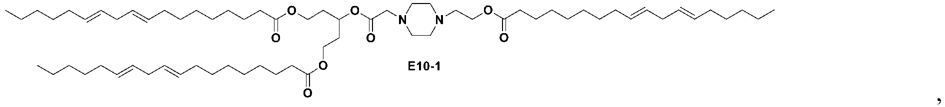 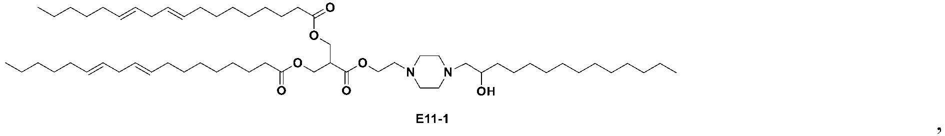 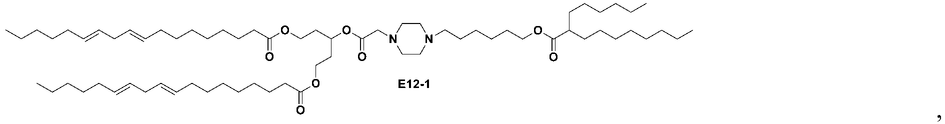 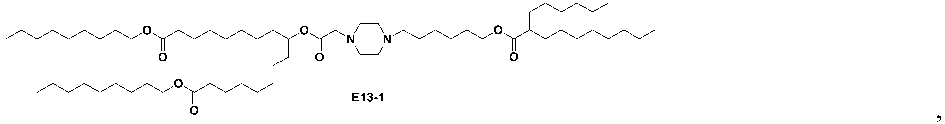 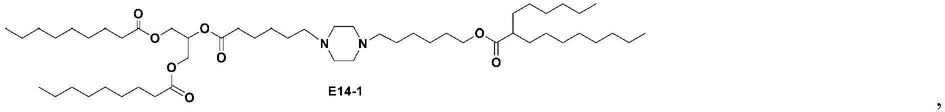 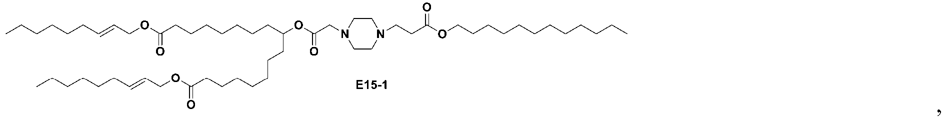 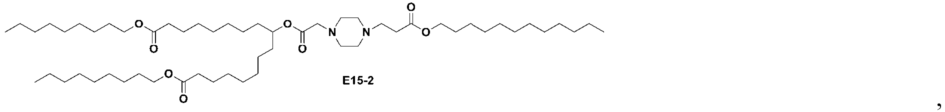 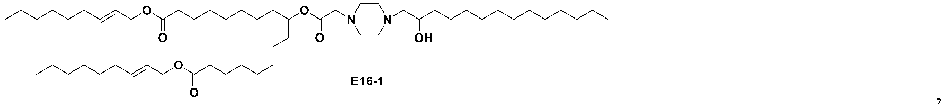 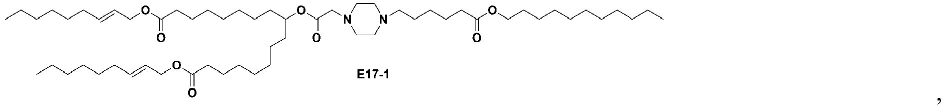 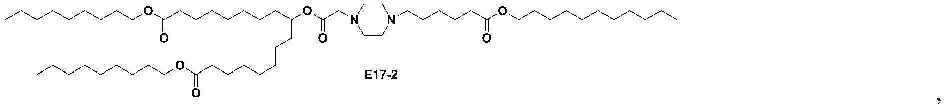 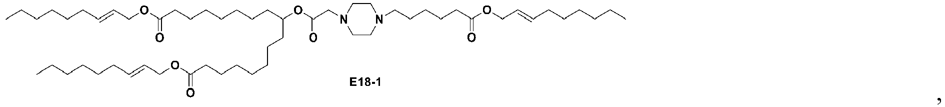 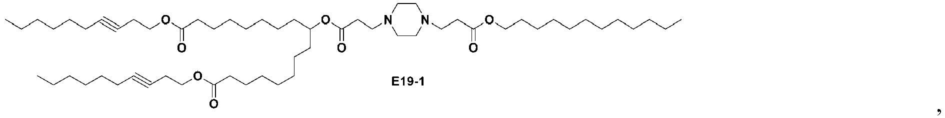 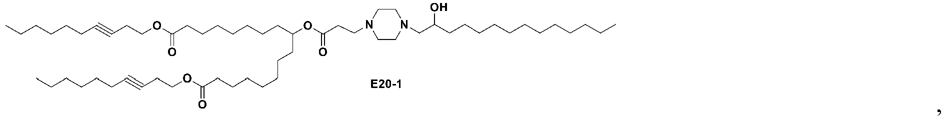 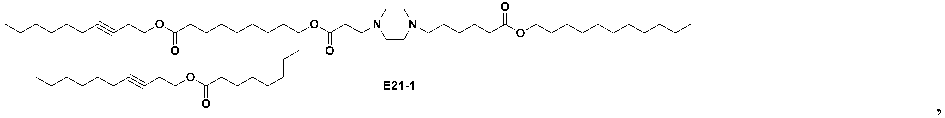 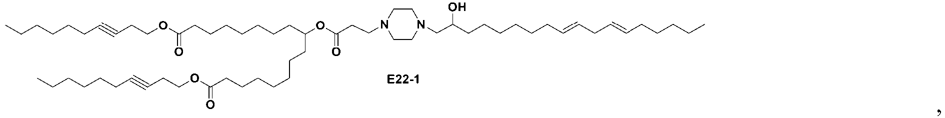 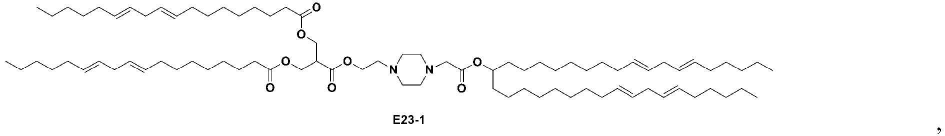 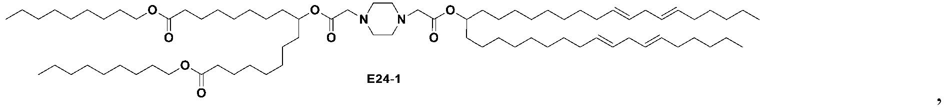 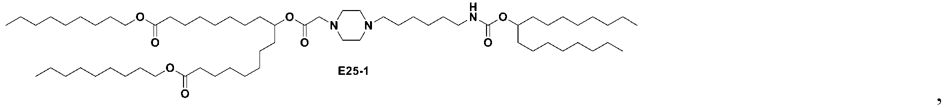 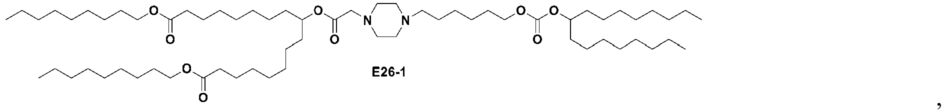 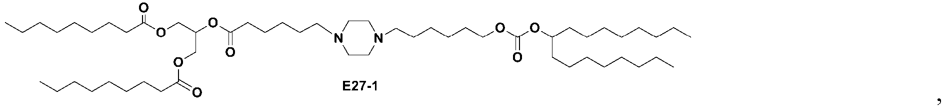 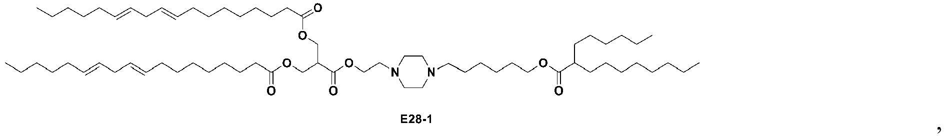 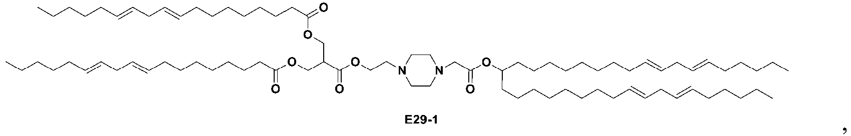 and 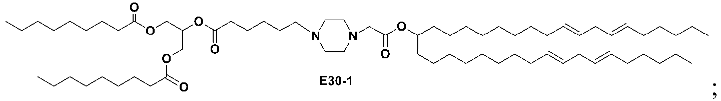 or the cationic lipid is selected from the group consisting of the following structures: 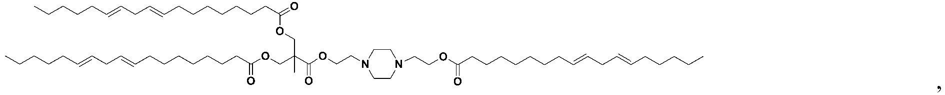 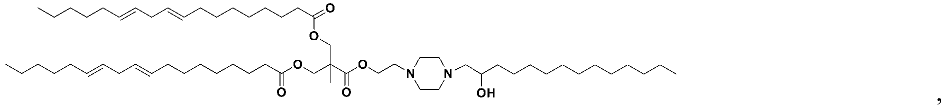 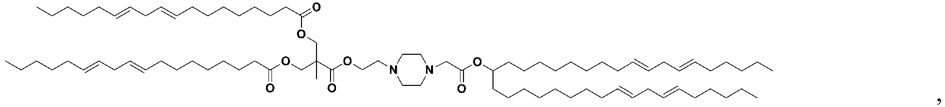 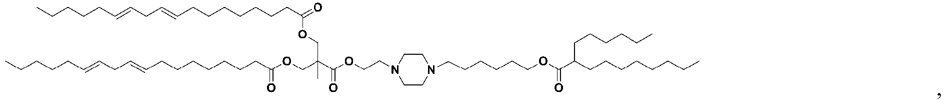 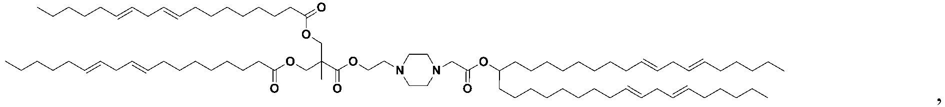 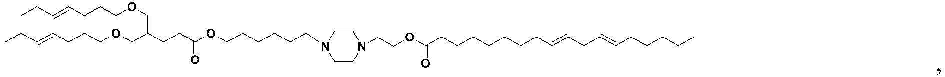 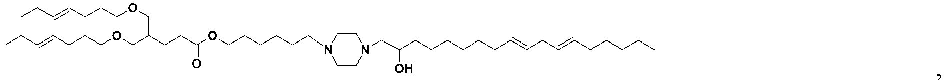 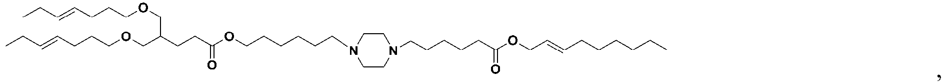 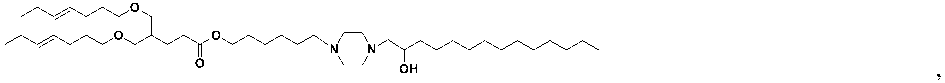 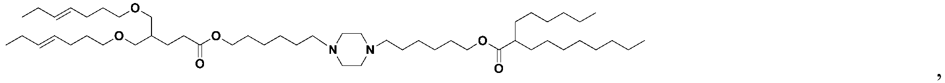 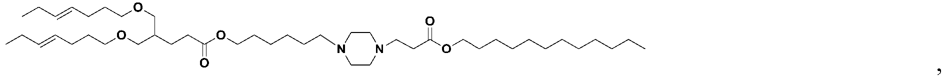 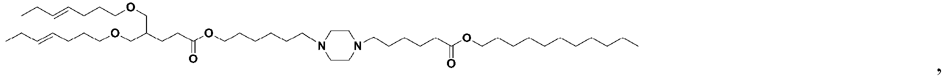 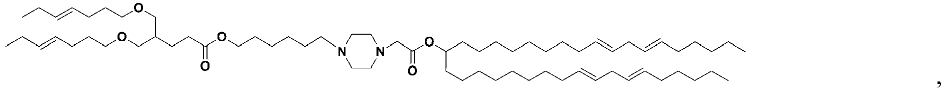 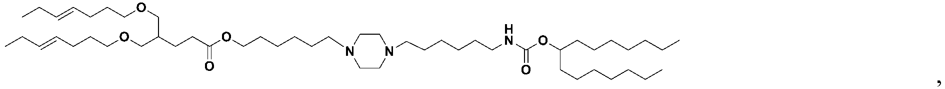 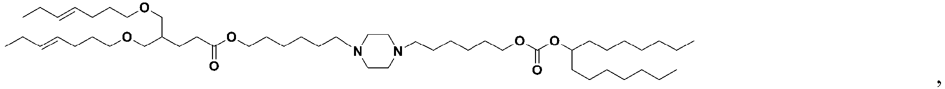 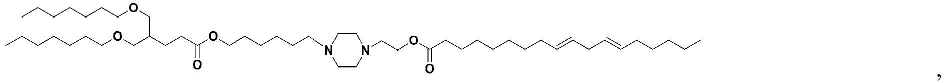 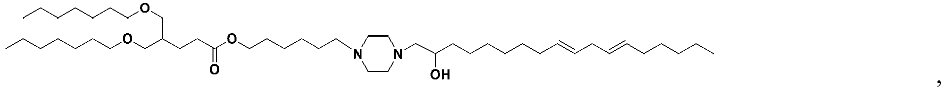 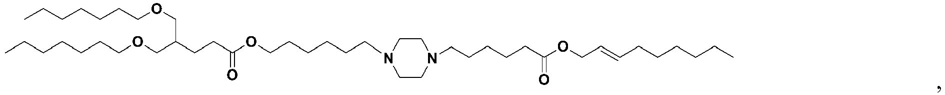 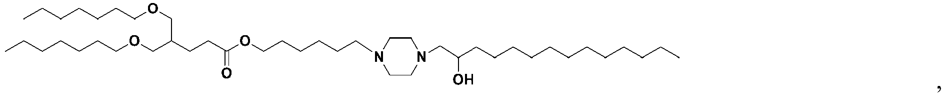 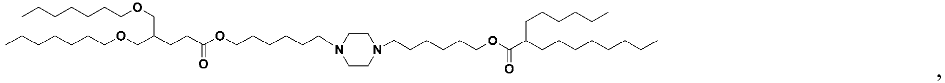 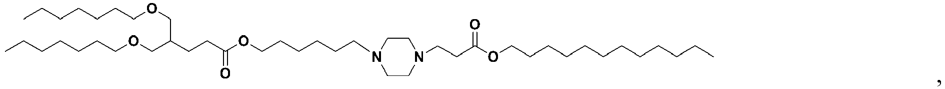 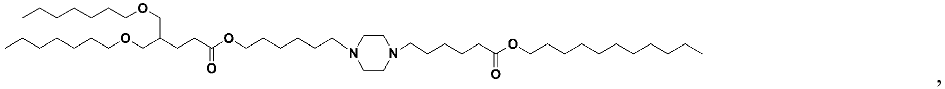 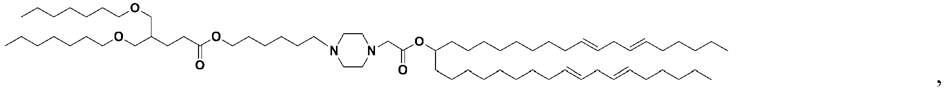 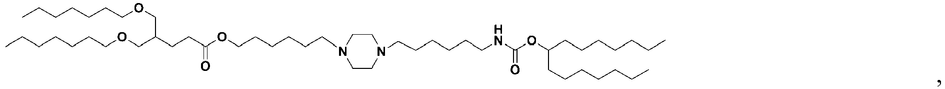 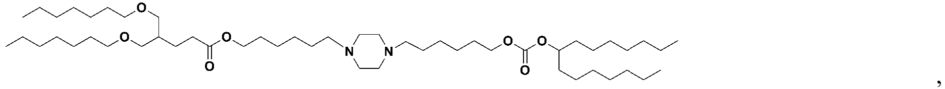 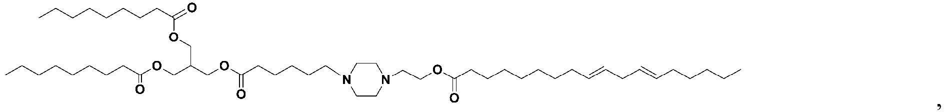 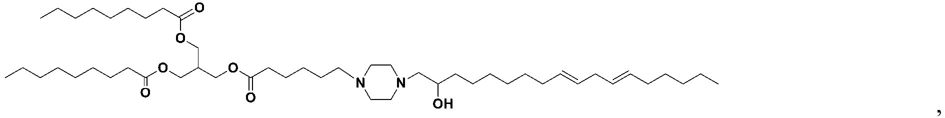 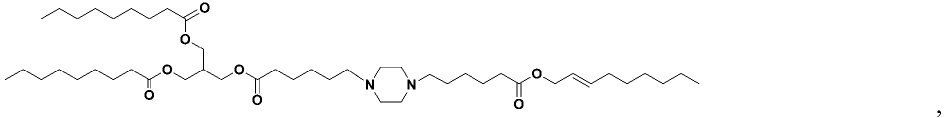 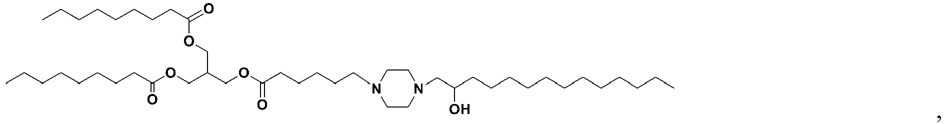 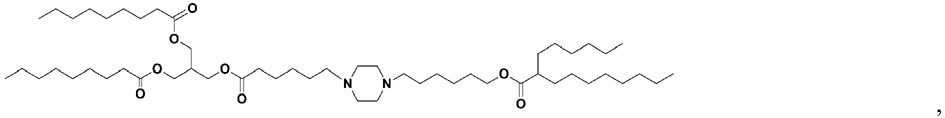 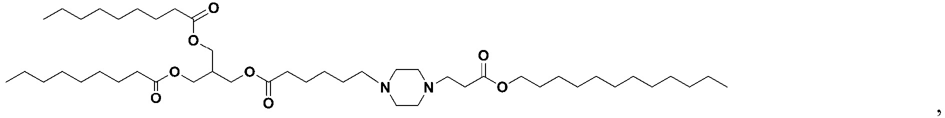 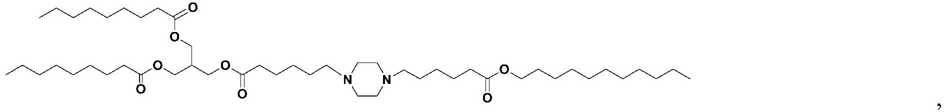 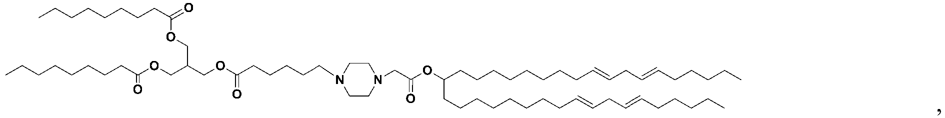 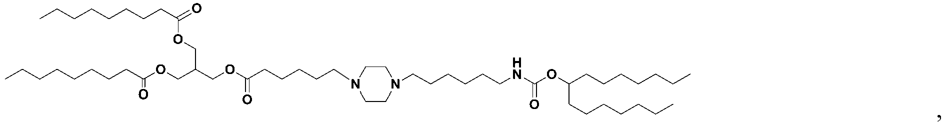 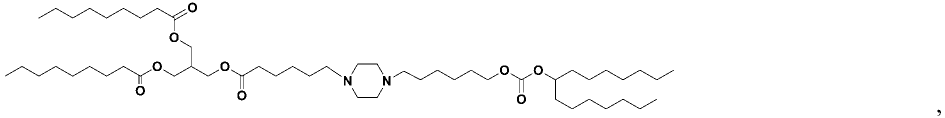 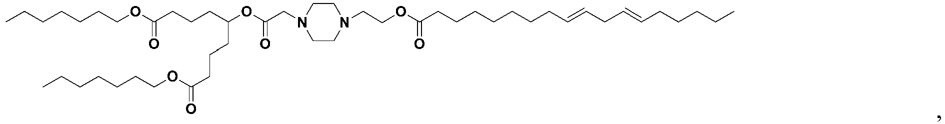 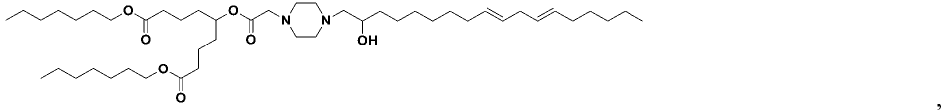 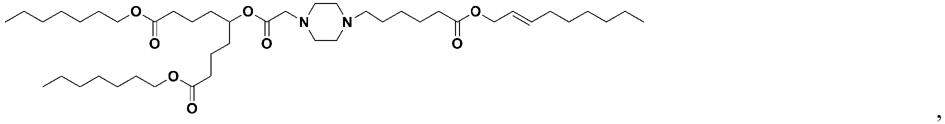 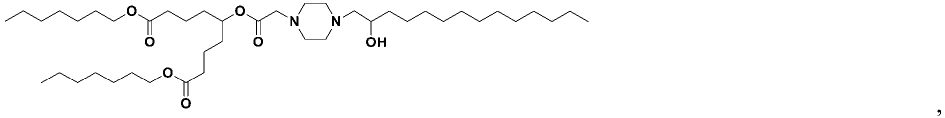 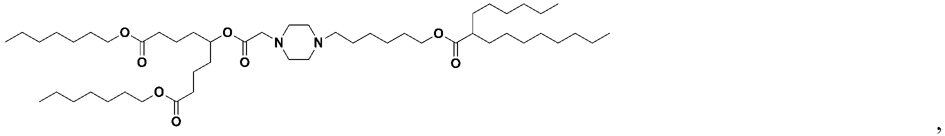 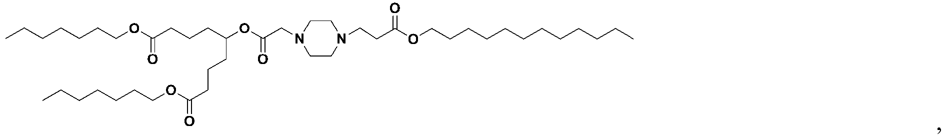 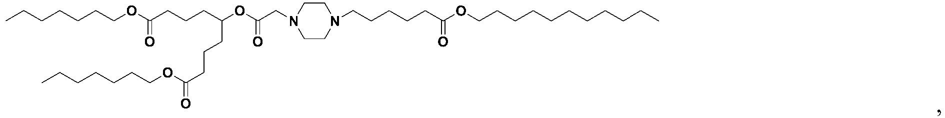 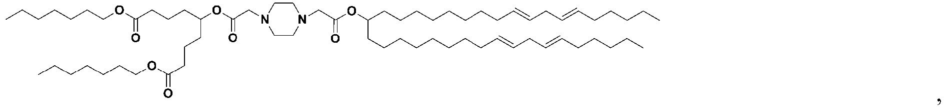 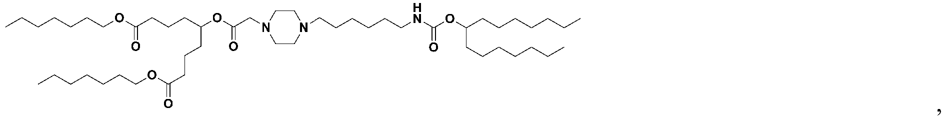 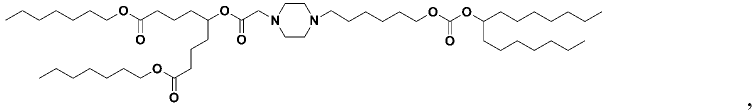 and

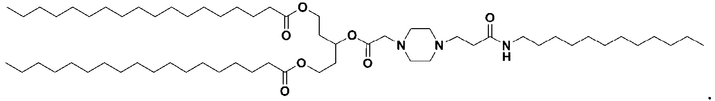

### 2. Preparation of Cationic Lipids

### 2.1. The preparation process is as follows:

The intermediates/starting materials involved in the preparation process of cationic lipids of the present application include but are not limited PIP₀, IM-C, IM-C', PIP-C, and PIP-C'.

The preparation process of the present application may involve a starting material PIP₀ containing a piperazine ring and its derivatives, the PIP₀ contains a single reactive group F_{q} or F₀, or a pair of identical or different functional groups F_{q} and F₀, and its structure is represented by 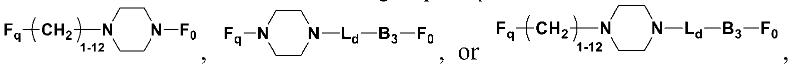 wherein F_{q} and F₀ are selected from the group consisting of H, Boc, a protected or unprotected form of -OH, and a protected or unprotected form of -COOH; the definitions of other symbols are the same as those in formula (1).

In the present application, PIP₀ is selected from the group consisting of the following structures:

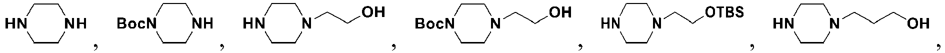

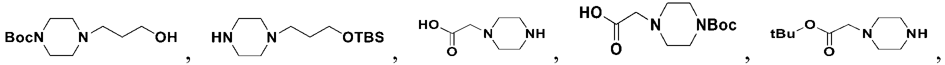

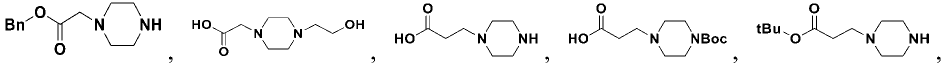

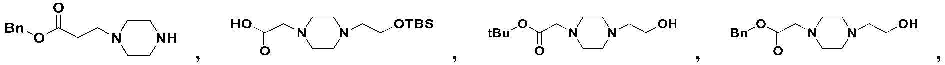

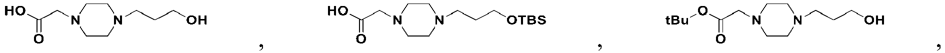

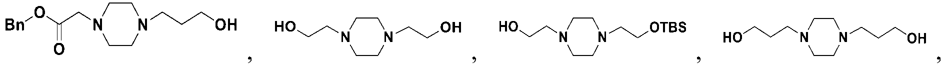

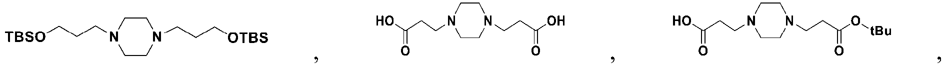

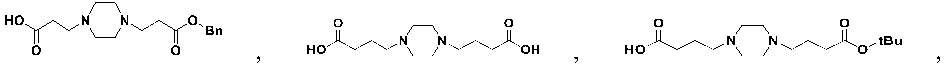

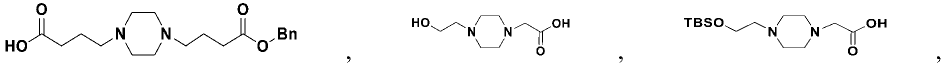

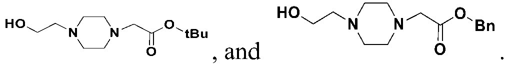

The preparation process of the present application may involve an intermediate IM-C containing a reactive group and a carbon-branched hydrophobic hydrocarbon tail chain, and its structure is represented by 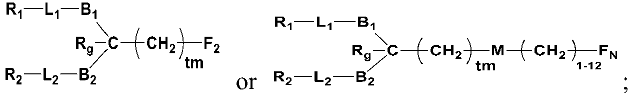 wherein, F₂ is a reactive group that can react with the F_{q} of PIP₀, F_{N} is a reactive group that can react with -NH- of PIP₀, F₂ is preferably selected from a protected or unprotected form of -OH, or a protected or unprotected form of -COOH, F_{N} is preferably selected from -F, -Cl, -Br, -CHO, or -CH=CH-; both B₁, B₂, L₁, and L₂ are no linking bonds, the definitions of other symbols are the same as those in formula (1). IM-C can be prepared by any suitable chemical reaction, which includes simple esterification, amidation, alkylation, addition, and substitution reactions, etc., either as single-step or multi-step processes. For example, in Example 1, the intermediate IM-C 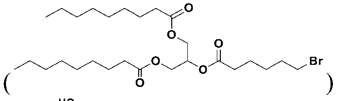 can be obtained by a esterification reaction between S1-1 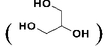 and S1-2 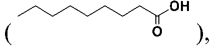 and then performing another esterification reaction with S1-4

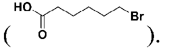

In the present application, IM-C is selected from the group consisting of the following structures:

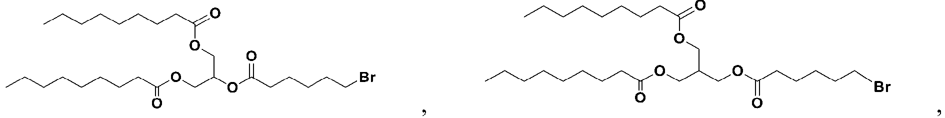

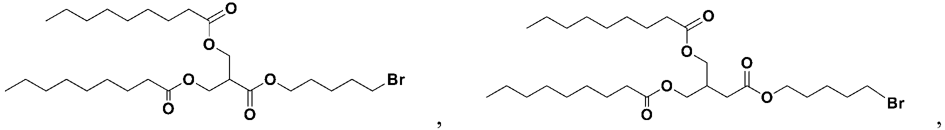

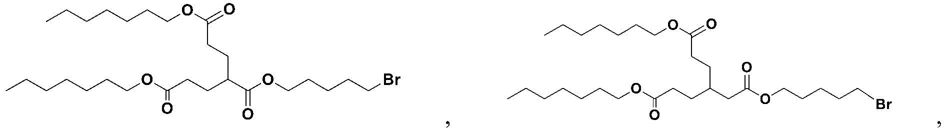

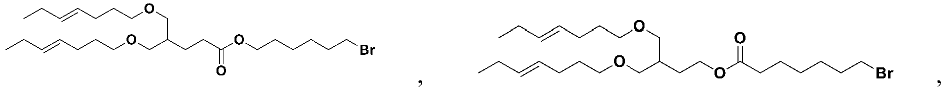

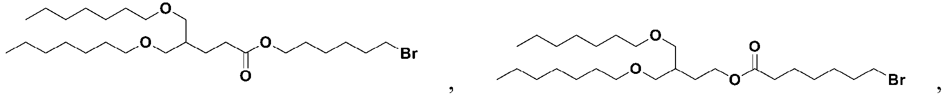

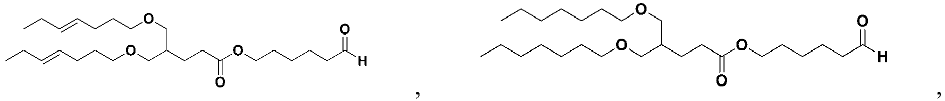

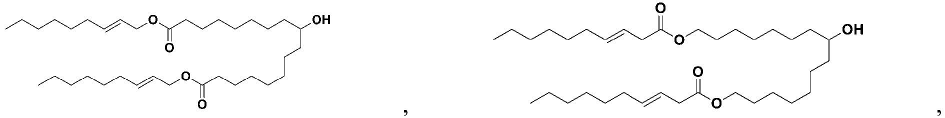

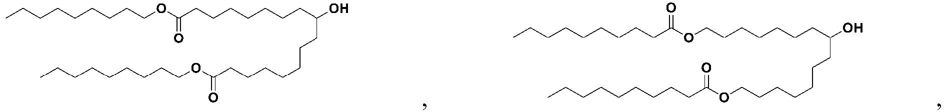

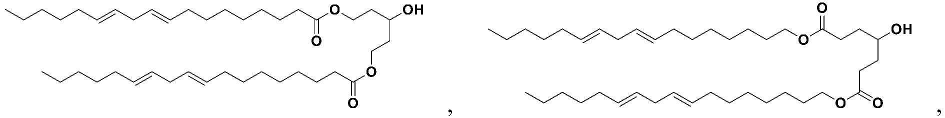

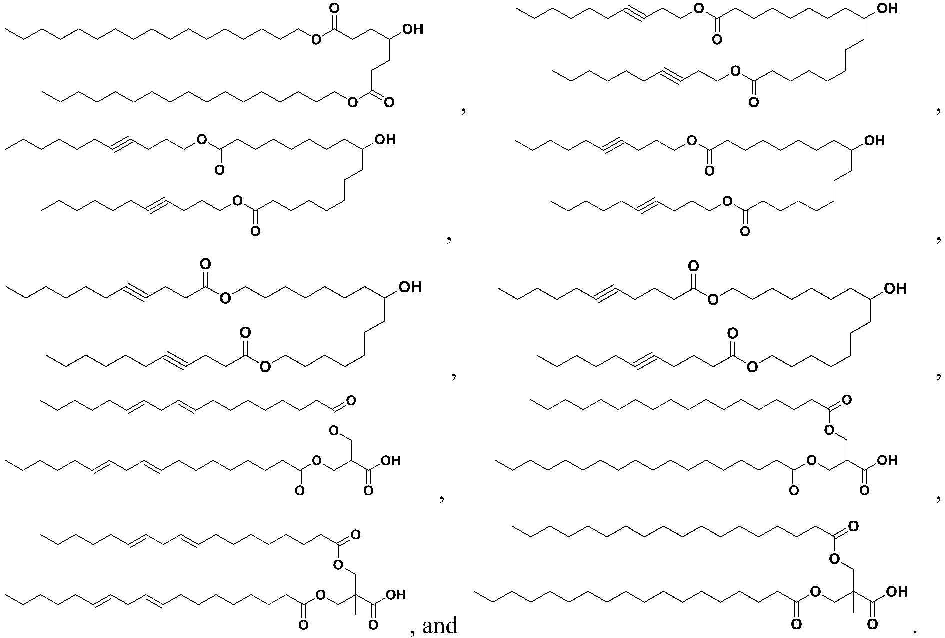

The preparation process of the present application may involve a small-molecule intermediate IM-C' with a reactive group and a hydrophobic hydrocarbon tail chain, and its structure is represented by F_{B}-B₃-L₃-R₃ or F_{A}-R₃; wherein, F_{B} is a reactive group that can react with -NH- of PIP₀, and F_{A} is a reactive group that can react with the F₀ of PIP₀, preferably selected from -OH, -COOH, -Br, -CHO, 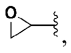 , or -CH=CH-, the definitions of other symbols are the same as those in formula (1). IM-C' can be prepared by any suitable chemical reaction, which includes simple esterification, amidation, alkylation, addition, and substitution reactions, etc., either as single-step or multi-step processes. For example, in Example 2, the small-molecule intermediate IM-C' 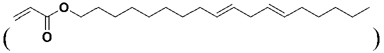 can be obtained by a substitution reaction between S2-9 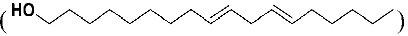 and S2-10

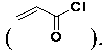

In the present application, the structure of the aforementioned IM-C' is preferably selected from the group consisting of F_{B}-B₃-L₃-R₃, F_{B}-L₃-R₃, and F_{A}-R₃; specifically, IM-C' is selected from the group consisting of the following structures:

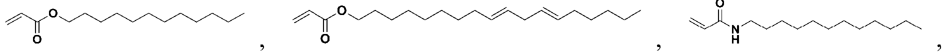

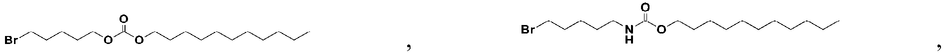

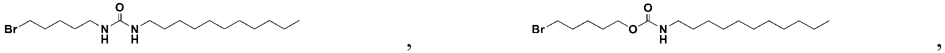

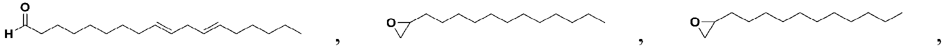

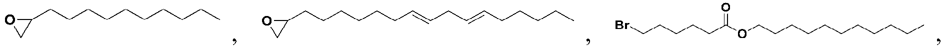

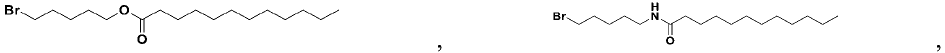

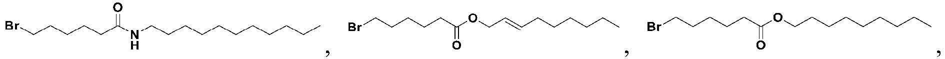

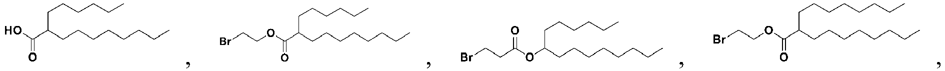

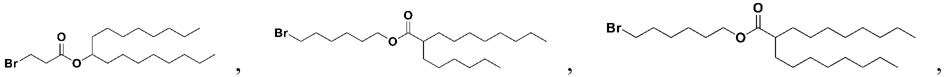

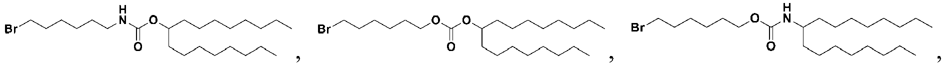

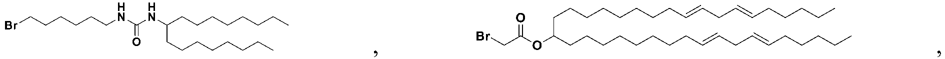

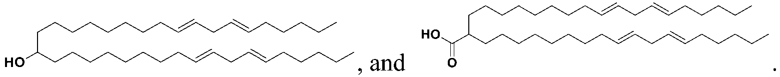

The preparation process of the present application may involve an intermediate PIP-C containing a piperazine ring, a secondary amine group, and a carbon-branched hydrophobic hydrocarbon tail chain, the structure of which is represented by 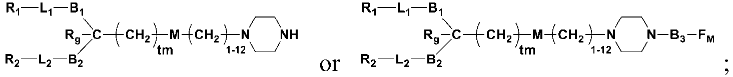 wherein, F_{M} is a reactive group that can react with the F_{A} of IM-C', preferably is -COOH or -OH; B₁, B₂, B₃, L₁, and L₂ are all no linking bonds, the definitions of other symbols are the same as those in formula (1). PIP-C can be obtained by reacting the intermediate IM-C with the starting material PIP₀. For example, in Example 2, the intermediate PIP-C 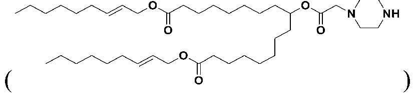 can be obtained by a esterification reaction between intermediate IM-C 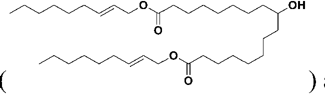 and PIP₀

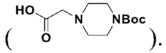

In the present application, PIP-C is selected from the group consisting of the following structures: 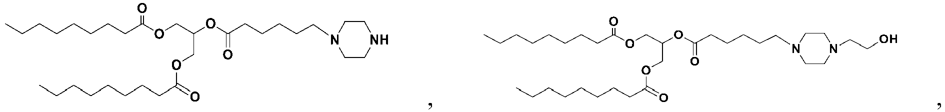 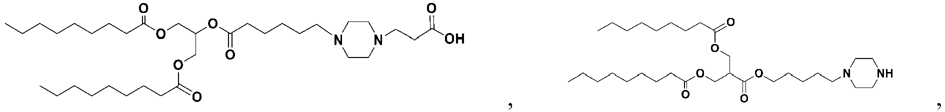 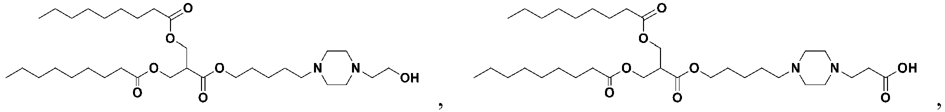 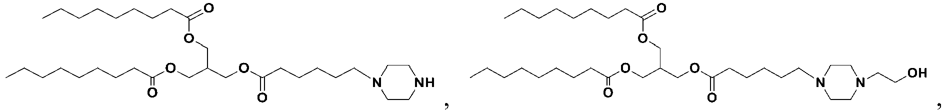 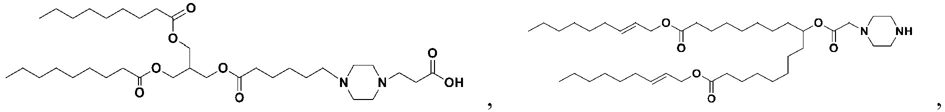 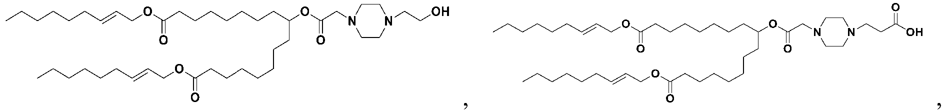 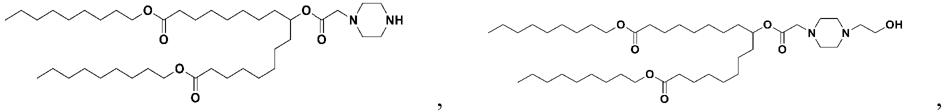 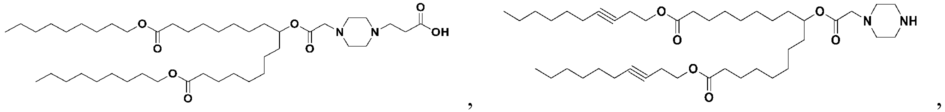 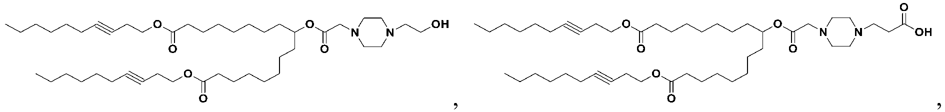 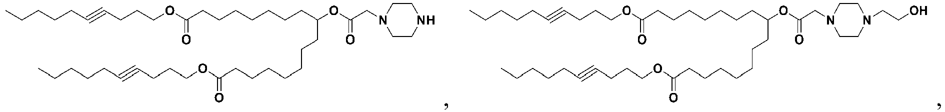 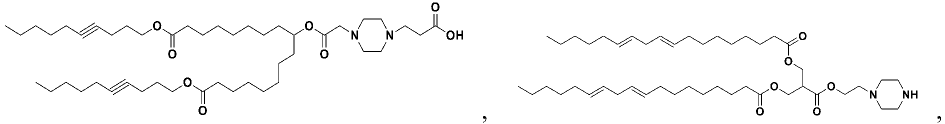 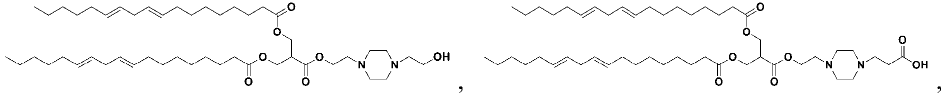 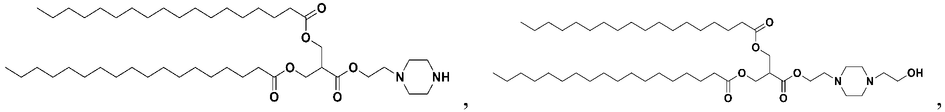 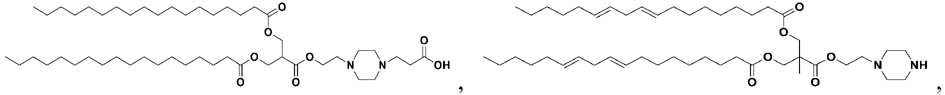 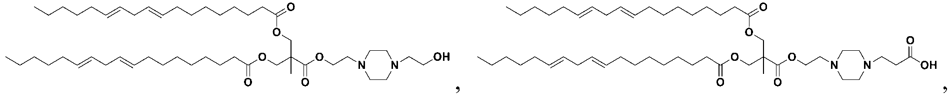 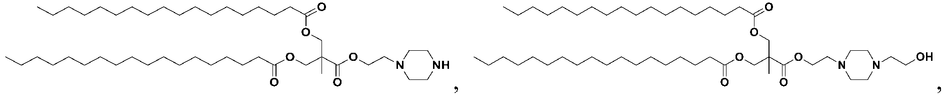 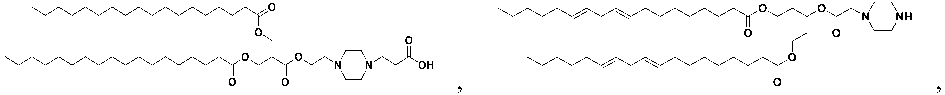 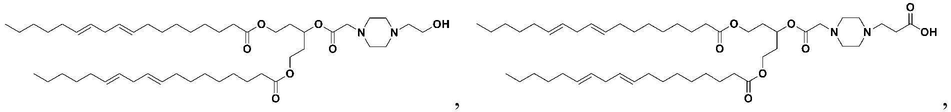 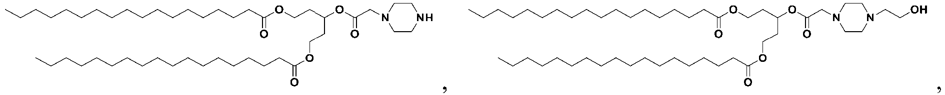 and

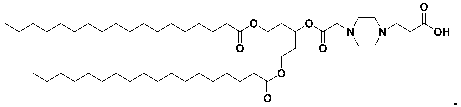

The preparation process of the present application may involve an intermediate PIP-C' containing a piperazine ring, a secondary amine group, and the other carbon-branched hydrophobic hydrocarbon tail chain, the structure of which is represented by 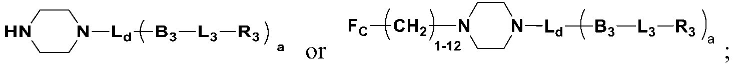 wherein, F_{C} is a reactive group that can react with the F₂ of IM-C, preferably is -COOH or -OH-; the definitions of other symbols are the same as those in formula (1). PIP-C' can be obtained by reacting PIP₀ with IM-C' through either a single reaction or multiple reactions. For example, in Example 5, the intermediate PIP-C' 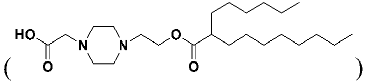 can be obtained by a esterification reaction between S5-1 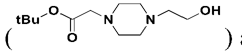 and S5-2 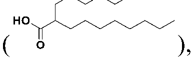 followed by the removal of protecting group.

In the present application, PIP-C' is selected from the group consisting of the following structures: 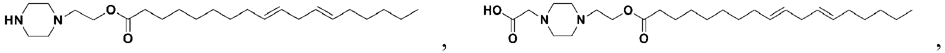 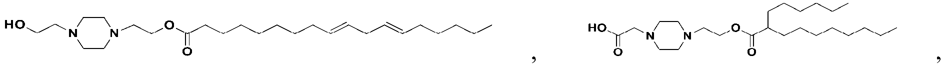 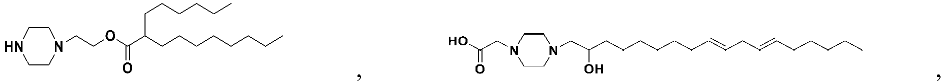 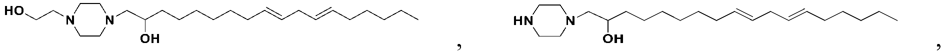 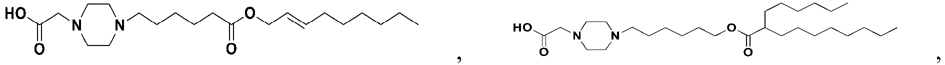 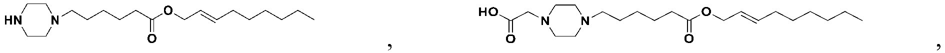 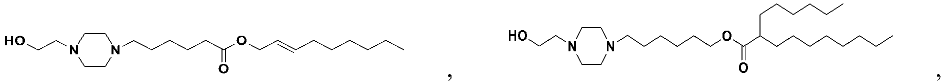 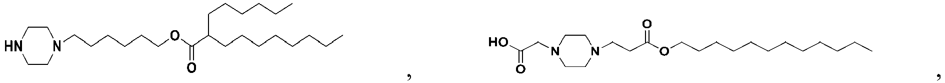 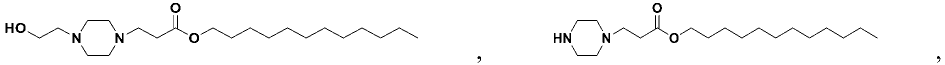 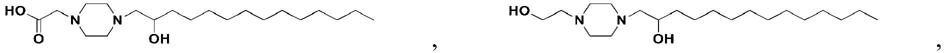 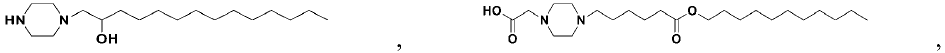 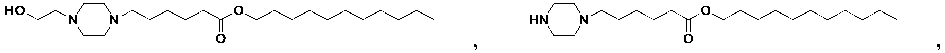 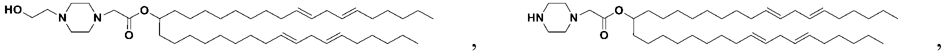 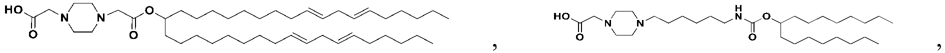 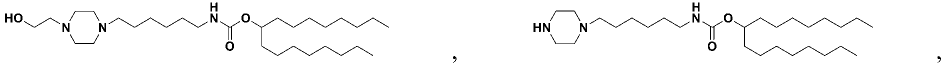 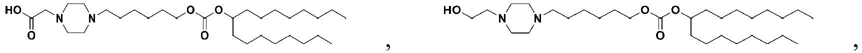 and

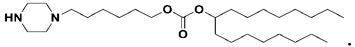

In the present application, the cationic lipid can be obtained by reacting any of the aforementioned IM-C with any of PIP₀ to obtain PIP-C, and then PIP-C reacting with IM-C'; and it can also be obtained by reacting any of the aforementioned IM-C' with any of PIP₀ to obtain PIP-C', and then PIP-C' reacting with IM-C.

### 2.2. Description of Relevant Starting Materials and/or Steps in the Preparation Process

### 2.2.1. Condensation Agents, Oxidizing Agents, and Reducing Agents

In the present application, the condensation agent used in reactions is not particularly limited, but is preferably selected from the group consisting of *N,N*'-dicyclohexylcarbodiimide (DCC), 1-ethyl-(3-dimethylaminopropyl)carbodiimide hydrochloride (EDC·HCl, EDCI), 2-(7-azabenzotriazole)-*N,N,N',N'*-tetramethyluronium hexafluorophosphate (HATU), and benzotriazole-*N,N,N',N'*-tetramethyluronium hexafluorophosphate (HBTU), most preferably DCC. Relevant reactions can be facilitated by the addition of an appropriate catalyst (e.g., 4-dimethylaminopyridine).

In the present application, the oxidizing agent used in reactions is not particularly limited, as long as it is a compound or a combination of compounds that can increase the valency of the substrate, and is preferably selected from the group consisting of phenyliodine(III) bis(trifluoroacetate), 1,4-benzoquinone, benzyltrimethylamonium tribromide, pyridine dichromate, potassium dichromate, ozone, oxygen, hypofluorous acid, sodium hypochlorite, cobalt(III) acetate, cobalt acetate, manganese acetate, palladium acetate, copper acetate, monoperoxyphthalic acid, iodine, N-iodosuccinimide, iodoylbenzene, 2-iodoylbenzoic acid, dimethyldioxirane, dimethyl sulfoxide-chloroformyl, dimethyl sulfoxide-acetic anhydride, DDQ, dichlorotris(triphenylphosphine) ruthenium, manganese dioxide, diacetoxyiodobenzene, periodic acid, sodium periodate, sodium periodate-osmium tetroxide, potassium permanganate, sodium perborate, benzoic acid peroxide, dibenzoyl peroxide, nickel peroxide, hydrogen peroxide, cumene hydroperoxide, t-butyl hydroperoxide, peracetic acid, m-chloroperbenzoic acid, N-chlorosuccinimide, pyridinium chlorochromate, palladium chloride-copper chloride, urea-hydrogen peroxide complex, trityl tetrafluoroborate, tributyltin oxide, cobalt trifluoride, vanadium oxytrifluoride, chromium trioxide, manganese triacetate, TEMPO, ammonium ceric nitrate, bromine, N-oxidopyridine, silver oxide, O-ethylcarbonate peroxide, manganese acetylacetonate, vanadium oxyacetylacetonate, aluminum isoproxide, potassium peroxymonosulfate, dichloroiodobenzene, etc., and combinations thereof; more preferably, the oxidizing agent is selected from the group consisting of oxygen, sodium hypochlorite, hydrogen peroxide, dichloroiodobenzene, potassium peroxymonosulfate, etc., and combinations thereof.

In the present application, there is no particular limitation on the reducing agent used in reactions, as long as it can reduce the Schiff base formed from the reaction between ammonia and aldehydes or ketones to an amino group, and is preferably selected from the group consisting of sodium borohydride, sodium cyanoborohydride, lithium aluminum hydride, borane, diborane, diisobutyl aluminum hydride, diisopine campyl borane, lithium borohydride, zinc borohydride, borane-pyridine, borane-methyl sulfide, borane-tetrahydrofuran, etc., and combinations thereof; the reducing agent is more preferably sodium cyanoborohydride.

In the present application, the solvent used in reactions may be absent or selected from non-protonic solvents. The non-protonic solvents include toluene, benzene, xylene, acetonitrile, ethyl acetate, ether, methyl t-butyl ether, tetrahydrofuran, chloroform, dichloromethane, dimethyl sulfoxide, dimethylformamide or dimethylacetamide, preferably tetrahydrofuran, dichloromethane, dimethyl sulfoxide, and dimethylformamide.

In the present application, the base used in reactions is an inorganic base or an organic base, preferably an organic base (e.g., triethylamine, pyridine, 4-dimethylaminopyridine, imidazole, or diisopropylethylamine); preferably, the base is triethylamine or pyridine.

### 2.2.2. "Protection" and "Deprotection" of Relevant Groups Involved in the Reaction Process

In the present application, reaction processes may involve "protection" and "deprotection" processes of relevant groups. To prevent a certain functional group from affecting the reaction, it is usually protected. In addition, when two or more functional groups are present, only the target functional group is selectively required to react, and the other functional groups should therefore be protected. The protecting group not only protects the functional group stably but also needs to be easily removed as needed. Therefore, in organic synthesis, it is important to remove only the protecting group bonded to the specified functional group under appropriate conditions.

In the present application, the definitions of "carboxyl protecting group" and "amino protecting group" are consistent with those in the "Description of Terms" section, and will not be repeated herein.

### 2.2.3. Alkylation reactions

The alkylation reactions of the present application are preferably based on the alkylation of hydroxyl groups, sulfhydryl groups or amino groups, corresponding to the formation of ether bonds, thioether bonds, secondary amino groups or tertiary amino groups, respectively. Examples include the following:

### 2.2.3.1. Alkylation reaction of substrate alcohols with sulfonates or halides

Under basic conditions, an ether intermediate can be obtained via nucleophilic substitution of the substrate alcohol with a sulfonate derivative or halide. Wherein, the molar equivalent of the sulfonate or halide is 1 to 50 times that of the substrate alcohol, preferably 1 to 5 times. When the molar equivalent of the sulfonate or halide is less than 1 times that of the substrate alcohol, the substitution might be incomplete and cause difficulty in the purification process. When the molar equivalent of the sulfonate or halide exceeds 50 times that of the substrate alcohol, the excess sulfonate or halide tends to bring difficulty in the purification process, and might be brought into the subsequent step and therefore cause more side reactions which further increase difficulty in the purification.

The resulting product is a mixture of ether intermediate and excess sulfonate or halide, and can be purified by means such as anion exchange resin, osmosis, ultrafiltration, etc. Wherein, the anion exchange resin is not particularly limited, as long as the target product can undergo ion-exchange and adsorption in the resin, and is preferably the ion exchange resin of tertiary amines or quaternary ammonia salts, with the matrix being dextran, agarose, polyacrylate, polystyrene, or poly(diphenylethylene), etc. The solvents used for osmosis or ultrafiltration are not limited, generally water or organic solvents; wherein, the organic solvent is not particularly limited, as long as it can dissolve the product, but is preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base (e.g., triethylamine, pyridine, 4-dimethylaminopyridine, imidazole, diisopropylethylamine) or an inorganic base (e.g., sodium carbonate, sodium hydroxide, sodium bicarbonate, sodium acetate, potassium carbonate, potassium hydroxide), preferably an organic base, and more preferably triethylamine or pyridine. The molar equivalent of the base used is 1 to 50 times that of sulfonates or halides, preferably 1 to 10 times, more preferably 3 to 5 times.

### 2.2.3.2. Alkylation reaction of substrate amines with sulfonates or halides

Under basic condition, an amine intermediate can be obtained via nucleophilic substitution of the substrate amine with a sulfonate derivative or halide. Wherein, the molar equivalent of the sulfonate or halide is 1 to 50 times that of the substrate amine, and preferably 1 to 5 times. When the molar equivalent of sulfonate or halide is less than 1 times that of the substrate amine, the substitution may not be complete, causing difficulty in the purification process. When the molar equivalent of sulfonate or halide exceeds 50 times that of the substrate amine, the excess sulfonate or halide tends to cause difficulty in the purification process, and may be mixed into the subsequent step, resulting in an increase in side reactions in the subsequent step and increasing the difficulty of purification.

The resulting product is a mixture of amine intermediate and excess sulfonate or halide, and can be purified by a purification means such as column chromatography, anion exchange resin, osmosis treatment, ultrafiltration treatment, and the like. Wherein, the anion exchange resin is not particularly limited as long as the target product can undergo ion-exchange and adsorb on the resin, preferably the ion exchange resin of a tertiary amine or quaternary ammonia salt based on dextran, agarose, polyacrylate, polystyrene, poly(diphenylethylene), or the like. The solvents used for osmosis treatment and ultrafiltration treatment are not limited, generally water or an organic solvent. The organic solvent is not particularly limited as long as the product can be dissolved therein, preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base (e.g., triethylamine, pyridine, 4-dimethylaminopyridine, imidazole, and diisopropylethylamine), or an inorganic base (e.g., sodium carbonate, sodium hydroxide, sodium bicarbonate, sodium acetate, potassium carbonate, and potassium hydroxide), preferably an organic base, and more preferably triethylamine or pyridine. The molar amount of base is 1 to 50 times that of the sulfonate or halide, and preferably 1 to 10 times.

### 2.2.3.3. Alkylation reaction of substrate amines with aldehyde derivatives

The substrate amine reacts with an aldehyde derivative to obtain an imine intermediate, which forms an amine intermediate under the action of reducing agents. Wherein, the molar equivalent of the aldehyde derivative is 1 to 20 times that of the substrate amine, preferably 1 to 2 times, and more preferably 1 to 1.5 times. When the molar equivalent of aldehyde exceeds 20 times that of the substrate amine, the excess reagent tends to cause difficulty in the purification process and may be mixed into the subsequent step to increase the purification difficulty. When the molar equivalent of aldehyde is less than 1 times that of the substrate amine, the substitution may not be complete, causing the purification process to be difficult. Wherein, the resulting product can be obtained after purification by means such as cation exchange resin, osmosis treatment, ultrafiltration treatment, and the like. The cation exchange resin is not particularly limited as long as it can undergo ion-exchange with quaternary ammonium cations and realize the isolation. The solvents used for osmosis treatment and ultrafiltration treatment are not limited, generally water or an organic solvent. The organic solvent is not particularly limited as long as the product can be dissolved therein, preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, etc., and more preferably water or methanol.

The reducing agent is not particularly limited as long as the imine can be reduced to an amine, preferably sodium borohydride, lithium aluminum hydride, sodium cyanoborohydride, Zn/AcOH, etc., and more preferably sodium cyanoborohydride. The molar amount of reducing agent is generally 0.5 to 50 times that of the aldehyde derivatives, and preferably 1 to 10 times.

### 3.1. Lipid Compositions

In the present application, provided herein is a lipid composition comprising any cationic lipid whose structure is represented by the general formula (1).

In a specific embodiment of the present application, the lipid composition comprises a cationic lipid whose structure is represented by general formula (1), and preferably, further comprises one or more types of lipids selected from the group consisting of phospholipid, steroid lipid and PEGylated lipid; the lipid composition is selected from any one of the following cases:
Case (1): the lipid composition further comprises a phospholipid;
Case (2): the lipid composition further comprises a steroid lipid;
Case (3): the lipid composition further comprises a PEGylated lipid;
Case (4): the lipid composition further comprises a phospholipid and a steroid lipid;
Case (5): the lipid composition further comprises a phospholipid and a PEGylated lipid;
Case (6): the lipid composition further comprises a steroid lipid and a PEGylated lipid;
Case (7): the lipid composition further comprises a phospholipid, a steroid lipid, and a PEGylated lipid;
Case (8): the lipid composition further comprises a phospholipid, a steroid lipid, a PEGylated lipid, and another cationic lipid;
Case (9): the lipid composition further comprises a phospholipid, a steroid lipid, a PEGylated lipid, and an anionic lipid;
more preferably, the lipid composition further comprises three types of lipids, simultaneously, which are a neutral lipid, a steroid lipid, and a PEGylated lipid.

In a specific embodiment of the present application, the phospholipid in the lipid composition is preferably selected from the group consisting of 1,2-dilinoleoyl-sn-glycero-3-phosphocholine (DLPC), 1,2-dimyristoleoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphatidylcholine (DSPC), 1,2-diundecanoyl-sn-glycero-3-phosphatidylcholine (DUPC), 1-plamitoyl-2-oleoyl-sn-glycero-3-phosphocholine (POPC), 1,2-di-*O*-octadecenyl-sn-glycero-3-phosphatidylcholine (18:0 Diether PC), 1-oleoyl-2-cholesterylhemisuccinyl-sn-glycero-3-phosphocholine (OChemsPC), 1-O-hexadecyl-sn-glycero-3-phosphatidylcholine (C16 Lyso PC), 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine (LPE), and combinations thereof.

In a specific embodiment of the present application, the steroid lipid in the lipid composition is preferably selected from the group consisting of cholesterol, fecal sterol, sitosterol, ergosterol, campesterol, stigmasterol, rapeseed sterol, lycopene, ursolic acid, α-tocopherol, and combinations thereof.

In a specific embodiment of the present application, the PEGylated lipid in the lipid composition is preferably selected from the group consisting of 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), and PEG-dialkyloxypropyl (PEG-DAA), specifically including PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-oleoylphosphatidylethanolamine, PEG2000-1,2-oleoylphosphatidylethanolamine, PEG2000-2,3-dimyristoylglycerol (PEG-DMG), and combinations thereof.

In a specific embodiment of the present application, the PEGylated lipid in the lipid composition is preferably selected from the group consisting of the following structures and combinations thereof: 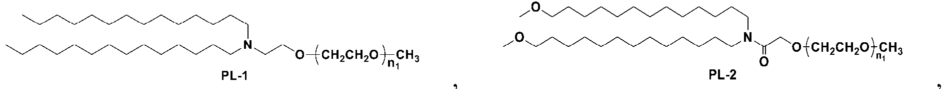 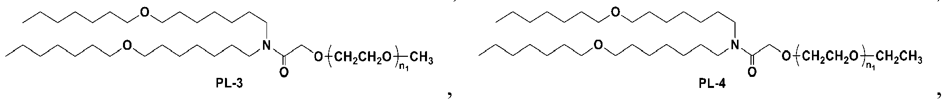 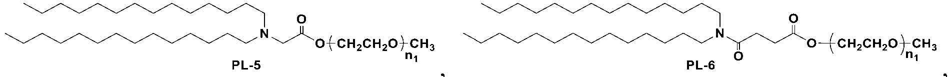 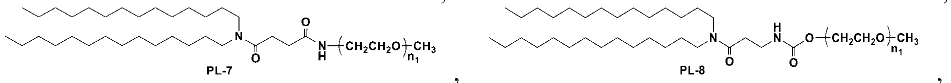 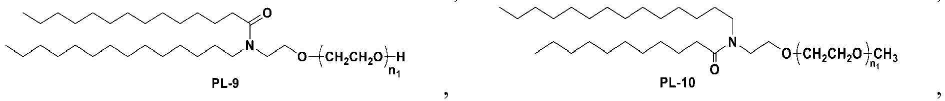 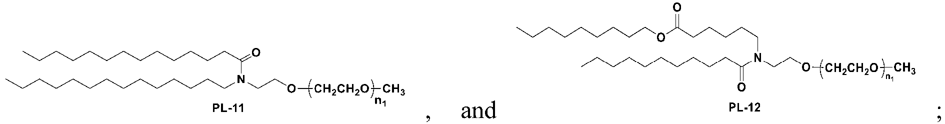 wherein, n₁ is an integer of 25 to 300, and is more preferably selected from the group consisting of 44, 45, 46, 47, and 48.

In a specific embodiment of the present application, the other cationic lipid in the lipid composition is preferably selected from the group consisting of 1,2-dioleoyl-3-trimethylammonium-propane (DOTAP), 1,2-bisoctadecoxy-3-methylammonium propane chloride (DOTMA), 1-[2-(oleoyloxy)ethyl]-2-oleo-3-(2-hydroxyethyl)imidazoline chloride (DOTIM), 1,2-diolecanol-3-dimethylamino-propane (DODMA), 2,3-bis(tetradecanoyloxy)propyltrimonium chloride (DMTAP), didecyldimonium chloride (DDAC), bisdecyldimonium bromide (DDAB), *N*,*N*-diole-*N*,*N*-dimethylammonium chloride (DODAC), N-(4-carboxybenzyl)-*N,N*-dimethyl-2,3-bis(oleoyloxy)propyl-1-ammonium (DOBAQ), 3,6-bis{4-[bis(2-hydroxydodecyl)amino]butyl}piperazine-2,5-dione (cKK-E12), 1'-((2-(4-(2-(2-(2-(bis(2-hydroxydodecyl)amino)ethyl)(2-hydroxydodecyl)amino)ethyl)pipera zine-1-yl)ethyl)azanediyl)bis(dodecane-2-ol) (C12-200), 4-(*N,N*-dimethylamino)butyric acid(dilinole)methyl ester (DLin-MC3-DMA), 1,2-dimyristoyl-sn-glycero-3-ethylphosphocholine (EPC), ((4-hydroxybutyl) azadiyl)bis(hexane-6,1-diyl)bis(2-hexyldecanoate) (ALC-0315), 8-[(2-hydroxyethyl)(6-oxo-6-(undecoxy)hexyl)amino]caprylic acid (hepta-9-yl) ester (SM-102), and ((2-(2-hydroxyethoxy)ethyl)azadialkyl)bis(hexane-6,1-diyl)bis(2-hexyldecanoate) (DHA-1).

In a specific embodiment of the present application, the anionic lipid in the lipid composition is preferably selected from the group consisting of 1,2-dioleoyl-sn-glycerol-3-phosphate sodium salt (18:1 PA), 1,2-dimyristoyl-sn-glycerol-3-phosphate sodium salt (14:0 PA), bis(monoolein) phosphatidylmonomethylethanolamine (18:1 BMP), and cardiolipin (CL).

In a specific embodiment of the present application, the lipid composition comprises 20-80% cationic lipid represented by formula (1), 5-16% phospholipid, 25-55% steroid lipid, and 0.5-10% PEGylated lipid; the percentages refer to the molar percentages of each lipid in the total lipids in the solvent-comprising solution.

In a specific embodiment of the present application, in any of the aforementioned lipid compositions, the molar percentage of cationic lipids in the total lipids in the solvent-comprising solution is preferably 30-65%, more preferably about 35%, 40%, 45%, 46%, 47%, 48%, 49%, 50%, or 55%.

In a specific embodiment of the present application, in any of the aforementioned lipid compositions, the molar percentage of phospholipids in the total lipids in the solvent-comprising solution is preferably about 7.5-16%, more preferably about 8%, 9%, 10%, 11%, 12%, or 16%.

In a specific embodiment of the present application, in any of the aforementioned lipid compositions, the molar percentage of steroid lipids in the total lipids in the solvent-comprising solution is preferably 35-50%, more preferably about 40%, 41%, 42%, 43%, 44%, 45%, 46%, 47%, 48%, 49%, or 50%.

In a specific embodiment of the present application, in any of the aforementioned lipid compositions, the molar percentage of PEGylated lipids in the total lipids in the solvent-comprising solution is preferably 0.5-5%, more preferably 1-3%, more preferably about 1.5%, 1.6%, 1.7%, 1.8%, 1.9%, 2.0%, or 2.5%.

### 3.2. Preparation of Lipid Compositions

In the present application, the lipid composition can be prepared by the following methods, including but not limited to ethanol injection, microfluidic method, T-tube mixing method, and periplasmic extrusion method, preferably ethanol injection and microfluidic method.

### 4. Lipid Pharmaceutical Compositions and Formulations Thereof

### 4.1. Lipid Pharmaceutical Compositions

In one embodiment of the present application, provided herein is a lipid pharmaceutical composition, which comprises any aforementioned lipid composition and a drug; wherein, the lipid composition comprises any aforementioned cationic lipid represented by formula (1-A), and the drug is selected from the group consisting of a nucleic acid drug, a gene vaccine, an antitumor drug, a small molecule drug, a peptide drug, and a protein drug.

In a specific embodiment of the present application, in the lipid pharmaceutical composition, the nucleic acid drug is selected from the group consisting of RNA, DNA, antisense nucleic acid, plasmid, interfering nucleic acid, aptamer, antagomir, and ribozyme; the RNA is selected from the group consisting of mRNA, saRNA, circRNA, miRNA, and siRNA; the nucleic acid drug is preferably selected from the group consisting of DNA, mRNA, miRNA, and siRNA.

In a specific embodiment of the present application, the lipid pharmaceutical composition is preferably used as a drug, selected from any of the following drugs: an antineoplastic agent, an antiviral agent, an antifungal agent, and a vaccine.

In a specific embodiment of the present application, the drug in the lipid pharmaceutical composition includes, but is not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, and maytansinoid.

In a specific embodiment of the present application, the N/P ratio of the lipid composition to the nucleic acid is preferably (0.1~100): 1, more preferably (0.2~30): 1, and most preferably (0.5~20): 1.

### 4.2. Formulations of Lipid Pharmaceutical Compositions

In a specific embodiment of the present application, the drug in the lipid pharmaceutical composition is a nucleic acid drug, and the working solution in the formulation of lipid pharmaceutical composition is preferably deionized water, ultrapure water, phosphate buffer or normal saline, more preferably phosphate buffer or normal saline, and most preferably normal saline; the ratio of the lipid composition to the working solution is preferably (0.05~20) g: 100 mL, more preferably (0.1~10) g: 100 mL, and most preferably (0.2~5) g: 100 mL.

In a specific embodiment of the application, provided herein is a formulation of lipid pharmaceutical composition, which comprises the aforementioned lipid pharmaceutical composition and a pharmaceutically acceptable diluent or excipient; the diluent or excipient is preferably deionized water, ultrapure water, phosphate buffer or normal saline, more preferably phosphate buffer or normal saline, and most preferably normal saline.

In the present application, the preparation of the formulation of lipid pharmaceutical composition includes the following steps:
(1) equilibrate the lipid composition in the diluent or excipient;
(2) add the nucleic acid drug to the equilibrated mixture comprising the lipid composition and the diluent or excipient, for complexation;
wherein, the equilibration time is preferably 0.1~12 hours, preferably 0.2~6 hours, and more preferably 0.5~3 hours; the complexation time is preferably 0.1~12 hours, preferably 0.2~5 hours, and more preferably 0.5~2 hours.

### 5. Liposomes, Lipid Nanoparticles (LNPs) and Preparation Thereof

### 5.1. Liposomes or LNPs

In a specific embodiment of the present application, provided herein is a liposome or LNP, which comprises any aforementioned lipid pharmaceutical composition.

In a specific embodiment of the present application, the aforementioned LNP is preferably an LNP-pharmaceutical composition, an LPP-pharmaceutical composition, or a PNP-pharmaceutical composition, preferably an LNP-pharmaceutical composition, more preferably an LNP-nucleic acid pharmaceutical composition, more preferably an LNP-mRNA pharmaceutical composition.

### 5.2. Preparation of Liposomes or LNPs

In a specific embodiment of the present application, liposomes can be prepared by the following methods, including but not limited to thin-film dispersion method, ultrasonic dispersion method, reverse-phase evaporation method, freeze-drying method, freeze-thaw method, double emulsion method, and injection method, preferably thin-film dispersion method, ultrasonic dispersion method and/or reverse-phase evaporation method.

In a specific embodiment of the present application, LNPs can be prepared by the following methods, including but not limited to microemulsion method, double emulsion method, high shear homogenization and ultrasonication, thin-film hydration extrusion method, and microfluidic method.

In a specific embodiment of the present application, liposomes are prepared by the thin-film dispersion method which includes the following steps:
Step (1): weigh cationic lipids, steroid lipids, neutral lipids, and PEGylated lipids, fully dissolve them in an organic solvent, shake well, remove the organic solvent by vacuum rotary evaporation to obtain an oil film, and furtherly dry with a vacuum pump to remove the organic solvent;
Step (2): add phosphate buffer with dissolved cryoprotectant, and use water-bath ultrasonication to obtain a translucent emulsion;
Step (3): add the emulsion to a high-pressure homogenizer for overpressure, and then add the over pressurized homogenized emulsion to the liposome extruder for film-passing to obtain the cationic liposome;
Step (4): optionally, dry the liposome in a freeze dryer to obtain a liposome powder.

In a specific embodiment of the present application, in the preparation methods of liposomes, the ratio of the liposome to the phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, the LNPs are preferably prepared by the method of microfluidics, vertexing, or simple mixing, and the steps are as follows:
Step (1): dissolve each lipid component in the organic solvent to obtain the lipid composition dissolved in organic phase, wherein the organic phase is preferably ethanol;
Step (2): add the nucleic acid drug to the buffer to obtain the aqueous solution, wherein the aqueous phase is preferably a citrate buffer or sodium acetate buffer;
Step (3): use a microfluidic device, a vortex or a pipette to mix the organic phase solution with the aqueous phase solution to obtain the LNP composition, and perform purification such as ultrafiltration and the like to remove the organic solvent and the free nucleic acid molecules.

The preparation method of the present application includes any protection and deprotection processes of specific groups required for the reaction. The intermediates and final products prepared in this application can be purified by methods including but not limited to extraction, recrystallization, adsorption treatment, precipitation, reverse precipitation, thin-film dialysis, supercritical extraction, and column chromatography (including gel columns, ion columns, silica columns, etc.). The characterization of the structure, molecular weight, and purity of the intermediates and final products can be performed using methods including but not limited to ¹H NMR, electrophoresis, UV-visible spectrophotometry, FTIR, AFM, GPC, HPLC, MALDI-TOF MS, and circular dichroism spectroscopy. In the present application, the structure of cationic lipid final product is characterized by NMR, and its molecular weight is confirmed by mass spectrometry; the molecular weight (defaulting to number-average molecular weight, Mn) and polydispersity index (PDI) of the polymer are determined by gel permeation chromatography (GPC), and the degree of polymerization (defaulting to number-average degree of polymerization) of the polymer is calculated based on its molecular weight. The following specific examples are further descriptions of the preparation methods of cationic lipids, lipid compositions, and formulations of lipid pharmaceutical compositions, and the biological activity assays for lipid pharmaceutical compositions. The specific examples are disclosed to further illustrate the application, but should not be regarded as a limitation of the scope of present application.

### Example-1.1: Cationic Lipid (E1-1)

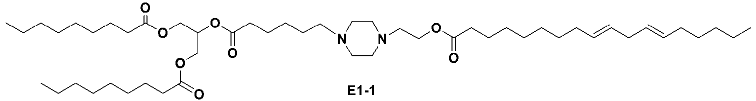

The preparation process is as follows:
Step a: glycerol (**S1-1,** 0.74 g, 8.0 mmol) was adsorbed onto an equal weight of silica gel by vigorous stirring for 30 minutes, then pelargonic acid (**S1-2,** 2.53 g, 16.0 mmol), immobilized **lipase R. miehei** (0.20 g), and **molecular sieves** (0.55 g) were added to the preparation, and then the preparation was suspended in diethyl ether (50 mL) and stirred at room temperature for 48 hours. The reaction was continuously monitored by **TLC** analysis. After the reaction was completed, the lipase and silica gel were separated by filtration, the filtrate was concentrated, and the crude product was recrystallized from methanol to obtain compound **S1-3** (2.42 g).
Step b: under nitrogen atmosphere, *N,N'*-dicyclohexylcarbodiimide (**DCC,** 2.27 g, 11.0 mmol) was added to a round-bottom flask containing **S1-3** (2.24 g, 6.0 mmol), 6-bromohexanoic acid (**S1-4,** 0.98 g, 5.0 mmol), and 4-dimethylaminopyridine (**DMAP,** 0.15 g, 1.3 mmol) dissolved in dichloromethane (60 mL), and then the reaction was carried out at room temperature for 16 hours. After the reaction was completed, the precipitate was removed by filtration, and the filtrate was concentrated. The crude product was purified by column chromatography to obtain **S1-5** (2.32 g).
Step c: under nitrogen atmosphere, compound N-hydroxyethylpiperazine (**S1-6,** 0.39 g, 3.0 mmol) was dissolved in acetonitrile (30 mL). Under slow stirring, **S1-5** (2.15 g, 3.9 mmol) and N,N-diisopropylethylamine (**DIPEA,** 0.50 g, 3.9 mmol) were added sequentially. The reaction was stirred at room temperature for approximately 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain **S1-7** (1.48 g).
Step d: under nitrogen atmosphere, **DCC** (0.68 g, 3.3 mmol) was added to a round-bottom flask containing linoleic acid (**S1-8,** 0.42 g, 1.5 mmol), **S1-7** (1.08 g, 1.8 mmol), and **DMAP** (0.05 g, 0.4 mmol) dissolved in dichloromethane (20 mL), and then the reaction was carried out at room temperature for 16 hours. After the reaction was completed, the precipitate was removed by filtration, and the filtrate was concentrated. The crude product was purified by column chromatography to obtain the cationic lipid **E1-1** (1.09 g). ¹H NMR (400 MHz, CDCl₃) δ: 5.37-5.31 (m, 5H; 1H, -C(=O)OC*H*<; 4H, -C*H*=C*H*CH₂C*H*=C*H*-), 4.38-4.29 (m, 2H, >CH(C*H*₂OC(=O))₂-), 4.20 (t, 2H, pip-CH₂CH₂OC(=O)-), 4.19-4.14 (m, 2H, >C*H*(C*H*₂OC(=O))₂-), 2.77 (t, 2H, -C*H=*C*H*C*H*₂C*H=*C*H*-), 2.72-2.40 (m, 12H; 8H, pip-*H*; 4H, pip-C*H*₂CH₂-), 2.32-2.27 (m, 8H, -CH₂C*H*₂C(=O)O-), 2.07-2.02 (m, 4H, -CH=CHC*H*₂CH₂-), 1.65-1.21 (m, 46H, -CH₂C*H*₂CH₂-, -C*H*₂CH₃), 0.87 (t, 9H, -CH₂C*H*₃). MS (ESI): m/z=860.7 ([M+H]⁺).

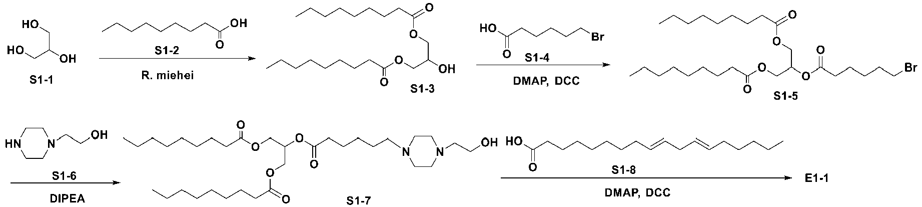

### Example-2.1: Cationic Lipid (E2-1)

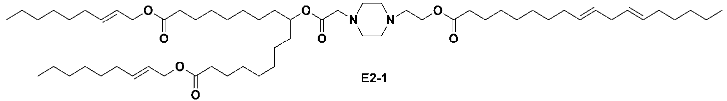

The preparation process is as follows:
Step a: To a clean round-bottom flask, freshly activated **magnesium turnings** (0.66 g, 27.6 mmol) and anhydrous ether (2 mL) were added sequentially. 9-Bromo-1-nonene (**S2-1,** 4.55 g, 22.2 mmol) was dissolved in anhydrous ether (60 mL) and transferred to a drop funnel. Under an ice bath, the bromide ether solution was added dropwise to the magnesium turnings. After the addition was completed, the reaction solution was stirred at 35°C for 1 hour and then cooled in an ice bath. **Ethyl formate** (0.74 g, 10.0 mmol) was dissolved in anhydrous ether (6 mL) and added to the reaction solution under stirring. After the reaction solution began to reflux, the remaining ether solution of ethyl formate was quickly added. The reaction solution was stirred at room temperature for an additional 1 hour. Then the reaction was quenched by the dropwise addition of acetone (5 mL) and ice water (15 mL). The reaction solution was treated with aqueous H₂SO₄ (10% v/v, 100 mL) until the solution became homogeneous and was allowed to stand for phase separation. The aqueous phase was extracted with ether (50 mL × 2), then the organic phases were combined and dried with anhydrous magnesium sulfate, filtered, and the filtrate was concentrated. The crude product was purified by column chromatography to obtain compound **S2-2** (2.52 g, 90%).
Step b: **S2-2** (1.96 g, 7.0 mmol) was dissolved in a mixture of dichloromethane (15 mL) and acetonitrile (15 mL), and ruthenium chloride (**RuCl₃,** 0.26 g, 1.2 mmol) was added, then the mixture was cooled to 10°C and an aqueous solution of **sodium periodate** (0.15 g, 0.7 mmol) was added dropwise, stirring at 10°C for 20 hours. After the reaction was completed, the reaction solution was diluted with water, and the organic phase and aqueous phase were separated, and then saturated brine was added to the organic phase under stirring, followed by the dropwise addition of a 3% sodium sulfide solution for decolorization. The organic and aqueous phase was separated, the organic phase was dried with anhydrous magnesium sulfate, filtered, and the filtrate was concentrated to obtain compound **S2-3** (1.90 g).
Step c: Under a nitrogen atmosphere, ***t*-butyldimethylsilyl chloride** (0.83 g, 5.5 mmol) was added to a round-bottom flask containing **S2-3** (1.58 g, 5.0 mmol) and **imidazole** (0.85 g, 12.5 mmol) dissolved in DMF (30 mL). The reaction solution was stirred at 50°C overnight. After the reaction was completed, the solution was cooled to room temperature, diluted once with water, and extracted twice with ethyl acetate. The combined organic phase was washed once with saturated brine, dried with anhydrous magnesium sulfate, filtered, and the filtrate was concentrated. The crude product was purified by column chromatography to obtain the diacid with the hydroxyl group protected by TBS (**S2-4,** 1.97 g, 91.2%).
Step d: Under a nitrogen atmosphere, **DCC** (3.71 g, 18.0 mmol) was added to a round-bottom flask containing 2-nonen-1-ol (**S2-5,** 1.42 g, 10.0 mmol), **S2-4** (1.72 g, 4.0 mmol), and **DMAP** (0.24 g, 2.0 mmol) dissolved in dichloromethane (80 mL). The reaction was carried out at room temperature for 16 hours. After the reaction was completed, the precipitate was removed by filtration, and the filtrate was concentrated. The residue was dissolved in tetrahydrofuran (20 mL), and then 20 mL of a 1 M TBAF solution in tetrahydrofuran was added, and the reaction was allowed to proceed overnight to remove the TBS protecting group. After the reaction was completed, the solution was concentrated, extracted, and the organic phase was combined, dried with anhydrous magnesium sulfate, filtered, and the filtrate was concentrated to obtain the crude product of S2-6, and then purified by column chromatography to obtain compound **S2-6** (1.72 g).
Step e: Under a nitrogen atmosphere, **DCC** (0.91 g, 4.4 mmol) was added to a round-bottom flask containing **S2-6** (1.36 g, 2.4 mmol), 4-Boc-1-piperazine acetic acid (**S2-7,** 0.49 g, 2.0 mmol), and **DMAP** (0.06 g, 0.5 mmol) dissolved in dichloromethane (30 mL), and the reaction was carried out at room temperature for 16 hours. After the reaction was completed, the precipitate was removed by filtration, and the filtrate was concentrated to obtain the crude product. The crude product was dissolved in dichloromethane, 0.1 M TFA was added, and the reaction was allowed to proceed for 4 hours. Tthen the reaction solution was adjusted to neutral pH, concentrated, diluted with purified water, and extracted with dichloromethane. The organic phase was dried with anhydrous magnesium sulfate, filtered, and the filtrate was concentrated. The crude product was purified by recrystallization to obtain compound **S2-8** (1.04 g).
Step f: Under ice bath conditions, linoleyl alcohol (**S2-9,** 0.80 g, 3.0 mmol) and **triethylamine** (0.86 g, 8.6 mmol) were added to dichloromethane (20 mL). A solution of acryloyl chloride (**S2-10,** 0.41 g, 4.5 mmol) in dichloromethane (10 mL) was then added dropwise to the reaction solution. The reaction solution was stirred at 20°C for 2 hours. After the reaction was completed, the precipitate was removed by filtration. The filtrate was successively washed with water and 5% hydrochloric acid. The organic phase was dried with anhydrous magnesium sulfate, filtered, and the filtrate was concentrated. The residue was purified by column chromatography to obtain the compound (9Z,12Z)-9,12-diene octadecyl acrylate (**S2-11,** 0.72 g).
Step g: **S2-8** (0.69 g, 1.0 mmol) was dissolved in isopropanol, and an excess of anhydrous potassium carbonate was added under stirring. The reaction solution was stirred at room temperature until the reaction solution became alkaline. **S2-11** (0.48 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E2-1** (0.76 g). ¹H NMR (400 MHz, CDCl₃) δ: 5.80-5.72 (m, 2H, -C(=O)OCH₂C*H*=CH-), 5.60-5.51 (m, 2H, -C(=O)OCH₂CH=C*H*-), 5.40-5.31 (m, 4H, -C*H*=C*H*CH₂C*H*=C*H*-), 4.94-4.87 (m, 1H, *-C(=O)OCH<),* 4.51 (d, 4H, -C(=O)OC*H*₂CH=CH-), 4.20 (s, 2H, pip-CH₂C*H*₂OC(=O)-), 3.18 (s, 2H, pip-C*H*₂C(=O)O-), 2.77 (t, 2H, -CH=CHC*H*₂CH=CH-), 2.73-2.40 (m, 10H; 8H, pip-*H*; 2H, pip-C*H*₂CH₂-), 2.32-2.27 (m, 6H, -CH₂C*H*₂C(=O)O-), 2.07-2.02 (m, 8H, -CH=CHC*H*₂CH₂-), 1.63-1.22 (m, 56H, -CH₂C*H*₂CH₂-, -C*H*₂CH₃), 0.88 (t, 9H, -CH₂C*H*₃). MS (ESI): m/z=997.7 ([M+H]⁺).

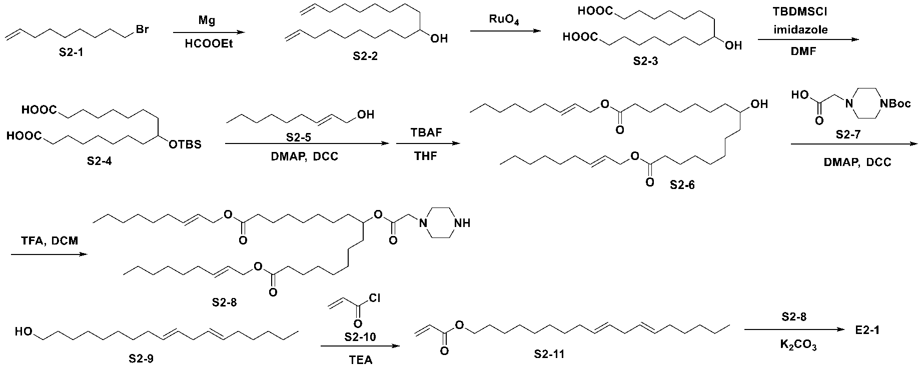

### Example 2.2: Cationic Lipid (E2-2)

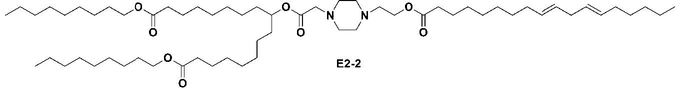

The preparation process is as follows:
Step a: Under nitrogen atmosphere, **DCC** (3.71 g, 18.0 mmol) was added to a round-bottom flask containing 1-nonenol (**S2-12,** 1.44 g, 10.0 mmol), **S2-4** (1.72 g, 4.0 mmol), and **DMAP** (0.24 g, 2.0 mmol) dissolved in dichloromethane (80 mL), and then the reaction was carried out at room temperature for 16 hours. After the reaction was completed, the precipitate was removed by filtration, and the filtrate was concentrated. The residue was dissolved in tetrahydrofuran (20 mL), and then 20 mL of a 1 M TBAF solution in tetrahydrofuran was added, and the reaction was allowed to proceed overnight to remove the TBS protecting group. After the reaction was completed, the solution was concentrated, extracted, and the organic phase was combined, dried with anhydrous magnesium sulfate, filtered, and the filtrate was concentrated to obtain the crude product of S2-13, and then purified by column chromatography to obtain compound **S2-13** (1.73 g).
Step b: Under nitrogen atmosphere, **DCC** (0.91 g, 4.4 mmol) was added to a round-bottom flask containing **S2-13** (1.37 g, 2.4 mmol), **S2-7** (0.49 g, 2.0 mmol), and **DMAP** (0.06 g, 0.5 mmol) dissolved in dichloromethane (30 mL). The reaction was carried out at room temperature for 16 hours. After the reaction was completed, the precipitate was removed by filtration, and the filtrate was concentrated to obtain the crude product. The crude product was dissolved in dichloromethane, 0.1 M TFA was added, and the reaction was allowed to proceed for 4 hours, and then the reaction solution was adjusted to neutral pH, concentrated, diluted with purified water, and extracted with dichloromethane. The organic phase was dried with anhydrous magnesium sulfate, filtered, and the filtrate was concentrated. The crude product was purified by recrystallization to obtain compound **S2-14** (1.04 g).
Step c: **S2-14** (0.69 g, 1.0 mmol) was dissolved in isopropanol, and an excess of anhydrous potassium carbonate was added under stirring. The reaction solution was stirred at room temperature until the reaction solution became alkaline. **S2-11** (0.48 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E2-2** (0.77 g). ¹H NMR (400 MHz, CDCl₃) δ: 5.41-5.31 (m, 4H, -C*H*=C*H*CH₂C*H*=C*H*-), 4.95-4.87 (m, 1H, -C(=O)OC*H*<), 4.20 (t, 2H, pip-CH₂C*H*₂OC(=O)-), 4.06 (t, 4H, -C(=O)OC*H*₂CH₂CH₂-), 3.17 (s, 2H, pip-C*H*₂C(=O)O-), 2.77 (t, 2H, -CH=CHC*H*₂CH=CH-), 2.73-2.41 (m, 10H; 8H, pip-*H*; 2H, pip-C*H*₂CH₂-), 2.32-2.27 (m, 6H, -CH₂C*H*₂C(=O)O-), 2.07-2.02 (m, 4H, -CH=CHC*H*₂CH₂-), 1.65-1.21 (m, 68H, -CH₂C*H*₂CH₂-, -C*H*₂CH₃), 0.87 (t, 9H, -CH₂C*H*₃). MS (ESI): m/z=1000.8 ([M+H]⁺).

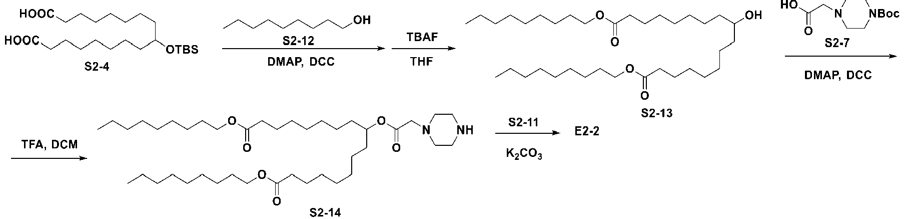

### Example 3: Cationic Lipid (E3-1)

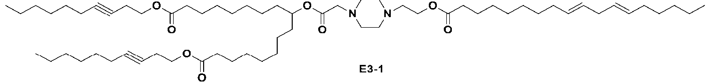

The preparation process is as follows:
Step a: Under nitrogen atmosphere, **DCC** (3.71 g, 18.0 mmol) was added to a round-bottom flask containing 3-decyn-1-ol (**S3-1,** 1.54 g, 10.0 mmol), **S2-4** (1.72 g, 4.0 mmol), and **DMAP** (0.24 g, 2.0 mmol) dissolved in dichloromethane (80 mL), then the reaction was carried out at room temperature for 16 hours. After the reaction was completed, the precipitate was removed by filtration, and the filtrate was concentrated. The residue was dissolved in tetrahydrofuran (20 mL), and then 20 mL of a 1 M TBAF solution in tetrahydrofuran was added, and the reaction was allowed to proceed overnight to remove the TBS protecting group. After the reaction was completed, the solution was concentrated, extracted, and the organic phase was combined, dried with anhydrous magnesium sulfate, filtered, and the filtrate was concentrated to obtain the crude product of S3-2, and then purified by column chromatography to obtain compound **S3-2** (1.75 g).
Step b: Under nitrogen atmosphere, **DCC** (0.91 g, 4.4 mmol) was added to a round-bottom flask containing **S3-2** (1.41 g, 2.4 mmol), **S2-7** (0.49 g, 2.0 mmol), and **DMAP** (0.06 g, 0.5 mmol) dissolved in dichloromethane (30 mL), and then the reaction was carried out at room temperature for 16 hours. After the reaction was completed, the precipitate was removed by filtration, and the filtrate was concentrated to obtain the crude product. The crude product was dissolved in dichloromethane, 0.1 M TFA was added, and the reaction was allowed to proceed for 4 hours. Then the reaction solution was adjusted to neutral pH, concentrated, diluted with purified water, and extracted with dichloromethane. The organic phase was dried with anhydrous magnesium sulfate, filtered, and the filtrate was concentrated. The crude product was purified by recrystallization to obtain compound **S3-3** (1.07 g).
Step c: **S3-3** (0.72 g, 1.0 mmol) was dissolved in isopropanol, and an excess of anhydrous potassium carbonate was added under stirring. The reaction solution was stirred at room temperature until the reaction solution became alkaline. **S2-11** (0.48 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E3-1** (0.78 g). ¹H NMR (400 MHz, CDCl₃) δ: 5.40-5.32 (m, 4H, -C*H*=C*H*CH₂C*H*=C*H*-), 4.90-4.79 (m, 1H, *-C(=O)OCH<),* 4.20 (s, 2H, pip-CH₂C*H*₂OC(=O)-), 4.12 (t, 4H, -C(=O)OC*H*₂CH₂C≡C-), 3.17 (s, 2H, pip-C*H*₂C(=O)O-), 2.77 (t, 2H, -CH=CHC*H*₂CH=CH-), 2.74-2.40 (m, 14H; 8H, pip-*H*; 2H, pip-C*H*₂CH₂-; 4H, C(=O)OCH₂C*H*₂C≡C-), 2.32-2.27 (m, 6H, -CH₂C*H*₂C(=O)O-), 2.16-2.08 (m, 4H, -C≡CC*H*₂(CH₂)₄-), 2.07-2.02 (m, 4H, -CH=CHC*H*₂CH₂-), 1.65-1.21 (m, 56H, -CH₂C*H*₂CH₂-, -C*H*₂CH₃), 0.87 (t, 9H, -CH₂C*H*₃). MS (ESI): m/z=1020.8 ([M+H]⁺).

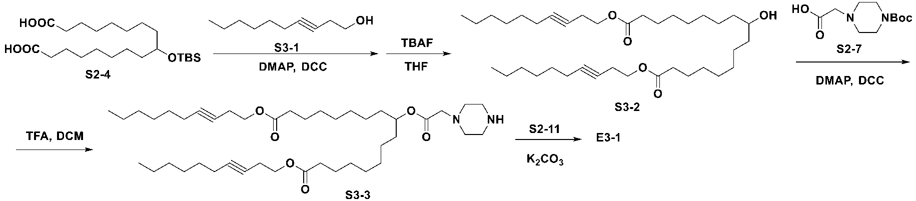

### Example 4: Cationic Lipid (E4-1)

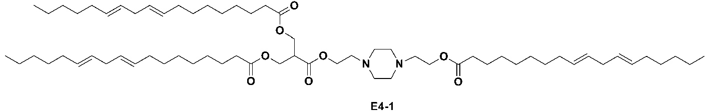

The preparation process is as follows:
Step a: Under ice bath conditions, **S4-1** (0.60 g, 5.0 mmol) was dissolved in dichloromethane, followed by **DCC** (1.13 g, 5.5 mmol) and **DMAP** (0.31 g, 2.5 mmol). After stirring the mixture for 10 minutes, ***t*-BuOH** (0.66 g, 7.5 mmol) was slowly added, and the reaction solution was stirred at room temperature overnight. After the reaction was completed, the reaction solution was filtered through diatomite, washed twice with ether and once with saturated brine. The organic phase was combined, dried with anhydrous magnesium sulfate, filtered, and concentrated to obtain the crude product of S4-2, and then purified by column chromatography to obtain **S4-2** (0.80 g, 91.1%).
Step b: Under nitrogen atmosphere, **DCC** (2.78 g, 13.5 mmol) was added to a round-bottom flask containing **S4-2** (0.53 g, 3.0 mmol), **S1-8** (2.10 g, 7.5 mmol), and **DMAP** (0.18 g, 1.5 mmol) dissolved in dichloromethane (60 mL), and then the reaction was carried out at room temperature for 16 hours. After the reaction was completed, the precipitate was removed by filtration, and the filtrate was concentrated to obtain the crude product. In a dry and clean round-bottom flask, a solution of trifluoroacetic acid/dichloromethane (1:2, v/v) was prepared, and the dichloromethane solution of the crude product was slowly added under ice bath conditions, and then the reaction was carried out at room temperature for 2 hours. After the reaction was completed, the reaction solution was concentrated, diluted with purified water, extracted with dichloromethane, dried with anhydrous magnesium sulfate, filtered, and the filtrate was concentrated. The crude product was purified by recrystallization to obtain compound **S4-3** (1.58 g).
Step c: Under nitrogen atmosphere, **DCC** (0.91 g, 4.4 mmol) was added to a round-bottom flask containing *t*-butyl-4-(2-hydroxyethyl)piperazine-1-carboxylate (**S4-4,** 0.55 g, 2.4 mmol), **S4-3** (1.29 g, 2.0 mmol), and **DMAP** (0.06 g, 0.5 mmol) dissolved in dichloromethane (30 mL), and then the reaction was carried out at room temperature for 16 hours. After the reaction was completed, the precipitate was removed by filtration, and the filtrate was concentrated to obtain the crude product. The crude product was dissolved in dichloromethane, 0.1 M TFA was added, and the reaction was allowed to proceed for 4 hours, and then the reaction solution was adjusted to neutral pH, concentrated, diluted with purified water, and extracted with dichloromethane. The organic phase was dried with anhydrous magnesium sulfate, filtered, and the filtrate was concentrated. The crude product was purified by recrystallization to obtain compound **S4-5** (1.14 g).
Step d: **S4-5** (0.76 g, 1.0 mmol) was dissolved in isopropanol, and an excess of anhydrous potassium carbonate was added under stirring, and then the reaction solution was stirred at room temperature until the reaction solution became alkaline. **S2-11** (0.48 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E4-1** (0.81 g). ¹H NMR (400 MHz, CDCl₃) δ: 5.40-5.31 (m, 12H, -C*H*=C*H*CH₂CH=C*H*-), 4.39-4.29 (m, 4H, >CH(C*H*₂OC(=O)₂-), 4.22 (t, 4H, pip-CH₂C*H*₂OC(=O)-), 3.02 (q, 1H, >C*H*C(=O)OCH₂-), 2.77 (t, 6H, -CH=CHC*H*₂CH=CH-), 2.73-2.40 (m, 12H; 8H, pip-*H*; 4H, pip-C*H*₂CH₂-), 2.32-2.27 (m, 6H, -CH₂C*H*₂C(=O)O-), 2.07-2.02 (m, 12H, -CH=CHC*H*₂CH₂-), 1.65-1.21 (m, 48H, -CH₂C*H*₂CH₂-, -C*H*₂CH₃), 0.87 (t, 9H, -CH₂C*H*₃). MS (ESI): m/z=1062.9 ([M+H]⁺).

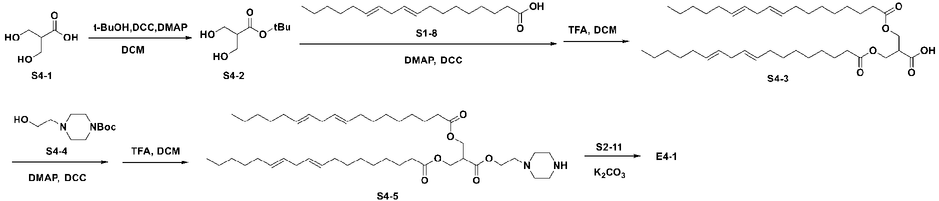

### Example 5: Cationic Lipid (E5-1)

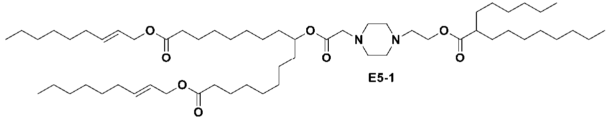

The preparation process is as follows:
Step a: Under nitrogen atmosphere, **DCC** (1.36 g, 6.6 mmol) was added to a round-bottom flask containing 2-hexyldecanoic acid (**S5-2,** 0.77 g, 3.0 mmol), **S5-1** (0.88 g, 3.6 mmol, S5-1 is obtained by reacting *t*-BuOH with the carboxyl end of [4-(2-hydroxyethyl)-1-piperazinyl]acetic acid), and **DMAP** (0.09 g, 0.8 mmol) dissolved in dichloromethane (30 mL), and then the reaction was carried out at room temperature for 16 hours. After the reaction was completed, the precipitate was removed by filtration, and the filtrate was concentrated to obtain the crude product. In a dry and clean round-bottom flask, a solution of trifluoroacetic acid/dichloromethane (1:2, v/v) was prepared, then the dichloromethane solution of the crude product was slowly added under ice bath conditions, and the reaction was carried out at room temperature for 2 hours. After the reaction was completed, the reaction solution was concentrated, diluted with purified water, extracted with dichloromethane, and the organic phase was dried with anhydrous magnesium sulfate, filtered, and then the filtrate was concentrated. The crude product was purified by recrystallization to obtain compound **S5-3** (1.01 g).
Step b: Under nitrogen atmosphere, **DCC** (0.91 g, 4.4 mmol) was added to a round-bottom flask containing **S2-6** (1.36 g, 2.4 mmol), **S5-3** (0.85 g, 2.0 mmol), and **DMAP** (0.06 g, 0.5 mmol) dissolved in dichloromethane (30 mL),and then the reaction was carried out at room temperature for 16 hours. After the reaction was completed, the precipitate was removed by filtration, and the filtrate was concentrated. The crude product was purified by column chromatography to obtain the cationic lipid **E5-1** (1.59 g). ¹H NMR (400 MHz, CDCl₃) δ: 5.80-5.72 (m, 2H, -C(=O)OCH₂C*H*=CH-), 5.60-5.51 (m, 2H, -C(=O)OCH₂CH=C*H*-), 4.94-4.88 (m, 1H, *-C(=O)OCH<),* 4.51 (d, 4H, -C(=O)OC*H*₂CH=CH-), 4.21 (t, 2H, pip-CH₂C*H*₂-), 3.18 (s, 2H, pip-C*H*₂C(=O)O-), 2.72-2.40 (m, 11H; 8H, pip-*H*; 2H, pip-C*H*₂CH₂-; 1H, >C*H*C(=O)O-), 2.32-2.27 (m, 4H, -CH₂C*H*₂C(=O)O-), 2.07-2.02 (m, 4H, -CH=CHC*H*₂CH₂-), 1.65-1.21 (m, 64H, -CH₂C*H*₂CH₂-, -C*H*₂CH₃), 0.87 (t, 12H, -CH₂C*H*₃). MS (ESI): m/z=972.9 ([M+H]⁺).

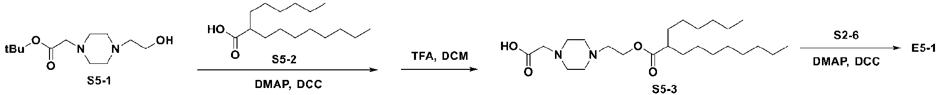

### Example 6: Cationic Lipid (E6-1)

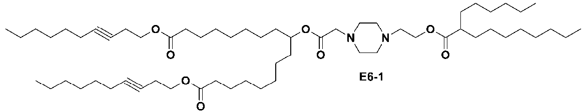

The preparation process is as follows:
Under nitrogen atmosphere, **DCC** (0.91 g, 4.4 mmol) was added to a round-bottom flask containing **S3-2** (1.41 g, 2.4 mmol), **S5-3** (0.85 g, 2.0 mmol), and **DMAP** (0.06 g, 0.5 mmol) dissolved in dichloromethane (30 mL), and then the reaction was carried out at room temperature for 16 hours. After the reaction was completed, the precipitate was removed by filtration, and the filtrate was concentrated. The crude product was purified by column chromatography to obtain the cationic lipid **E6-1** (1.64 g). ¹H NMR (400 MHz, CDCl₃) δ: 4.90-4.79 (m, 1H, *-C(=O)OCH<),* 4.19 (t, 2H, pip-CH₂C*H*₂-), 4.12 (t, 4H, -C(=O)OC*H*₂CH₂C≡C-), 3.18 (s, 2H, pip-C*H*₂C(=O)O-), 2.73-2.40 (m, 15H; 8H, pip-*H*; 2H, pip-C*H*₂CH₂-; 1H, >C*H*C(=O)O-; 4H, -C(=O)OCH₂CH₂C≡C-), 2.32-2.27 (m, 4H, -CH₂C*H*₂C(=O)O-), 2.16-2.08 (m, 4H, -C≡CC*H*₂(CH₂)₄-), 1.65-1.21 (m, 64H, -CH₂C*H*₂CH₂-, -C*H*₂CH₃), 0.87 (t, 12H, -CH₂C*H*₃). MS (ESI): m/z=996.9 ([M+H]⁺).

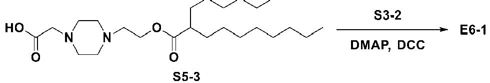

### Example 7.1: Cationic Lipid (E7-1)

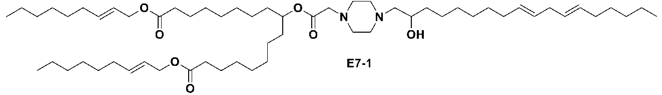

The preparation process is as follows:
Step a: Under ice bath conditions, (9Z,12Z)-octadeca-9,12-dienal (**S7-1,** 2.64 g, 10.0 mmol), **DL-proline** (0.35 g, 3.0 mmol), and N-chlorosuccinimide were added to acetonitrile (40 mL), and the solution was stirred at 0°C for 2 hours. After the reaction was completed, the reaction solution was diluted with anhydrous ethanol (30 mL), and **sodium borohydride** (0.53 g, 14.0 mmol) was added, then stirred at 0°C for 4 hours. After the reaction was completed, water (10 mL) was added to quench the reaction, and the solution was extracted with methyl tert-butyl ether. The combined organic phase was washed with saturated brine, dried with anhydrous magnesium sulfate, filtered, and the filtrate was concentrated to obtain the target product (9Z,12Z)-2-chlorooctadeca-9,12-dien-1-ol (**S7-2,** 2.82 g), and then directly in the next step.
Step b: At room temperature, **S7-2** (2.41 g, 8.0 mmol) and a NaOH aqueous solution (5 M, 32 mL) were added to 1,4-dioxane (30 mL). After completion of the addition,the solution was stirred at 35°C for 2 hours. After the reaction was completed, the reaction solution was separated using a separatory funnel, washed with saturated brine, dried with anhydrous magnesium sulfate, filtered, and evaporated to concentrate, and then purified by column chromatography to obtain the target product **S7-3** (1.56 g).
Step c: **S2-8** (1.54 g, 2.0 mmol) and **DIPEA** (0.15 g, 1.2 mmol) were sequentially added to a methanol solution of **S7-3** (0.79 g, 3.0 mmol), and then the reaction solution was refluxed at 90°C for 24 hours. After the reaction was completed, the reaction solution was concentrated, and the crude product was purified by column chromatography to obtain the cationic lipid **E7-1** (1.39 g). ¹H NMR (400 MHz, CDCl₃) δ: 5.80-5.72 (m, 2H, -C(=O)OCH₂C*H*=CH-), 5.60-5.51 (m, 2H, -C(=O)OCH₂CH=C*H*-), 5.40-5.31 (m, 4H, -C*H*=C*H*CH₂C*H*=C*H*-), 4.94-4.87 (m, 1H, -C(=O)OC*H*<), 4.51 (d, 4H, -C(=O)OC*H*₂CH=CH-), 3.68-3.60 (m, 1H, -C*H*(OH)-), 3.18 (s, 2H, pip-C*H*₂C(=O)O-), 2.77 (t, 2H, -CH=CHC*H*₂CH=CH-), 2.59-2.35 (m, 12H; 8H, pip-*H*; 2H, pip-C*H*₂CH<), 2.32-2.27 (m, 4H, -CH₂C*H*₂C(=O)O-), 2.07-2.02 (m, 8H, -CH=CHC*H*₂CH₂-), 1.65-1.21 (m, 56H, -CH₂C*H*₂CH₂-, -C*H*₂CH₃), 0.87 (t, 9H, -CH₂C*H*₃). MS (ESI): m/z=954.8 ([M+H]⁺).

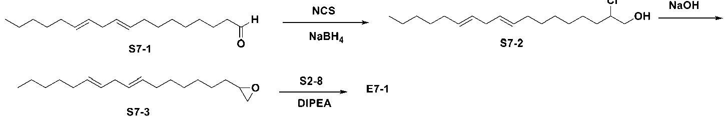

### Example 7.2: Cationic Lipid (E7-2)

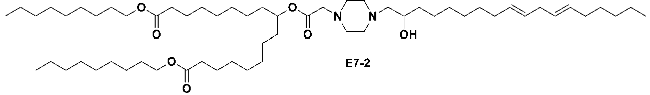

The preparation process is as follows:
**S2-14** (1.54 g, 2.0 mmol) and **DIPEA** (0.15 g, 1.2 mmol) were sequentially added to a methanol solution of **S7-3** (0.79 g, 3.0 mmol), then the reaction solution was refluxed at 90°C for 24 hours. After the reaction was completed, the reaction solution was concentrated, and the crude product was purified by column chromatography to obtain the cationic lipid **E7-2** (1.40 g). ¹H NMR (400 MHz, CDCl₃) δ: 5.39-5.31 (m, 4H, -C*H*=C*H*CH₂C*H*=C*H*-), 4.93-4.87 (m, 1H, -C(=O)OC*H*<), 4.04 (t, 4H, -C(=O)OC*H*₂CH₂-), 3.68-3.60 (m, 1H, -C*H*(OH)-), 3.18 (s, 2H, pip-C*H*₂C(=O)O-), 2.77 (t, 2H, -CH=CHC*H*₂CH=CH-), 2.59-2.35 (m, 10H; 8H, pip-*H*; 2H, pip-C*H*₂CH₂-), 2.32-2.27 (m, 4H, -CH₂C*H*₂C(=O)O-), 2.07-2.02 (m, 4H, -CH=CHC*H*₂CH₂-), 1.65-1.21 (m, 68H, -CH₂C*H*₂CH₂-, -C*H*₂CH₃), 0.87 (t, 9H, -CH₂C*H*₃). MS (ESI): m/z=958.9 ([M+H]⁺).

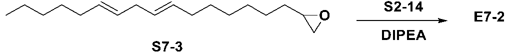

### Example 8: Cationic Lipid (E8-1)

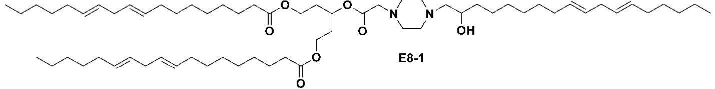

The preparation process is as follows:
Step a: Under ice bath conditions, *t*-butyldimethylsilyl chloride (**TBDMSCl**, 2.72 g, 18.0 mmol) and **imidazole** (2.04 g, 30.0 mmol) were added sequentially to a round-bottom flask containing 3-hydroxypentanedioic acid diethyl ester (**S8-1,** 2.64 g, 15.0 mmol) dissolved in dichloromethane (80 mL), and then the reaction solution was warmed to room temperature and stirred for 17 hours. After the reaction was completed, the solution was filtered and concentrated. The crude product was purified by column chromatography to obtain compound **S8-2** (2.96 g).
Step b: Under ice bath conditions, a solution of diisobutylaluminum hydride **(DIBAL-H)** in THF (1M, 7.2 mL, 7.2 mmol) was added to a round-bottom flask containing **S8-2** (2.61 g, 9.0 mmol) dissolved in dichloromethane (30 mL), and then the reaction solution was warmed to room temperature and stirred for 17 hours. After the reaction was completed, the reaction was quenched with MeOH, filtered, and concentrated. The crude product was purified by column chromatography to obtain compound **S8-3** (1.18 g).
Step c: Under nitrogen atmosphere, **DCC** (3.71 g, 18.0 mmol) was added to a round-bottom flask containing linoleic acid (**S1-8,** 2.80 g, 10.0 mmol), **S8-3** (0.94 g, 4.0 mmol), and **DMAP** (0.24 g, 2.0 mmol) dissolved in dichloromethane (50 mL), and then the reaction was carried out at room temperature for 16 hours. After the reaction was completed, the precipitate was removed by filtration, and the filtrate was concentrated. The residue was dissolved in tetrahydrofuran (20 mL), and then 20 mL of a 1 M TBAF solution in tetrahydrofuran was added, and the reaction was allowed to proceed overnight to remove the TBS protecting group. After the reaction was completed, the solution was concentrated, extracted, and the organic phase was combined, dried with anhydrous magnesium sulfate, filtered, and the filtrate was concentrated to obtain the crude product of S8-4, and then purified by column chromatography to yield compound **S8-4** (1.99 g).
Step d: Under nitrogen atmosphere, **DCC** (0.91 g, 4.4 mmol) was added to a round-bottom flask containing **S8-4** (1.55 g, 2.4 mmol), **S2-7** (0.49 g, 2.0 mmol), and **DMAP** (0.06 g, 0.5 mmol) dissolved in dichloromethane (30 mL), and then the reaction was carried out at room temperature for 16 hours. After the reaction was completed, the precipitate was removed by filtration, and the filtrate was concentrated to obtain the crude product. The crude product was dissolved in dichloromethane, 0.1 M TFA was added, and the reaction was allowed to proceed for 4 hours, and then the reaction solution was adjusted to neutral pH, concentrated, diluted with purified water, and extracted with dichloromethane. The organic phase was dried with anhydrous magnesium sulfate, filtered, and the filtrate was concentrated. The crude product was purified by recrystallization to obtain compound **S8-5** (1.16 g).
Step e: **S8-5** (0.77 g, 1.0 mmol) and **DIPEA** (0.10 g, 0.8 mmol) were sequentially added to a methanol solution of **S7-3** (0.40 g, 1.5 mmol). The reaction solution was refluxed at 90°C for 24 hours. After the reaction was completed, the reaction solution was concentrated, and the crude product was purified by column chromatography to obtain the cationic lipid **E8-1** (0.75 g). ¹H NMR (400 MHz, CDCl₃) δ: 5.46-5.29 (m, 12H, *-CH=CH-),* 5.17-5.08 (m, 1H, *-C(=O)OCH<),* 4.19-4.00 (m, 4H, -C(=O)OC*H*₂-), 3.68-3.60 (m, 1H, -C*H*(OH)-), 3.19 (s, 2H, pip-C*H*₂C(=O)O-), 2.77 (t, 6H, -CH=CHC*H*₂CH=CH-), 2.59-2.35 (m, 10H; 8H, pip-*H*; 2H, pip-C*H*₂CH₂-), 2.32-2.27 (m, 4H, -CH₂C*H*₂C(=O)O-), 2.07-2.02 (m, 12H, -CH=CHC*H*₂CH₂-), 1.65-1.21 (m, 52H, -CH₂C*H*₂CH₂-, -C*H*₂CH₃), 0.87 (t, 9H, -CH₂C*H*₃). MS (ESI): m/z=1034.9 ([M+H]⁺).

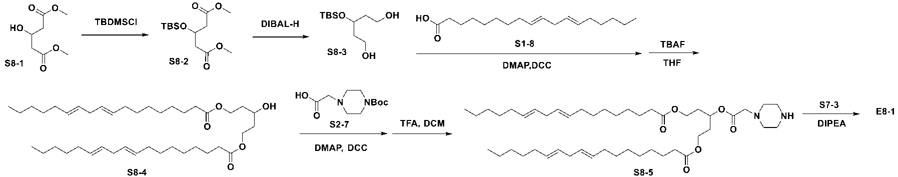

### Example 9: Cationic Lipid (E9-1)

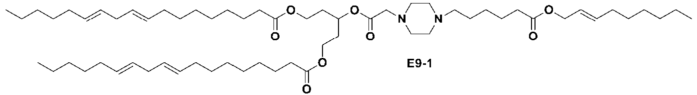

The preparation process is as follows:
Step a: Under nitrogen atmosphere, **DCC** (0.91 g, 4.4 mmol) was added to a round-bottom flask containing **S2-5** (0.68 g, 4.8 mmol), **S1-4** (0.78 g, 4.0 mmol), and **DMAP** (0.06 g, 0.5 mmol) dissolved in dichloromethane (30 mL), and then the reaction was carried out at room temperature for 16 hours. After the reaction was completed, the precipitate was removed by filtration, and the filtrate was concentrated. The crude product was purified by column chromatography to obtain **S9-1** (1.05 g).
Step b: Under nitrogen atmosphere, compound **S8-5** (1.54 g, 2.0 mmol) was dissolved in acetonitrile (30 mL), then **S9-1** (0.83 g, 2.6 mmol) and **DIPEA** (0.34 g, 2.6 mmol) were added sequentially under slow stirring, and the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E9-1** (1.66 g). ¹H NMR (400 MHz, CDCl₃) δ: 5.80-5.72 (m, 1H, -C(=O)OCH₂C*H*=CH-), 5.60-5.51 (m, 1H, -C(=O)OCH₂CH=C*H*-), 5.46-5.29 (m, 8H, *-CH=CH-),* 5.17-5.08 (m, 1H, *-C(=O)OCH<),* 4.51 (d, 2H, -C(=O)OC*H*₂CH=CH-), 4.19-4.00 (m, 4H, -C(=O)OC*H*₂-), 3.19 (s, 2H, pip-C*H*₂C(=O)O-), 2.77 (t, 4H, -CH=CHC*H*₂CH=CH-), 2.59-2.35 (m, 10H; 8H, pip-*H*; 2H, pip-C*H*₂CH₂-), 2.32-2.27 (m, 6H, -CH₂C*H*₂C(=O)O-), 2.07-2.02 (m, 10H, -CH=CHC*H*₂CH₂-), 1.65-1.21 (m, 50H, -CH₂C*H*₂CH₂-, -C*H*₂CH₃), 0.87 (t, 9H, -CH₂C*H*₃). MS (ESI): m/z=1008.8 ([M+H]⁺).

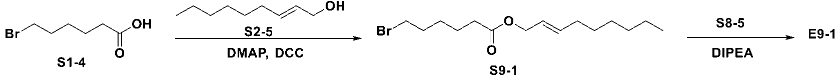

### Example 10: Cationic Lipid (E10-1)

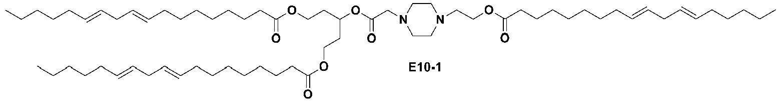

The preparation process is as follows:
**S8-5** (1.54 g, 2.0 mmol) was dissolved in isopropanol, and an excess of anhydrous potassium carbonate was added under stirring, then the reaction solution was stirred at room temperature until the reaction solution became alkaline. **S2-11** (0.79 g, 3.0 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E10-1** (1.70 g). ¹H NMR (400 MHz, CDCl₃) δ: 5.46-5.29 (m, 12H, *-CH=CH-),* 5.17-5.08 (m, 1H, *-C(=O)OCH<),* 4.20-4.00 (m, 6H, -C(=O)OC*H*₂-), 3.19 (s, 2H, pip-C*H*₂C(=O)O-), 2.77 (t, 6H, -CH=CHC*H*₂CH=CH-), 2.74-2.40 (m, 10H; 8H, pip-*H*; 2H, pip-C*H*₂CH₂-), 2.32-2.27 (m, 6H, -CH₂C*H*₂C(=O)O-), 2.07-2.02 (m, 12H, -CH=CHC*H*₂C*H*₂-), 1.65-1.21 (m, 52H, -CH₂C*H*₂CH₂-, -C*H*₂CH₃), 0.87 (t, 9H, -CH₂C*H*₃). MS (ESI): m/z=1076.9 ([M+H]⁺).

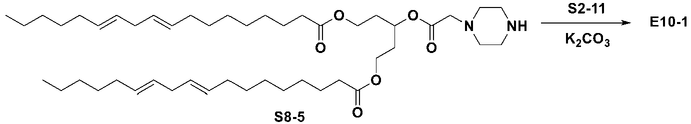

### Example 11: Cationic Lipid (E11-1)

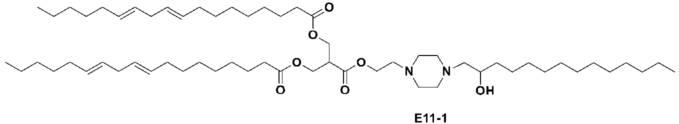

The preparation process is as follows:
**S4-5** (1.51 g, 2.0 mmol) and **DIPEA** (0.15 g, 1.2 mmol) were sequentially added to a methanol solution of 1,2-epoxytetradecane (**S11-1,** 0.64 g, 3.0 mmol), and then the reaction solution was refluxed at 90°C for 24 hours. After the reaction was completed, the reaction solution was concentrated, and the crude product was purified by column chromatography to obtain the cationic lipid **E11-1** (1.41 g). ¹H NMR (400 MHz, CDCl₃) δ: 5.46-5.29 (m, 8H, *-CH=CH-),* 4.39-4.29 (m, 4H, >CH(C*H*₂OC(=O)₂-), 4.19-4.00 (m, 2H, -C(=O)OC*H*₂-), 3.02 (q, 1H, >C*H*C(=O)OCH₂-), 3.70-3.61 (m, 1H, -CH(OH)-), 2.77 (t, 4H, -CH=CHC*H*₂CH=CH-), 2.59-2.35 (m, 12H; 8H, pip-*H*; 4H, pip-C*H*₂-), 2.32-2.27 (m, 4H, -CH₂C*H*₂C(=O)O-), 2.07-2.02 (m, 8H, -CH=CHC*H*₂CH₂-), 1.65-1.21 (m, 54H, -CH₂C*H*₂CH₂-, -C*H*₂CH₃), 0.87 (t, 9H, -CH₂C*H*₃). MS (ESI): m/z=968.9 ([M+H]⁺).

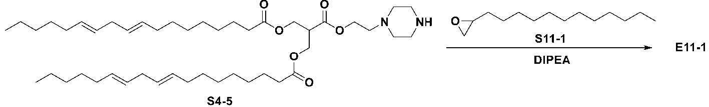

### Example 12: Cationic Lipid (E12-1)

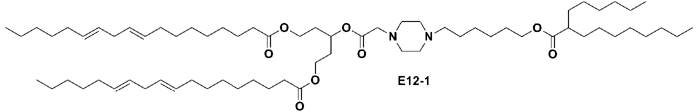

The preparation process is as follows:
Step a: Under nitrogen atmosphere, **DCC** (1.81 g, 8.8 mmol) was added to a round-bottom flask containing **S12-2** (0.87 g, 4.8 mmol), **S12-1** (1.02 g, 4.0 mmol), and **DMAP** (0.12 g, 1.0 mmol) dissolved in dichloromethane (50 mL), and then the reaction was carried out at room temperature for 16 hours. After the reaction was completed, the precipitate was removed by filtration, and the filtrate was concentrated. The crude product was purified by column chromatography to obtain **S12-3** (1.39 g).
Step b: Under nitrogen atmosphere, compound **S8-5** (1.54 g, 2.0 mmol) was dissolved in acetonitrile (30 mL), then **S12-3** (1.09 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HC1/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E12-1** (1.86 g). ¹H NMR (400 MHz, CDCl₃) δ: 5.46-5.29 (m, 8H, *-CH=CH-),* 5.17-5.08 (m, 1H, -C(=O)OC*H*<), 4.19-4.00 (m, 6H, -C(=O)OC*H*₂-), 3.19 (s, 2H, pip-C*H*₂C(=O)O-), 2.77 (t, 4H, -CH=CHC*H*₂CH=CH-), 2.73-2.40 (m, 11H; 8H, pip-*H*; 2H, pip-C*H*₂CH₂-; 1H, >C*H*C(=O)O-), 2.32-2.27 (m, 4H, -CH₂C*H*₂C(=O)O-), 2.07-2.02 (m, 8H, -CH=CHC*H*₂CH₂-), 1.65-1.21 (m, 68H, -CH₂C*H*₂CH₂-, -C*H*₂CH₃), 0.87 (t, 12H, -CH₂C*H*₃). MS (ESI): m/z=1109.0 ([M+H]⁺).

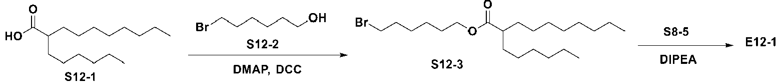

### Example 13: Cationic Lipid (E13-1)

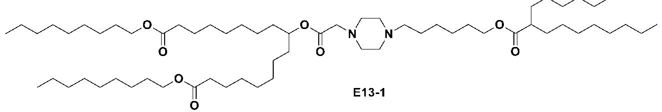

The preparation process is as follows:
Under nitrogen atmosphere, compound **S2-14** (1.39 g, 2.0 mmol) was dissolved in acetonitrile (30 mL), then **S12-3** (1.09 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E13-1** (1.71 g). ¹H NMR (400 MHz, CDCl₃) δ: 4.94-4.86 (m, 1H, *-C(=O)OCH<),* 4.05 (t, 6H, -C(=O)OC*H*₂CH₂-), 3.17 (s, 2H, pip-C*H*₂C(=O)O-), 2.73-2.41 (m, 11H; 8H, pip-*H*; 2H, pip-C*H*₂CH₂-; 1H, >C*H*C(=O)O-), 2.32-2.27 (m, 4H, -CH₂C*H*₂C(=O)O-), 1.65-1.21 (m, 84H, -CH₂C*H*₂CH₂-, -C*H*₂CH₃), 0.87 (t, 12H, -CH₂C*H*₃). MS (ESI): m/z=1033.0 ([M+H]⁺).

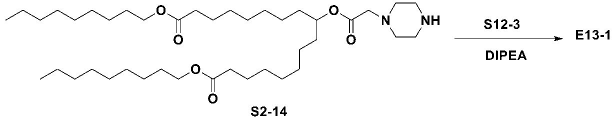

### Example 14: Cationic Lipid (E14-1)

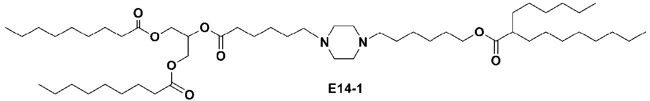

The preparation process is as follows:
Step a: Under nitrogen atmosphere, compound **S14-1** (0.56 g, 3.0 mmol) was dissolved in acetonitrile (30 mL), then **S1-5** (2.15 g, 3.9 mmol) and **DIPEA** (0.50 g, 3.9 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated to obtain the crude product. The crude product was dissolved in dichloromethane, 0.1 M TFA was added, and the reaction was allowed to proceed for 4 hours, and then the reaction solution was adjusted to neutral pH, concentrated, diluted with purified water, and extracted with dichloromethane. The organic phase was dried with anhydrous magnesium sulfate, filtered, and the filtrate was concentrated. The crude product was purified by recrystallization to obtain compound **S14-2** (1.44 g).
Step b: Under nitrogen atmosphere, compound **S14-2** (1.11 g, 2.0 mmol) was dissolved in acetonitrile (30 mL), then **S12-3** (1.09 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E14-1** (1.48 g). ¹H NMR (400 MHz, CDCl₃) δ: 5.37-5.31 (m, 1H, -C(=O)OC*H*<), 4.38-4.29 (m, 2H, >C*H*(CH₂OC(=O))₂-), 4.19-4.13 (m, 2H, >CH(C*H*₂OC(=O))₂-), 4.04 (t, 2H, -C(=O)OC*H*₂CH₂-), 2.71-2.40 (m, 13H; 8H, pip-*H*; 4H, pip-C*H*₂CH₂-; 1H, >C*H*C(=O)O-), 2.32-2.27 (m, 6H, -CH₂C*H*₂C(=O)O-), 1.65-1.21 (m, 62H, -CH₂C*H*₂CH₂-, -C*H*₂CH₃), 0.87 (t, 12H, -CH₂C*H*₃). MS (ESI): m/z=892.8 ([M+H]⁺).

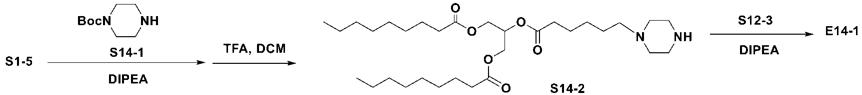

### Example 15.1: Cationic Lipid (E15-1)

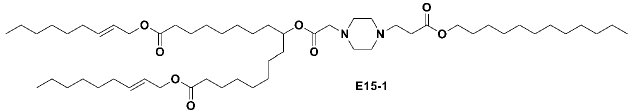

The preparation process is as follows:
**S2-8** (0.69 g, 1.0 mmol) was dissolved in isopropanol, and an excess of anhydrous potassium carbonate was added under stirring, and then the reaction solution was stirred at room temperature until the reaction solution became alkaline. Dodecyl acrylate (**S15-1,** 0.36 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E15-1** (0.79 g). ¹H NMR (400 MHz, CDCl₃) δ: 5.80-5.72 (m, 2H, -C(=O)OCH₂C*H*=CH-), 5.60-5.51 (m, 2H, -C(=O)OCH₂CH=C*H*-), 4.94-4.87 (m, 1H, *-C(=O)OCH<),* 4.51 (d, 4H, -C(=O)OC*H*₂CH=CH-), 4.05 (t, 2H, -C(=O)OC*H*₂CH₂-), 3.18 (s, 2H, pip-C*H*₂C(=O)O-), 2.71-2.41 (m, 10H; 8H, pip-*H*; 2H, pip-C*H*₂CH₂-), 2.37-2.25 (m, 6H, -CH₂C*H*₂C(=O)O-), 2.07-2.02 (m, 4H, -CH=CHC*H*₂CH₂-), 1.65-1.21 (m, 60H, -CH₂C*H*₂CH₂-, -C*H*₂CH₃), 0.87 (t, 9H, -CH₂C*H*₃). MS (ESI): m/z=931.9 ([M+H]⁺).

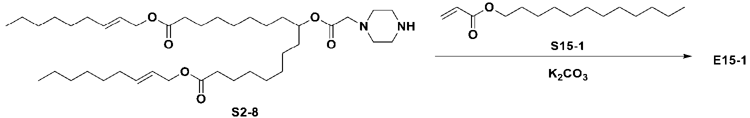

### Example 15.2: Cationic Lipid (E15-2)

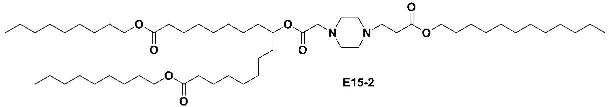

The preparation process is as follows:
**S2-14** (0.70 g, 1.0 mmol) was dissolved in isopropanol, and an excess of anhydrous potassium carbonate was added under stirring, and then the reaction solution was stirred at room temperature until the reaction solution became alkaline. **S15-1** (0.36 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E15-2** (0.81 g). ¹H NMR (400 MHz, CDCl₃) δ: 4.94-4.88 (m, 1H, -C(=O)OC*H*<), 4.07 (t, 6H, -C(=O)OC*H*₂CH₂-), 3.18 (s, 2H, pip-C*H*₂C(=O)O-), 2.71-2.41 (m, 10H; 8H, pip-*H*; 2H, pip-C*H*₂CH₂-), 2.37-2.25 (m, 6H, -CH₂C*H*₂C(=O)O-), 1.65-1.21 (m, 72H, -CH₂C*H*₂CH₂-, -C*H*₂CH₃), 0.87 (t, 9H, -CH₂C*H*₃). MS (ESI): m/z=934.8 ([M+H]⁺).

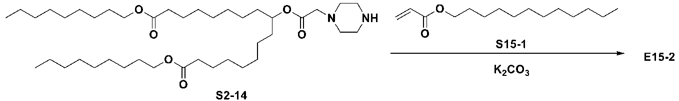

### Example 16: Cationic Lipid (E16-1)

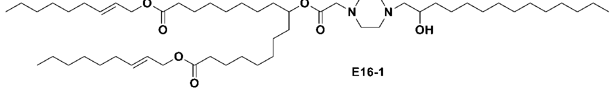

The preparation process is as follows:
**S2-8** (1.38 g, 2.0 mmol) and **DIPEA** (0.21 g, 1.6 mmol) were sequentially added to a methanol solution of **S11-1** (0.64 g, 3.0 mmol), and then the reaction solution was refluxed at 90°C for 24 hours. After the reaction was completed, the reaction solution was concentrated, and the crude product was purified by column chromatography to obtain the cationic lipid **E16-1** (1.32 g). ¹H NMR (400 MHz, CDCl₃) δ: 5.80-5.72 (m, 2H, -C(=O)OCH₂C*H*=CH-), 5.60-5.51 (m, 2H, -C(=O)OCH₂CH=C*H*-), 4.94-4.87 (m, 1H, *-C(=O)OCH<),* 4.51 (d, 4H, -C(=O)OC*H*₂CH=CH-), 3.70-3.61 (m, 1H, -C*H*(OH)-), 3.18 (s, 2H, pip-C*H*₂C(=O)O-), 2.59-2.35 (m, 10H; 8H, pip-*H*; 2H, pip-C*H*₂CH<), 2.37-2.25 (m, 4H, -CH₂C*H*₂C(=O)O-), 2.07-2.02 (m, 4H, -CH=CHC*H*₂CH₂-), 1.65-1.21 (m, 62H, -CH₂C*H*₂CH₂-, -C*H*₂CH₃), 0.87 (t, 9H, -CH₂C*H*₃). MS (ESI): m/z=902.8 ([M+H]⁺).

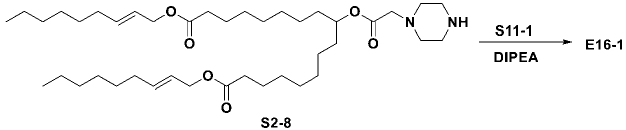

### Example 17.1: Cationic Lipid (E17-1)

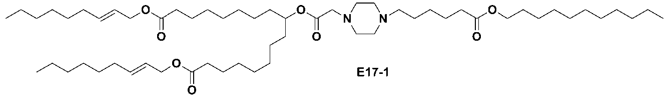

The preparation process is as follows:
Under nitrogen atmosphere, compound **S2-8** (1.38 g, 2.0 mmol) was dissolved in acetonitrile (30 mL), then **S17-1** (0.91 g, 2.6 mmol, **S17-1** is prepared by the reaction of undecanol 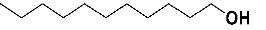 and 6-bromohexanoic acid 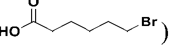 and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E17-1** (1.57 g). ¹H NMR (400 MHz, CDCl₃) δ: 5.80-5.72 (m, 2H, -C(=O)OCH₂C*H*=CH-), 5.60-5.51 (m, 2H, -C(=O)OCH₂CH=C*H*-), 4.94-4.86 (m, 1H, *-C(=O)OCH<),* 4.51 (d, 4H, -C(=O)OC*H*₂CH=CH-), 4.05 (t, 2H, -C(=O)OC*H*₂CH₂-), 3.18 (s, 2H, pip-C*H*₂C(=O)O-), 2.71-2.41 (m, 10H; 8H, pip-*H*; 2H, pip-C*H*₂CH₂-), 2.37-2.25 (m, 6H, -CH₂C*H*₂C(=O)O-), 2.07-2.02 (m, 4H, -CH=CHC*H*₂CH₂-), 1.65-1.21 (m, 64H, -CH₂C*H*₂CH₂-, -C*H*₂CH₃), 0.87 (t, 9H, -CH₂C*H*₃). MS (ESI): m/z=958.8 ([M+H]⁺).

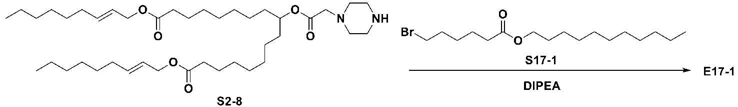

### Example 17.2: Cationic Lipid (E17-2)

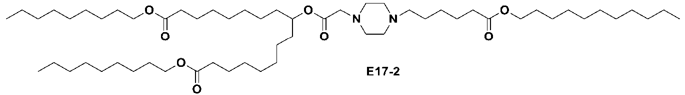

The preparation process is as follows:
Under nitrogen atmosphere, compound **S2-14** (1.39 g, 2.0 mmol) was dissolved in acetonitrile (30 mL), then **S17-1** (0.91 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E17-2** (1.60 g). ¹H NMR (400 MHz, CDCl₃) δ: 4.94-4.87 (m, 1H, *-C(=O)OCH<),* 4.05 (t, 6H, -C(=O)OC*H*₂CH₂-), 3.17 (s, 2H, pip-C*H*₂C(=O)O-), 2.70-2.42 (m, 8H, pip-*H*), 2.37-2.25 (m, 8H; 2H, pip-C*H*₂CH₂-; 6H, -CH₂C*H*₂C(=O)O-), 1.70-1.63 (m, 4H, >CH(C*H*₂)₂-), 1.56-1.48 (m, 6H, -C(=O)OC*H*₂CH₂-), 1.48-0.97 (m, 66H, -CH₂C*H*₂CH₂-, -C*H*₂CH₃), 0.89 (t, 9H, -CH₂C*H*₃). MS (ESI): m/z=963.7 ([M+H]⁺).

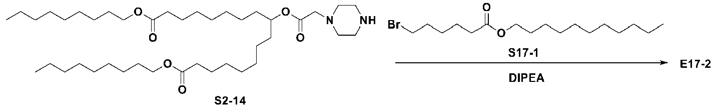

### Example 18: Cationic Lipid (E18-1)

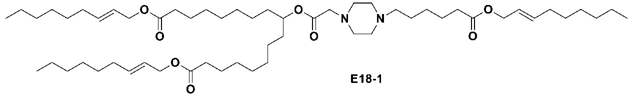

The preparation process is as follows:
Under nitrogen atmosphere, compound **S2-8** (1.38 g, 2.0 mmol) was dissolved in acetonitrile (30 mL), then **S9-1** (0.83 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E18-1** (1.53 g). ¹H NMR (400 MHz, CDCl₃) δ: 5.80-5.72 (m, 3H, -C(=O)OCH₂C*H*=CH-), 5.60-5.51 (m, 3H, -C(=O)OCH₂CH=C*H*-), 4.95-4.87 (m, 1H, -C(=O)OC*H*<)*,* 4.51 (d, 6H, -C(=O)OC*H*₂CH=CH-), 3.18 (s, 2H, pip-C*H*₂C(=O)O-), 2.71-2.41 (m, 10H; 8H, pip-*H*; 2H, pip-C*H*₂CH₂-), 2.37-2.25 (m, 6H, -CH₂C*H*₂C(=O)O-), 2.07-2.02 (m, 6H, -CH=CHC*H*₂CH₂-), 1.65-1.21 (m, 54H, -CH₂C*H*₂CH₂-, -C*H*₂CH₃), 0.87 (t, 9H, -CH₂C*H*₃). MS (ESI): m/z=928.8 ([M+H]⁺).

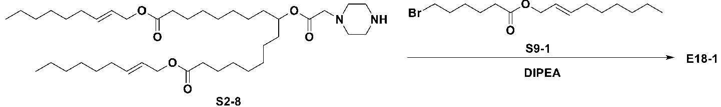

### Example 19: Cationic Lipid (E19-1)

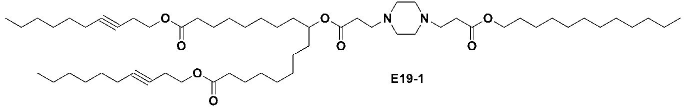

The preparation process is as follows:
**S3-3** (0.72 g, 1.0 mmol) was dissolved in isopropanol, and an excess of anhydrous potassium carbonate was added under stirring, and then the reaction solution was stirred at room temperature until the reaction solution became alkaline. **S15-1** (0.36 g, 1.5 mmol) was then added to the reaction solution, and the reaction solution was refluxed at 90°C for 36 hours. After the reaction was completed, the reaction solution was concentrated to obtain the crude product, and then purified by column chromatography to obtain the cationic lipid **E19-1** (0.82 g). ¹H NMR (400 MHz, CDCl₃) δ: 4.90-4.80 (m, 1H, -C(=O)OC*H*<), 4.13 (t, 4H, -C(=O)OC*H*₂CH₂C-C-), 4.04 (t, 2H, -C(=O)OC*H*₂CH₂CH₂-), 2.72-2.63 (m, 4H, -C(=O)OCH₂C*H*₂C^{s}C-), 2.62-2.36 (m, 16H; 8H, pip-*H*; 4H, pip-C*H*₂CH₂-; 4H, pip-CH₂C*H*₂-), 2.30 (t, 4H, -OC(=O)C*H*₂(CH₂)₆-), 2.17-2.08 (m, 4H, -C≡CC*H*₂(CH₂)₄-), 1.65-1.21 (m, 60H, -CH₂C*H*₂CH₂-, -C*H*₂CH₃), 0.87 (t, 9H, -CH₂C*H*₃). MS (ESI): m/z=969.7 ([M+H]⁺).

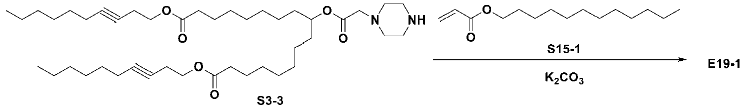

### Example 20: Cationic Lipid (E20-1)

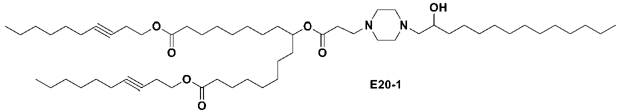

The preparation process is as follows:
**S3-3** (1.43 g, 2.0 mmol) and **DIPEA** (0.21 g, 1.6 mmol) were sequentially added to a methanol solution of **S11-1** (0.64 g, 3.0 mmol), and then the reaction solution was refluxed at 90°C for 24 hours. After the reaction was completed, the reaction solution was concentrated, and the crude product was purified by column chromatography to obtain the cationic lipid **E20-1** (1.37 g). ¹H NMR (400 MHz, CDCl₃) δ: 4.90-4.79 (m, 1H, *-C(=O)OCH<),* 4.12 (t, 4H, -C(=O)OC*H*₂CH₂C≡C-), 3.70-3.61 (m, 1H, -C*H*(OH)-), 2.70 (t, 4H, -C(=O)OC*H*₂CH₂C≡C-), 2.59-2.35 (m, 12H; 8H, pip-*H*; 4H, pip-C*H*₂CH₂-), 2.33-2.21 (m, 6H; -OC(=O)C*H*₂(CH₂)₆-, 4H; pip-CH₂C*H*₂-, 2H), 2.16-2.08 (m, 4H, -C≡CC*H*₂(CH₂)₄-), 1.64-1.20 (m, 62H, -CH₂C*H*₂CH₂-, -C*H*₂CH₃), 0.88 (t, 9H, -CH₂C*H*₃). MS (ESI): m/z=942.0 ([M+H]⁺).

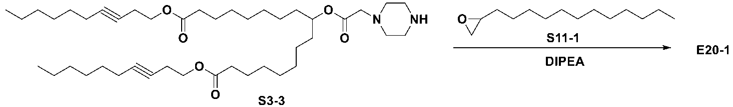

### Example 21: Cationic Lipid (E21-1)

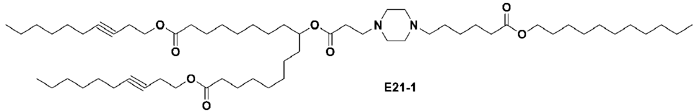

The preparation process is as follows:
Under nitrogen atmosphere, compound **S3-3** (1.43 g, 2.0 mmol) was dissolved in acetonitrile (30 mL), then **S17-1** (0.91 g, 2.6 mmol) and **DIPEA** (0.34 g, 2.6 mmol) were added sequentially under slow stirring, and then the reaction solution was stirred at room temperature for approximately 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E21-1** (1.66 g). ¹H NMR (400 MHz, CDCl₃) δ: 4.90-4.79 (m, 1H, *-C(=O)OCH<),* 4.12 (t, 4H, -C(=O)OC*H*₂CH₂C≡C-), 4.04 (t, 2H, -C(=O)OC*H*₂CH₂CH₂-), 2.72 (t, 4H, -C(=O)OCH₂C*H*₂C≡C-), 2.59-2.35 (m, 12H; 8H, pip-*H*; 4H, pip-C*H*₂CH₂-), 2.33-2.21 (m, 8H, -OC(=O)C*H*₂-), 2.16-2.08 (m, 4H, -C≡CC*H*₂(CH₂)₄-), 1.64-1.20 (m, 64H, -CH₂C*H*₂CH₂-, -C*H*₂CH₃), 0.88 (t, 9H, -CH₂C*H*₃). MS (ESI): m/z=996.9 ([M+H]⁺).

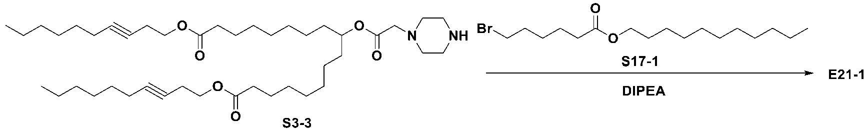

### Example 22: Cationic Lipid (E22-1)

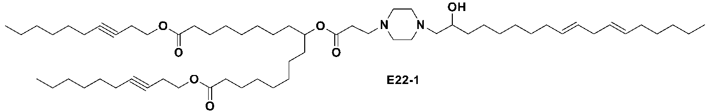

The preparation process is as follows:
**S3-3** (1.43 g, 2.0 mmol) and **DIPEA** (0.21 g, 1.6 mmol) were sequentially added to a methanol solution of **S7-3** (0.79 g, 3.0 mmol), and then the reaction solution was refluxed at 90°C for 24 hours. After the reaction was completed, the reaction solution was concentrated, and the crude product was purified by column chromatography to obtain the cationic lipid **E22-1** (1.45 g). ¹H NMR (400 MHz, CDCl₃) δ: 5.40-5.31 (m, 4H, -C*H*=C*H*CH₂C*H*=C*H*-)*,* 4.90-4.79 (m, 1H, *-*C(=O)OC*H*<)*,* 4.12 (t, 4H, -C(=O)OC*H*₂CH₂C≡C-), 3.70-3.61 (m, 1H, -C*H*(OH)-), 2.77 (t, 2H, *-*CH=CHC*H*₂CH=CH-)*,* 2.71 (t, 4H, -C(=O)OCH₂C*H*₂C≡C-), 2.59-2.35 (m, 12H; 8H, pip-*H*; 4H, pip-C*H*₂CH₂-), 2.33-2.21 (m, 6H, -OC(=O)C*H*₂-), 2.16-2.08 (m, 4H, -C≡CC*H*₂(CH₂)₄-), 2.07-2.02 (m, 4H, -CH=CHC*H*₂CH₂-), 1.64-1.20 (m, 56H, -CH₂C*H*₂CH₂-, -C*H*₂CH₃), 0.88 (t, 9H, -CH₂C*H*₃). MS (ESI): m/z=992.9 ([M+H]⁺).

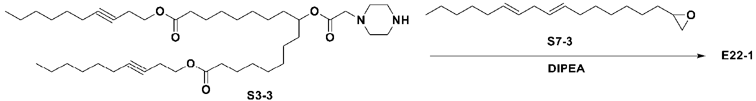

### Example 23: Cationic Lipid (E23-1)

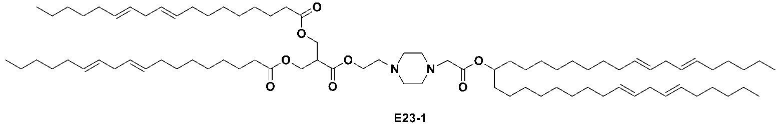

The preparation process is as follows:
Step a: freshly activated **magnesium turnings** (0.26 g, 11.0 mmol) and 2 mL of anhydrous ether were added to a clean round-bottom flask. Linolenyl bromide **(S23-1,** 2.92 g, 8.9 mmol) was dissolved in anhydrous ether (30 mL) and placed in a dropp funnel. Under ice bath conditions, the bromide ether solution was added dropwise to the magnesium turnings, after the addition was completed, the reaction solution was stirred at 35°C for 1 hour and then cooled in an ice bath. **Ethyl formate** (0.30 g, 4.0 mmol) was dissolved in anhydrous ether (3 mL) and transferred to the dropp funnel, and then added to the reaction solution under stirring. After the reaction solution began to reflux, the remaining ether solution of ethyl formate was quickly added, and then the reaction solution was stirred at room temperature for 1 hour. The reaction was quenched by the dropwise addition of acetone (3 mL) and ice water (10 mL). The reaction solution was treated with aqueous H₂SO₄ (10% v/v, 50 mL) until the solution became homogeneous and was allowed to stand for phase separation. The aqueous phase was extracted with ether (30 mL × 2), then the organic phases were combined and dried over anhydrous magnesium sulfate, filtered, and the filtrate was concentrated. The crude product was purified by column chromatography to obtain compound **S23-2** (1.93 g, 91.1%).
Step b: Under nitrogen atmosphere, **DCC** (0.91 g, 4.4 mmol) was added to a round-bottom flask containing **S23-2** (1.27 g, 2.4 mmol), bromoacetic acid **(S23-3,** 0.28 g, 2.0 mmol), and **DMAP** (0.06 g, 0.5 mmol) dissolved in dichloromethane (30 mL), then the reaction was carried out at room temperature for 16 hours. After the reaction was completed, the precipitate was removed by filtration, and the filtrate was concentrated. The crude product was purified by column chromatography to obtain **S23-4** (1.07 g).
Step c: Under nitrogen atmosphere, compound **S4-5** (0.76 g, 1.0 mmol) was dissolved in acetonitrile (30 mL), then **S23-4** (0.85 g, 1.3 mmol) and **DIPEA** (0.17 g, 1.3 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E23-1** (1.10 g). ¹H NMR (400 MHz, CDCl₃) δ: 5.40-5.31 (m, 16H, -C*H*=C*H*CH₂C*H*=C*H*-)*,* 4.94-4.88 (m, 1H, -C(=O)OC*H*<), 4.39-4.29 (m, 4H, >CH(C*H*₂OC(=O)₂-), 4.23 (t, 2H, pip-CH₂C*H*₂OC(=O)-), 3.17 (s, 2H, pip-C*H*₂C(=O)O-), 3.02 (q, 1H, >C*H*C(=O)OCH₂-), 2.77 (t, 8H, -CH=CHC*H*₂CH=CH-)*,* 2.59-2.35 (m, 10H; 8H, pip-*H*; 2H, pip-C*H*₂CH₂-), 2.33-2.21 (m, 4H, -OC(=O)C*H*₂-), 2.07-2.02 (m, 16H, -CH=CHC*H*₂CH₂-), 1.64-1.20 (m, 72H, -CH₂C*H*₂CH₂-, -C*H*₂CH₃), 0.88 (t, 12H, -CH₂C*H*₃). MS (ESI): m/z=1325.1 ([M+H]⁺).

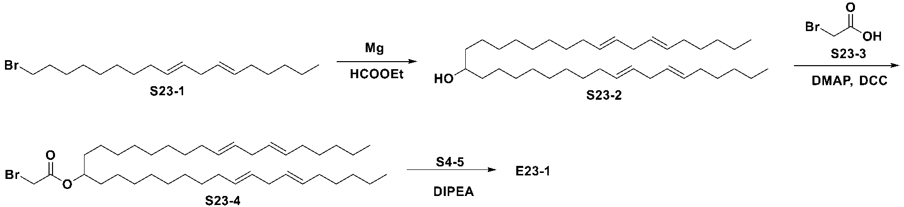

### Example 24: Cationic Lipid (E24-1)

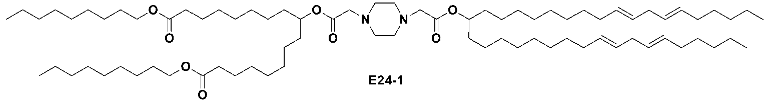

The preparation process is as follows:
Under nitrogen atmosphere, compound **S2-14** (0.70 g, 1.0 mmol) was dissolved in acetonitrile (30 mL), then **S23-4** (0.85 g, 1.3 mmol) and **DIPEA** (0.17 g, 1.3 mmol) were added sequentially under slow stirring, and then reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E24-1** (1.04 g). ¹H NMR (400 MHz, CDCl₃) δ: 5.40-5.31 (m, 8H, -C*H*=C*H*CH₂C*H=*C*H*-)*,* 4.93-4.87 (m, 2H, -C(=O)OC*H*<), 4.05 (t, 4H, -C(=O)OC*H*₂CH₂-), 3.17 (s, 4H, pip-C*H*₂C(=O)O-), 2.77 (t, 4H, -C*H*=CHC*H*₂CH=CH-)*,* 2.59-2.35 (m, 8H, pip-*H*), 2.33-2.21 (m, 4H, -OC(=O)C*H*₂-), 2.07-2.02 (m, 8H, -CH=CHC*H*₂CH₂-), 1.64-1.20 (m, 92H, -CH₂C*H*₂CH₂-, -C*H*₂CH₃), 0.88 (t, 12H, -CH₂C*H*₃). MS (ESI): m/z=1263.1 ([M+H]⁺).

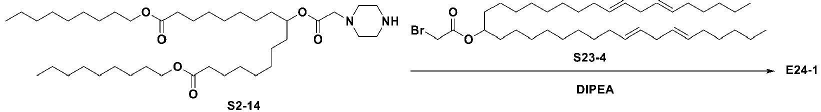

### Example 25: Cationic Lipid (E25-1)

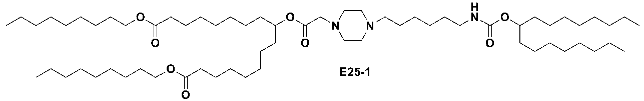

The preparation process is as follows:
Step a: 9-Heptadecanol **(S25-1,** 2.56 g, 10.0 mmol) was dissolved in dichloromethane (60 mL), and triethylamine (3 mL) and N,N'-carbonyldiimidazole **(CDI,** 1.62 g, 10.0 mmol) were added sequentially, and then the reaction was carried out at 50°C for 1.5 hours. Then, 6-amino-1-hexanol **(S25-2,** 1.76 g, 15.0 mmol) was added, and the reaction continued at 50°C for 16 hours. After the reaction was completed, the temperature was cooled to room temperature, and the solution was washed successively with 5% citric acid (30 mL × 2) and saturated brine (30 mL). The organic phase was dried with anhydrous magnesium sulfate, filtered, and concentrated to obtain the crude product. The crude product was dissolved in dichloromethane (30 mL), stirred for 10 minutes, and filtered. The filter cake was washed with a small amount of dichloromethane, and the product **S25-3** (2.62 g) was obtained after concentration.
Step b: **S25-3** (2.40 g, 6.0 mmol) was dissolved in dichloromethane (60 mL), and triphenylphosphine **(PPh₃,** 2.36 g, 9.0 mmol) was added. Under ice bath conditions, carbon tetrabromide **(CBr₄,** 2.98 g, 9.0 mmol) was added in batches, and the reaction was carried out for 20 minutes under ice bath conditions. The reaction was quenched with 10 mL of methanol, and the solution was directly concentrated to obtain the crude product. The crude product was purified by column chromatography to obtain **S25-4** (1.63 g).
Step c: Under nitrogen atmosphere, compound **S2-14** (1.39 g, 2.0 mmol) was dissolved in acetonitrile (30 mL), then **S25-4** (1.20 g, 2.6 mmol) and **DIPEA** (0.34 g, 2.6 mmol) were added sequentially under slow stirring. The reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E25-1** (1.79 g). ¹H NMR (400 MHz, CDCl₃) δ: 4.94-4.85 (m, 1H, -C(=O)OC*H*<), 4.13-4.10 (m, 1H, -NHC(=O)OC*H*<), 4.03 (t, 4H, -C(=O)OC*H*₂CH₂-), 3.17 (s, 2H, pip-C*H*₂C(=O)O-), 3.15 (t, 2H, -C*H*₂NHC(=O)O-), 2.59-2.35 (m, 10H; 8H, pip-*H*; 2H, pip-C*H*₂CH₂-), 2.33-2.21 (m, 4H, -OC(=O)C*H*₂-), 1.64-1.20 (m, 88H, -CH₂C*H*₂CH₂-, -C*H*₂CH₃), 0.88 (t, 12H, -CH₂C*H*₃). MS (ESI): m/z=1075.9 ([M+H]⁺).

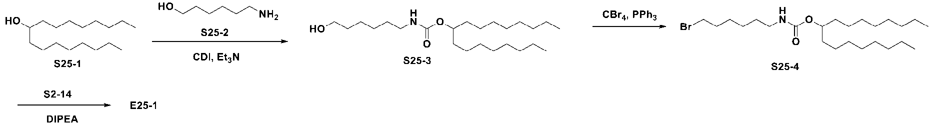

### Example 26: Cationic Lipid (E26-1)

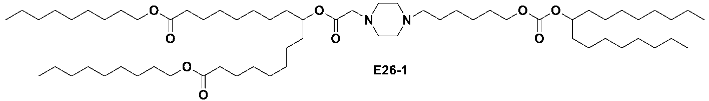

The preparation process is as follows:
Step a: **S12-2** (0.91 g, 5.0 mmol) was dissolved in 50 mL of dichloromethane, followed by **DMAP** (1.29 g, 10.0 mmol), then ***p*-Nitrophenyl chloroformate** (1.11 g, 5.5 mmol) was added in portions, and then the reaction solution was stirred at room temperature for 3 hours. **S25-1** (1.43 g, 5.6 mmol) was added to the reaction solution, and the solution was stirred at room temperature overnight. After the reaction was completed, the solution was diluted with 20 mL of dichloromethane and washed with 30 mL of saturated brine. The organic phase was dried with anhydrous magnesium sulfate, filtered, and the filtrate was concentrated. The crude product was purified by column chromatography to obtain **S26-1** (1.67 g).
Step b: Under nitrogen atmosphere, **S2-14** (1.39 g, 2.0 mmol) was dissolved in 20 mL of acetonitrile, then **S26-1** (1.21 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E26-1** (1.77 g). ¹H NMR (400 MHz, CDCl₃) δ: 4.93-4.87 (m, 1H, *-C(=O)OCH<),* 4.15-4.10 (m, 3H, *-CH₂OC(=O)OCH<),* 4.04 (t, 4H, -C(=O)OC*H*₂CH₂-), 3.17 (s, 2H, pip-C*H*₂C(=O)O-), 2.59-2.35 (m, 10H; 8H, pip-*H*; 2H, pip-C*H*₂CH₂-), 2.33-2.21 (m, 4H, -OC(=O)C*H*₂-), 1.64-1.20 (m, 88H, -CH₂C*H*₂CH₂-, -C*H*₂CH₃), 0.88 (t, 12H, -CH₂C*H*₃). MS (ESI): m/z=1076.9 ([M+H]⁺).

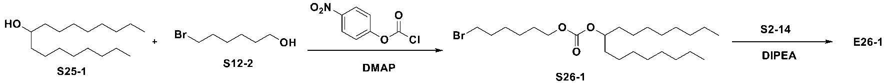

### Example 27: Cationic Lipid (E27-1)

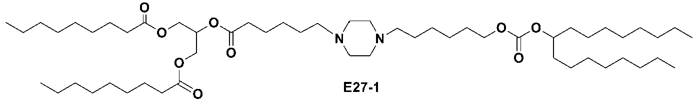

The preparation process is as follows:
Under nitrogen atmosphere, compound **S14-2** (1.11 g, 2.0 mmol) was dissolved in acetonitrile (30 mL), then **S26-1** (1.21 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E27-1** (1.55 g). ¹H NMR (400 MHz, CDCl₃) δ: 5.37-5.31 (m, 1H, -C(=O)OC*H*<), 4.38-4.29 (m, 2H, >CH(C*H*₂OC(=O))₂-), 4.19-4.13 (m, 5H; 2H, >CH(C*H*₂OC(=O))₂-; 3H, -C*H*₂OC(=O)OC*H*<), 2.59-2.35 (m, 12H; 8H, pip-*H*; 4H, pip-C*H*₂CH₂-), 2.33-2.21 (m, 6H, -OC(=O)C*H*₂-), 1.64-1.20 (m, 66H, -CH₂C*H*₂CH₂-, -C*H*₂CH₃), 0.88 (t, 12H, -CH₂C*H*₃). MS (ESI): m/z=936.8 ([M+H]⁺).

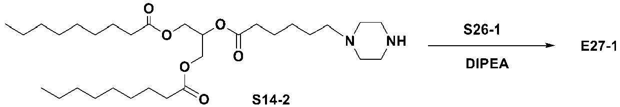

### Example 28: Cationic Lipid (E28-1)

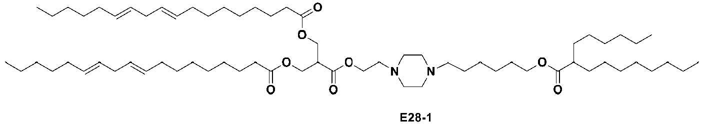

The preparation process is as follows:
Under nitrogen atmosphere, compound **S4-5** (1.51 g, 2.0 mmol) was dissolved in acetonitrile (30 mL), then **S12-3** (1.09 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E28-1** (1.81 g). ¹H NMR (400 MHz, CDCl₃) δ: 5.40-5.31 (m, 8H, -C*H*=C*H*CH₂C*H=*C*H*-)*,* 4.39-4.29 (m, 4H, >CH(C*H*₂OC(=O)₂-), 4.20 (t, 2H, pip-CH₂C*H*₂OC(=O)-), 4.04 (t, 2H, -C(=O)OC*H*₂CH₂CH₂-), 3.02 (q, 1H, >C*H*C(=O)OCH₂CH₂-pip), 2.77 (t, 4H, -CH=CHC*H*₂CH=CH-)*,* 2.72-2.40 (m, 13H; 8H, pip-*H*; 4H, pip-C*H*₂CH₂-; 1H, >C*H*C(=O)O-), 2.32-2.27 (m, 4H, -CH₂C*H*₂C(=O)O-), 2.07-2.02 (m, 8H, -CH=CHC*H*₂CH₂-), 1.64-1.20 (m, 64H, -CH₂C*H*₂CH₂-, -C*H*₂CH₃), 0.88 (t, 12H, -CH₂C*H*₃). MS (ESI): m/z=1095.0 ([M+H]⁺).

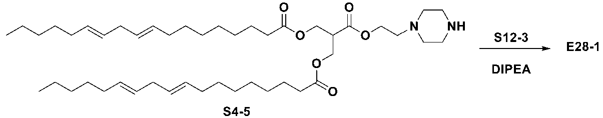

### Example 29: Cationic Lipid (E29-1)

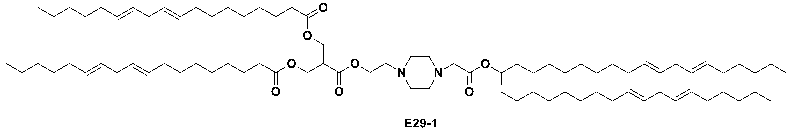

The preparation process is as follows:
Under nitrogen atmosphere, compound **S4-5** (1.51 g, 2.0 mmol) was dissolved in acetonitrile (30 mL). then **S23-4** (1.69 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E29-1** (2.20 g). ¹H NMR (400 MHz, CDCl₃) δ: 5.40-5.31 (m, 16H, -C*H*=C*H*CH₂C*H*=C*H*-)*,* 4.96-4.87 (m, 1H, -C(=O)OC*H*<), 4.39-4.29 (m, 4H, >CH(C*H*₂OC(=O)₂-), 4.20 (t, 2H, pip-CH₂C*H*₂-), 3.17 (s, 2H, pip-C*H*₂C(=O)O-), 3.02 (q, 1H, >C*H*C(=O)OCH₂-), 2.77 (t, 8H, -CH=CHC*H*₂CH=CH-)*,* 2.75-2.40 (m, 10H; 8H, pip-*H*; 2H, pip-C*H*₂CH₂-), 2.32-2.27 (m, 4H, -CH₂C*H*₂C(=O)O-), 2.07-2.02 (m, 16H, -CH=CHC*H*₂CH₂-), 1.64-1.20 (m, 72H, -CH₂C*H*₂CH₂-, -C*H*₂CH₃), 0.88 (t, 12H, -CH₂C*H*₃). MS (ESI): m/z=1325.7 ([M+H]⁺).

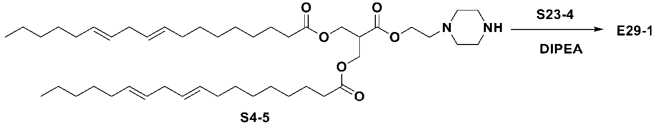

### Example 30: Cationic Lipid (E30-1)

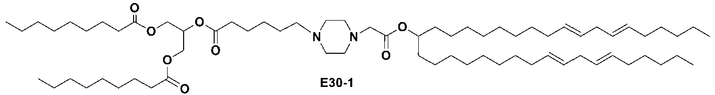

The preparation process is as follows:
Under nitrogen atmosphere, compound **S14-2** (1.11 g, 2.0 mmol) was dissolved in acetonitrile (30 mL), then **S23-4** (1.69 g, 2.6 mmol) and **DIPEA** (0.34 g, 2.6 mmol) were added sequentially under slow stirring, and then the reaction solution was stirred at room temperature for about 20 hours. After the reaction was completed, the reaction solution was concentrated and dissolved in dichloromethane, then successively extracted with 0.6 M HCl/10% NaCl solution and saturated sodium bicarbonate solution. The organic phase was combined, dried with anhydrous magnesium sulfate, filtered, and the filtrate was concentrated. The crude product was purified by column chromatography to obtain the cationic lipid **E30-1** (2.23 g). ¹H NMR (400 MHz, CDCl₃) δ: 5.40-5.31 (m, 9H; 1H, -C(=O)OC*H*<; 8H, -C*H*=C*H*CH₂C*H*=C*H*-)*,* 4.94-4.87 (m, 1H, *-C(=O)OCH<),* 4.38-4.29 (m, 2H, >CH(C*H*₂OC(=O))₂-), 4.19-4.13 (m, 2H, >CH(C*H*₂OC(=O))₂-), 3.17 (s, 2H, pip-C*H*₂C(=O)O-), 2.77 (t, 4H, -CH=CHC*H*₂CH=CH-)*,* 2.73-2.35 (m, 16H; 8H, pip-*H*; 2H, pip-C*H*₂CH₂-; 4H, >CHCH₂OC(=O)C*H*₂-; 2H, >CHOC(=O)C*H*₂-), 2.07-2.02 (m, 8H, -CH=CHC*H*₂CH₂-), 1.64-1.20 (m, 70H, -CH₂C*H*₂CH₂-, -C*H*₂CH₃), 0.88 (t, 12H, -CH₂C*H*₃). MS (ESI): m/z=1123.1 ([M+H]⁺).

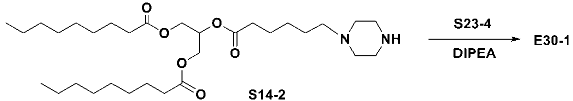

### Example 31: Preparation of LNP-mRNA Pharmaceutical Compositions and Characterization of Physicochemical Properties Thereof

### Example 31.1: Preparation of LNP-mRNA Pharmaceutical Compositions

In the present embodiment, LNP-mRNA pharmaceutical compositions (LNP-mRNAs) containing Fluc-mRNA were prepared; wherein, the phospholipid contained in each group was DSPC, the steroid lipid contained in each group was cholesterol, and the PEGylated lipid contained in each group was PEG2k-DMG; the difference among the groups lied in the cationic lipid.

The preparation method of LNP-mRNA is as follows:
Step a: A certain amount of cationic lipid, DSPC, cholesterol, and PEGylated lipid stock solutions were pipetted and dissolved in ethanol at a molar ratio of 50:10:38:1.5 to obtain the ethanol phase solution (the control groups **L-CT1, L-CT2,** and **L-CT3** used cationic lipids from prior art, while the experimental groups L-1 to L-34 used the azacycle-containing cationic lipids from Examples 1 to 30 of the present application).

Wherein, the cationic lipid used in L-CT1 and L-CT2 were respectively C-1 and C-2, prepared according to the method disclosed in patent literature CN115010681A; the cationic lipid used in L-CT3 was C-3, prepared according to the method disclosed in patent literature WO2023036148A1. The structures of C-1, C-2, and C-3 are as follows:

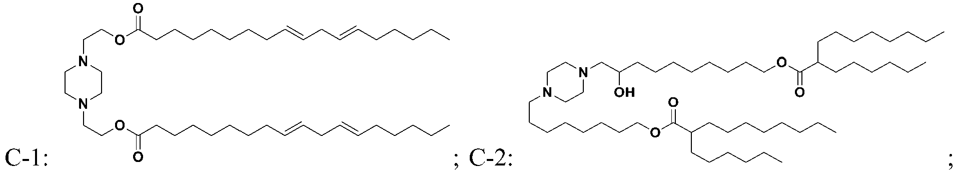

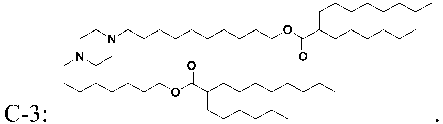

Step b: Fluc-mRNA was added to 10~50 mM citrate buffer (pH=4) to obtain the aqueous solution.

Step c: the ethanol phase solution and the aqueous phase solution were mixed (1:3, v/v) to prepare LNP-mRNA, which was washed by multiple DPBS ultrafiltration to remove ethanol and free molecules. Finally, it was filtered through a 0.2 µm sterile filter and stored for subsequent use.

### Example 31.2: Characterization of Physicochemical Properties of LNP-mRNA Pharmaceutical Compositions

Determination of encapsulation rate: The encapsulation efficiency of LNP-mRNA compositions was determined using the Quant-it Ribogreen RNA Quantification Kit. The results showed that the lipid compositions of the present application (L-1~L-36) exhibited high nucleic acid encapsulation efficiencies for nucleic acid drugs, all in the range of 80%-96%, and most in the range of 90%-96%. The results showed that mRNA could be effectively encapsulated in the experimental groups with azacycle-containing cationic lipids, wherein the encapsulation efficiencies were comparable to or better than those of C-1, C-2, and C-3; there were also differences in the encapsulation efficiency among the azacycle-containing cationic lipids with different structures.

Determination of particle size: In the present embodiment, the particle size of LNP-mRNA was determined by dynamic light scattering (DLS). The measured LNP-mRNAs exhibited high dimensional uniformity, with PDI values less than 0.3. The particle sizes of the LNP-mRNAs prepared from the lipid compositions of the present application were in the range of 90-120 nm, which meets the requirements of particle size for gene vectors.

**Table 1: Summary of Formulations of Lipid Compositions and Particle Sizes and Encapsulation Efficiency of the Prepared LNP-mRNAs**

| **LNP-mRNA** | **L-CT1** | **L-CT2** | **L-CT3** | **L-1** | **L-2** | **L-3** |
|---|---|---|---|---|---|---|
| **Cationic Lipid, CL** | C-1 | C-2 | C-3 | E1-1 | E2-1 | E2-2 |
| **Encapsulation Efficiency /%** | 86.82 | 92.87 | 93.15 | 93.52 | 95.82 | 92.39 |
| **Particle Size /nm** | 93.59 | 95.43 | 96.59 | 94.12 | 94.53 | 95.53 |

| **LNP-mRNA** | **L-4** | **L-5** | **L-6** | **L-7** | **L-8** | **L-9** |
|---|---|---|---|---|---|---|
| **Cationic Lipid, CL** | E3-1 | E4-1 | E5-1 | E6-1 | E7-1 | E7-2 |
| **Encapsulation** | 94.26 | 94.11 | 91.72 | 94.25 | 93.21 | 94.15 |
| **Efficiency /%** | | | | | | |
| **Particle Size /nm** | 95.42 | 95.50 | 95.41 | 93.35 | 94.72 | 95.83 |

| **LNP-mRNA** | **L-10** | **L-11** | **L-12** | **L-13** | **L-14** | **L-15** |
|---|---|---|---|---|---|---|
| **Cationic Lipid, CL** | E8-1 | E9-1 | E10-1 | E11-1 | E12-1 | E13-1 |
| **Encapsulation Efficiency /%** | 90.63 | 92.16 | 94.66 | 93.52 | 94.82 | 94.46 |
| **Particle Size /nm** | 92.09 | 93.72 | 92.29 | 94.74 | 96.37 | 97.53 |

| **LNP-mRNA** | **L-16** | **L-17** | **L-18** | **L-19** | **L-20** | **L-21** |
|---|---|---|---|---|---|---|
| **Cationic Lipid, CL** | E14-1 | E15-1 | E15-2 | E16-1 | E17-1 | E17-2 |
| **Encapsulation Efficiency /%** | 94.85 | 94.72 | 93.85 | 94.39 | 94.83 | 94.68 |
| **Particle Size /nm** | 97.45 | 95.73 | 93.45 | 94.52 | 96.25 | 99.42 |

| **LNP-mRNA** | **L-22** | **L-23** | **L-24** | **L-25** | **L-26** | **L-27** |
|---|---|---|---|---|---|---|
| **Cationic Lipid, CL** | E18-1 | E19-1 | E20-1 | E21-1 | E22-1 | E23-1 |
| **Encapsulation Efficiency /%** | 94.82 | 94.88 | 95.33 | 94.41 | 92.55 | 93.52 |
| **Particle Size /nm** | 95.81 | 92.51 | 93.46 | 95.38 | 94.37 | 92.12 |

| **LNP-mRNA** | **L-28** | **L-29** | **L-30** | **L-31** | **L-32** | **L-33** |
|---|---|---|---|---|---|---|
| **Cationic Lipid, CL** | E24-1 | E25-1 | E26-1 | E27-1 | E28-1 | E29-1 |
| **Encapsulation Efficiency /%** | 92.63 | 93.52 | 94.42 | 93.93 | 94.62 | 93.53 |
| **Particle Size /nm** | 95.72 | 96.70 | 94.75 | 94.62 | 94.96 | 95.31 |

| **LNP-mRNA** | **L-34** | | | | | |
|---|---|---|---|---|---|---|
| **Cationic Lipid, CL** | E30-1 | | | | | |
| **Encapsulation Efficiency /%** | 94.63 | | | | | |
| **Particle Size /nm** | 95.22 | | | | | |

### Example 32: Biological Activity Tests of LNP-mRNA Pharmaceutical Compositions

### (1) Serum Stability Evaluation

LNP-mRNA pharmaceutical compositions were added to the medium containing 10% fetal bovine serum (FBS) with agitation at 37°C. Samples was taken at regular intervals to determine the particle size change of LNP-mRNA, and the serum stability of the LNP-mRNA pharmaceutical composition formulation was analyzed by testing its particle size change. The experimental results showed that within 7 days, the particle size change of control groups and experimental groups were less than 10%, and the particle size change of all experimental groups were less than 5%, indicating that the formulation of LNP-mRNA pharmaceutical composition prepared from the cationic lipids of the present application exhibited good serum stability.

### (2) Assays for Cytotoxicity (Biocompatibility)

DMEM high glucose complete culture medium containing 10% FBS was prepared. The samples (L-1 to L-34 and L-CT1 to L-CT3) were respectively formulated into working solutions of 0.1, 0.15, 0.2, 0.25, 0.3 µg/100 µL using the complete culture medium, and were stored for subsequent use. 293T cells in the logarithmic growth phase was inoculated onto a 96-well plate at a density of 7×10³ cells/well, with 100 µL/well. Both the control and experimental groups were set with six replicate wells. After incubation in a constant temperature incubator with 5% CO₂ at 37°C for 24 hours, the original culture medium was retained, the control group was supplemented with 100 µL/well of complete culture medium, and the sample group was supplemented with 100 µL/well of the working solution. After further incubation for 24 hours, 100 µL/well of culture medium containing 10% CCK-8 was added, and the solution was cultured in a constant temperature incubator with 5% CO₂ at 37°C for 2 hours. The absorbance at 450 nm was measured using a microplate reader. The relative cell viability was calculated using the following formula:
Relative Viability% = (Sample Group Absorbance - Background Absorbance) / (Control Group Absorbance - Background Absorbance) × 100%; wherein, the background absorbance refers to the absorbance of the sample containing only CCK-8 reagent and culture medium.

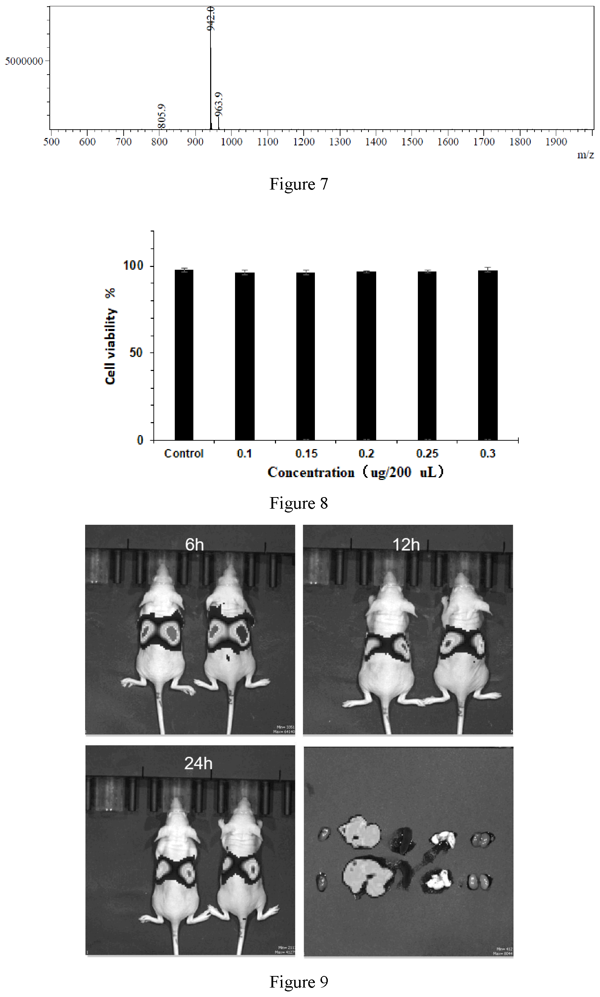

The experimental results showed that the LNP-mRNA pharmaceutical composition prepared with the cationic lipid of the present application has no obvious cytotoxicity at five concentration gradients, with cell viability above 95%. Specifically, for example, the results of the experimental group **L-24** are shown in Figure 8.

### (3) Evaluation of In Vitro Transfection Efficiency

In order to investigate the mRNA transfection efficiency at the cellular level of each group of LNP-mRNA pharmaceutical compositions prepared in Example **31.1** of the present application, luciferase bioluminescence was used for testing. The LNP-mRNA pharmaceutical composition was dissolved in medium to prepare the required dose. 293T cells were used as the cell model, and cell suspensions of 100 µL/well were inoculated in a black-edged, clear-bottomed 96-well plate at a density of 6000 cells/well. After inoculation was completed, the cells were cultured in a cell culture incubator for 24 hours, and then the cells were dosed with 0.2 µg mRNA per well, while the blank control group was added with the corresponding dose of free Fluc-mRNA. After 24 hours of transfection, the old medium was replaced with fresh medium containing D-fluorescein sodium substrate (1.5 mg/mL). After 5 min of incubation, bioluminescence was detected using a microplate reader. The stronger the fluorescence, the more Fluc-mRNA is transported into the cytoplasm and translated the corresponding fluorescent protein. The results are shown in Table 2; wherein, the relative value of fluorescence intensity is the ratio of the fluorescence intensity of each group to the fluorescence intensity of the control group.

The results show that the LNP-mRNA drug compositions prepared in the present application exhibited excellent in vitro transfection effects, indicating that the LNPs in the experimental groups are effective nucleic acid delivery carriers. This is probably attributed to the fact that the transfection efficiency of the LNPs prepared with the cationic lipids of the present application is superior to that of the LNPs prepared with the cationic lipids of the prior art in L-CT1, and most are superior to the L-CT2 and L-CT3 groups. This may be because C-1 contains only two linear hydrophobic hydrocarbon tail chains, a structure that is not conducive to forming a conical geometric shape, thereby resulting in lower transfection efficiency. C-2 and C-3 differ from the cationic lipids of the present application in terms of the type and number of degradable groups, the number of hydroxyl groups, and the saturation of the hydrophobic hydrocarbon tail chains, thus exhibiting different transfection efficiency. Therefore, the experimental groups L-8 (E7-1), L-9 (E7-2), L-10 (E8-1), L-16 (E14-1), L-24 (E20-1), L-26 (E22-1), and L-31 (E27-1) have higher relative fluorescence values. This may be because these cationic lipid compounds contain a greater number of degradable groups that can prevent the endosomal accumulation of LNP-mRNA, promote the endosomal escape of LNP-mRNA, and thus enhance delivery efficacy; and/or the presence of unsaturated hydrophobic hydrocarbon tail chains can increase membrane fluidity and cellular uptake, thereby improving delivery efficacy; and/or the hydroxyl groups can interact with the phosphate groups on the nucleic acids through hydrogen bonding, thereby enhancing delivery efficiency.

**Table 2: Results of Cell Transfection Tests**

| **Entry** | **Relative Fluorescence Value** | **Entry** | **Relative Fluorescence Value** | **Entry** | **Relative Fluorescence Value** |
|---|---|---|---|---|---|
| **Blank** | 1 | **L-11** | 9.4 | **L-25** | 9.3 |
| **L-CT1** | 5.2 | **L-12** | 8.5 | **L-26** | 10.2 |
| **L-CT2** | 8.9 | **L-13** | 10.3 | **L-27** | 8.7 |
| **L-CT3** | 8.5 | **L-14** | 9.8 | **L-28** | 8.3 |
| **L-1** | 8.8 | **L-15** | 9.7 | **L-29** | 9.1 |
| **L-2** | 9.2 | **L-16** | 10.1 | **L-30** | 9.2 |
| **L-3** | 8.2 | **L-17** | 8.6 | **L-31** | 9.8 |
| **L-4** | 8.3 | **L-18** | 8.4 | **L-32** | 9.5 |
| **L-5** | 8.6 | **L-19** | 10.6 | **L-33** | 8.5 |
| **L-6** | 8.1 | **L-20** | 9.6 | **L-34** | 8.3 |
| **L-7** | 8.2 | **L-21** | 9.1 | | |
| **L-8** | 10.3 | **L-22** | 8.8 | | |
| **L-9** | 10.1 | **L-23** | 9.7 | | |
| **L-10** | 10.2 | **L-24** | 10.8 | | |

### (4) Evaluation of In Vivo Transfection Efficiency

The lipid nanoparticle **L-24** was delivered to 6-8-week-old female BALB/c mice at a dose of 10 µg per mouse by tail vein injection, and in vivo fluorescence imaging of the mice was performed at 6, 12, and 24 hours post-administration. After the last imaging was completed, the mice were euthanized, and imaging was conducted on the major organs including heart, liver, spleen, lungs, and kidneys (from left to right in the figure). 10 to 15 minutes prior to imaging, 0.2 mL of D-luciferin sodium salt (15 mg/mL) was injected intraperitoneally. The experimental results (Figure 9) indicate that the lipid-nucleic acid pharmaceutical composition prepared from the cationic lipid of the present application can achieve efficient in vivo delivery of nucleic acid drugs, and the LNP-mRNA pharmaceutical composition delivered into the body was primarily distributed in the liver and spleen.

Those described above are only embodiments of the present invention, not limiting the patent scope of the present invention. Any transformation of equivalent structure or equivalent route based on the specification content of the present invention, directly or indirectly applied in other related arts, should be included in the protected patent scope of the present invention in the same way.

For those skilled in the art, without departing from the purpose and the scope of the present invention, and without unnecessary experimentation, the present invention can be implemented in a wider range with equivalent parameters, concentrations, and conditions. While the present invention has given particular examples, it should be understood that the present invention can be further modified. In conclusion, in accordance with the principles of the present invention, the present application is intended to cover any alterations, uses, or improvements of the present invention, including changes departing from the scope disclosed in this application but made using conventional techniques known in the art.

## Claims

1. A cationic lipid, wherein the structure is represented by the general formula (1): 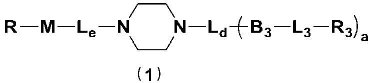
or a salt, tautomer, stereoisomer, deuterated derivative, or solvate thereof;
wherein, L_{d} is a linking bond, and a is 1;
Lₑ is a C₁₋₁₂ alkylene group;
M is selected from the group consisting of -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, -NHC(=O)S-, -C(=S)-, -OC(=S)-, -C(=S)O-, -OC(=S)O-, -NHC(=S)-, -C(=S)NH-, -NHC(=S)NH-, -OC(=S)NH-, and -NHC(=S)O-;
R is 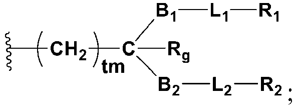
wherein, tm is an integer from 0 to 6; B₁, B₂, and B₃ are each independently a C₁₋₂₀ alkylene group; L₁, L₂, and L₃ are each independently selected from the group consisting of -CH(OH)-, -OC(=O)-, -C(=O)O-, -OC(=O)O-, -C(=O)-, -O-, -NH-, -O(CH₂)ₛO-, -S-, -S-S-, -C(=O)S-, -SC(=O)-, -NHC(=O)-, -C(=O)NH-, -NHC(=O)NH-, -OC(=O)NH-, -NHC(=O)O-, -SC(=O)NH-, and -NHC(=O)S-, wherein s is 1, 2, or 3; R_{g} is H or a C₁₋₆ alkyl group; R₁, R₂, and R₃ are each independently a linear or branched C₅₋₄₀ hydrocarbon group.

2. The cationic lipid according to claim **1,** wherein the B₁, B₂, and B₃ are each independently selected from the group consisting of a methylene group, an ethylene group, a propylene group, a butylene group, a pentylene group, a hexylene group, and a heptylene group.

3. The cationic lipid according to claim **1,** wherein the L₁, L₂, and L₃ are each independently selected from the group consisting of -CH(OH)-, -OC(=O)-, -C(=O)O-, -C(=O)NH-, -NHC(=O)-, -NHC(=O)O-, and -OC(=O)NH-.

4. The cationic lipid according to claim **1,** wherein the linear C₅₋₄₀ hydrocarbon group is a C₅₋₄₀ linear alkyl group, a C₅₋₄₀ linear alkenyl group, or a C₅₋₄₀ linear alkynyl group, preferably each independently a C₅₋₂₅ linear alkyl group, a C₅₋₂₅ linear alkenyl group, or a C₅₋₂₅ linear alkynyl group; the linear C₅₋₄₀ hydrocarbon group is more preferably selected from the group consisting of the following structures: 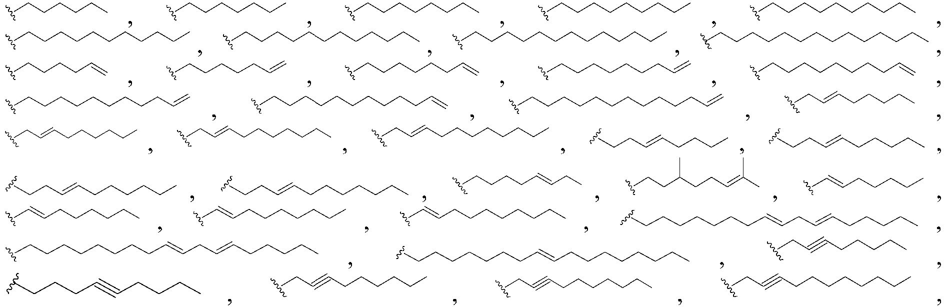 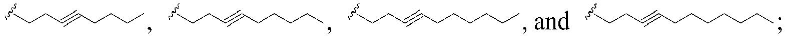 the branched C₅₋₄₀ hydrocarbon group is a C₅₋₄₀ branched alkyl group, a C₅₋₄₀ branched alkenyl group, or a C₅₋₄₀ branched alkynyl group, each independently represented by 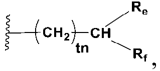 wherein tn is an integer from 0 to 12; Rₑ and R_{f} are each independently selected from the group consisting of a C₁₋₁₅ alkyl group, a C₂₋₂₀ alkenyl group, and a C₂₋₁₅ alkynyl group; more preferably, Rₑ and R_{f} are each independently selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a pentenyl group, a hexenyl group, a heptenyl group, an octenyl group, a nonenyl group, a decenyl group, an octadecane-6,9-dienyl group, and substituted forms thereof; the branched C₅₋₄₀ hydrocarbon group is most preferably selected from the group consisting of the following structures: 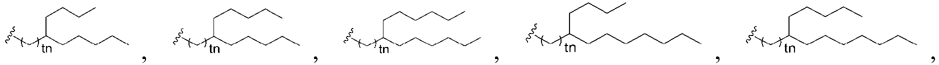 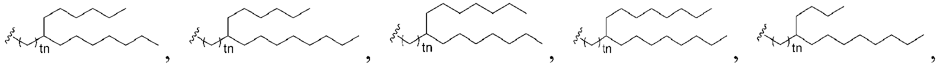 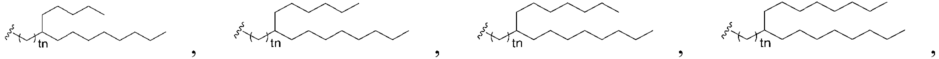 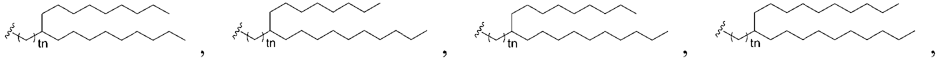 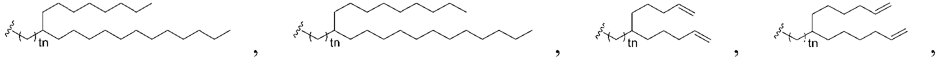 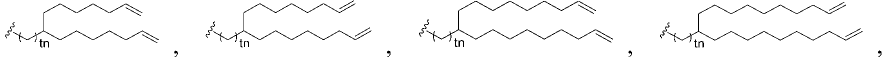 and

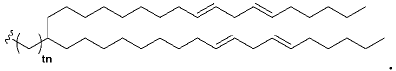

5. The cationic lipid according to claim 1, wherein the R₁, R₂, and R₃ are selected from any of the following cases:
Case (1): R₁, R₂, and R₃ are all linear C₅₋₄₀ hydrocarbon groups;
Case (2): R₁, R₂, and R₃ are all branched C₅₋₄₀ hydrocarbon groups;
Case (3): any one of R₁, R₂, and R₃ is a linear C₅₋₄₀ hydrocarbon group, and the other two are branched C₅₋₄₀ hydrocarbon groups;
Case (4): any two of R₁, R₂, and R₃ are linear C₅₋₄₀ hydrocarbon groups, and the other is a branched C₅₋₄₀ hydrocarbon group; preferably, R₁ and R₂ are linear C₅₋₄₀ hydrocarbon groups, and R₃ is a branched C₅₋₄₀ hydrocarbon group.

6. The cationic lipid according to claim **1,** wherein the fragment -B₃-L₃-R₃ is independently selected from the group consisting of the following structures:

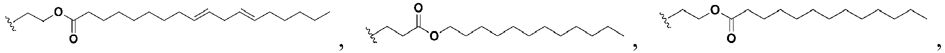

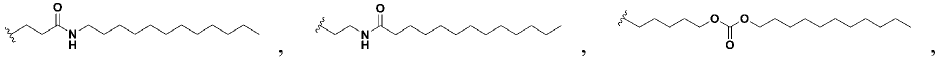

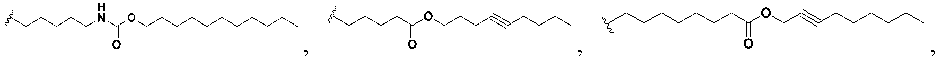

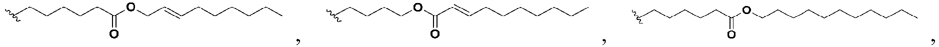

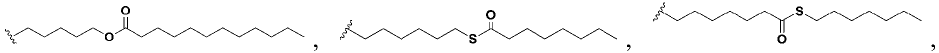

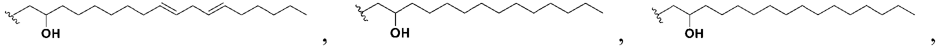

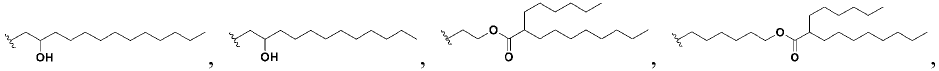

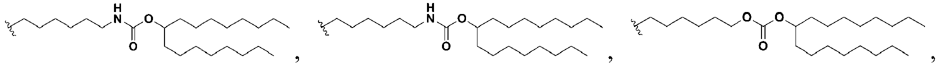

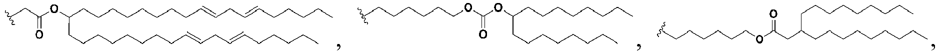

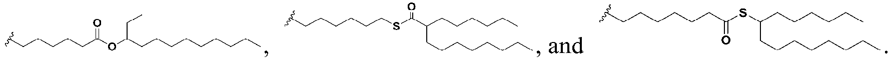

7. The cationic lipid according to claim **1,** wherein the R is independently selected from the group consisting of the following structures:

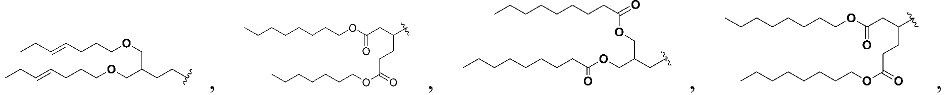

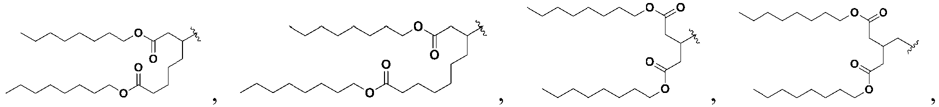

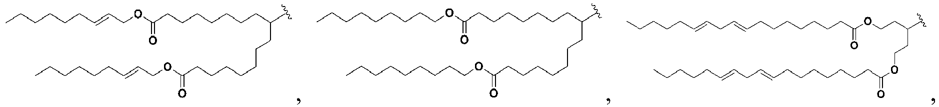

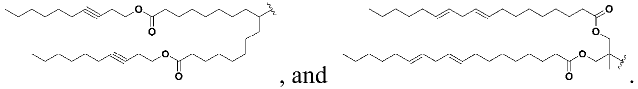

8. The cationic lipid according to claim **1,** wherein the M is selected from the group consisting of -OC(=O)-, -C(=O)O-, -OC(=O)O-, -NHC(=O)-, -C(=O)NH-, -OC(=O)NH-, and -NHC(=O)O-;
the cationic lipid preferably satisfies any one of the following general formulas: 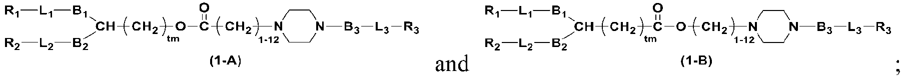
more preferably, the cationic lipid satisfies any one of the following general formulas: 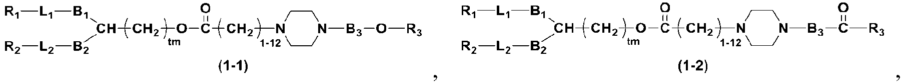 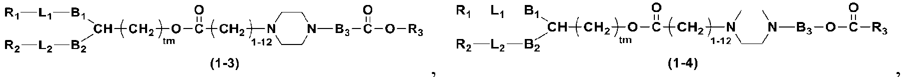 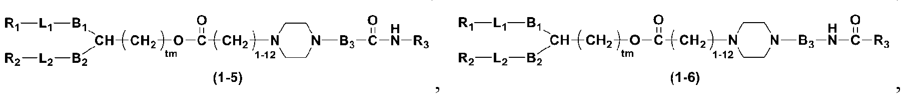 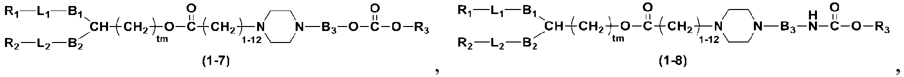 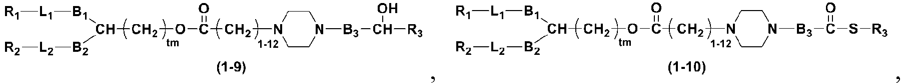 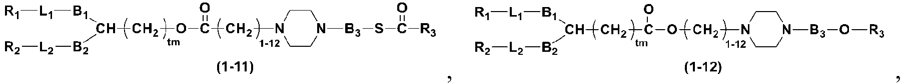 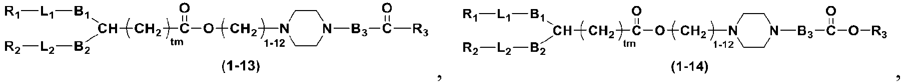 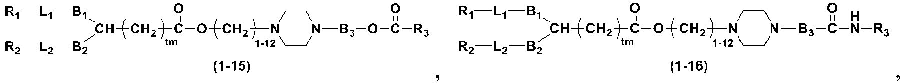 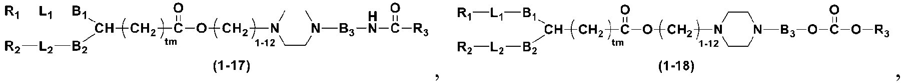 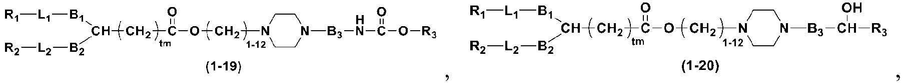 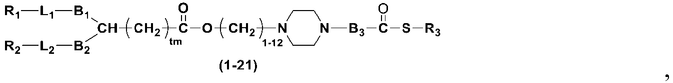 and 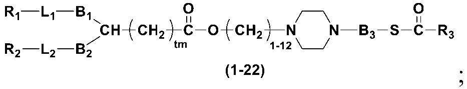
preferably, in the aforementioned general formulas (1-1) to (1-22), B₁ and B₂ are the same and each a C₁₋₁₂ alkylene group; L₁ and L₂ are the same and each selected from the group consisting of -OC(=O)-, -C(=O)O-, -NHC(=O)-, -C(=O)NH-, and -OC(=O)O-; R₁ and R₂ are the same and each a linear C₅₋₄₀ hydrocarbon group; R₃ is a linear C₅₋₄₀ hydrocarbon group.

9. The cationic lipid according to claim 1, wherein the cationic lipid is selected from the group consisting of the following structures: 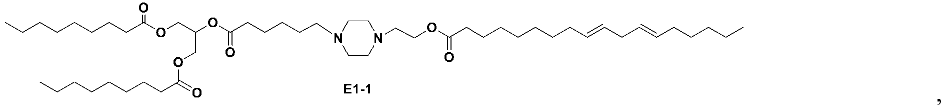 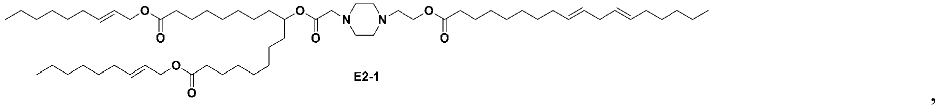 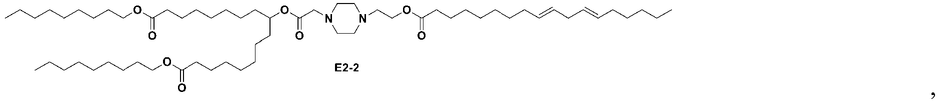 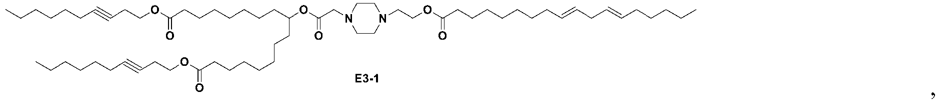 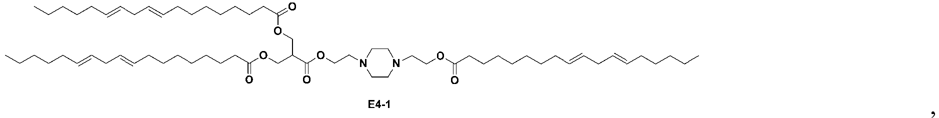 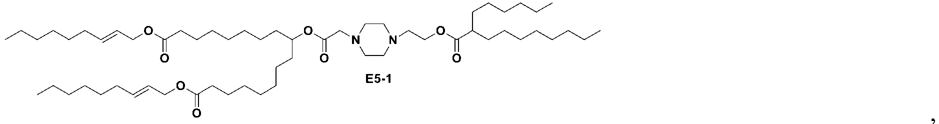 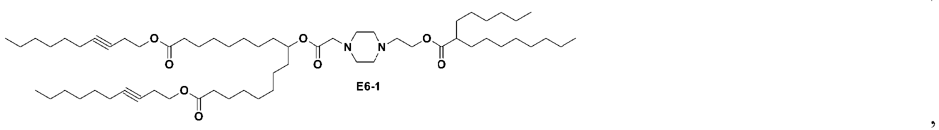 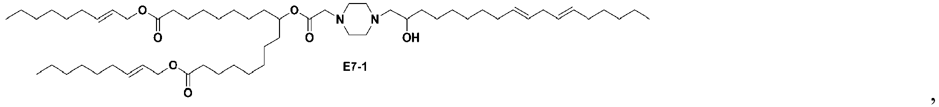 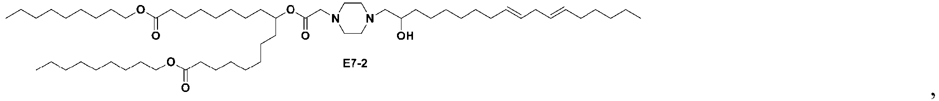 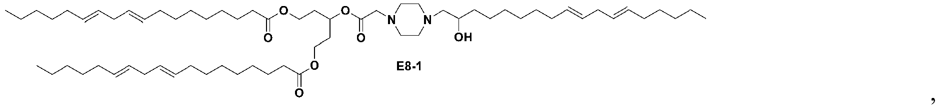 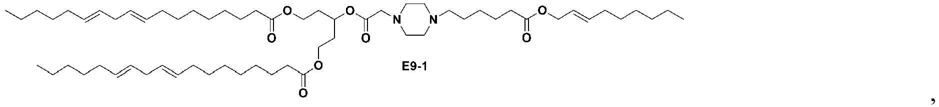 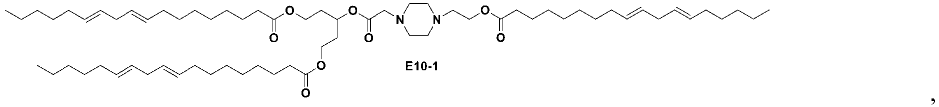 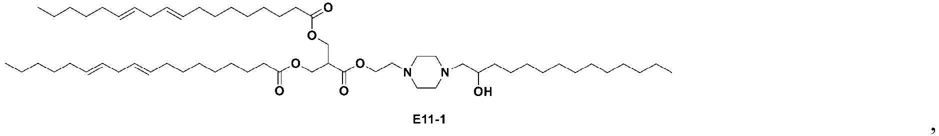 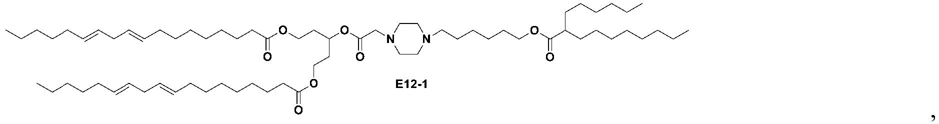 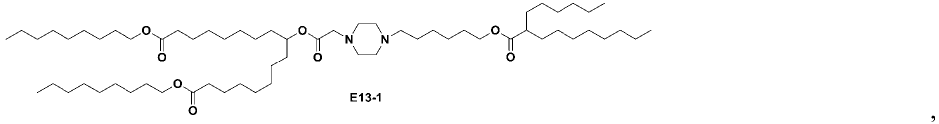 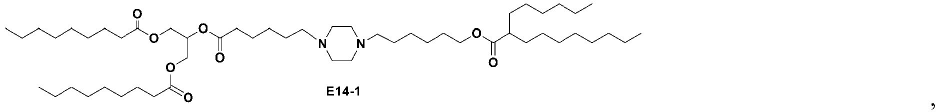 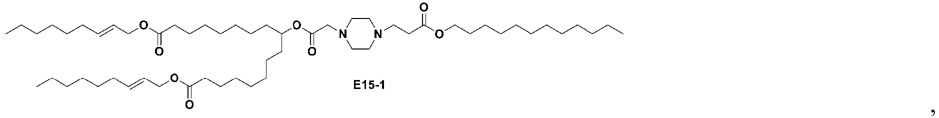 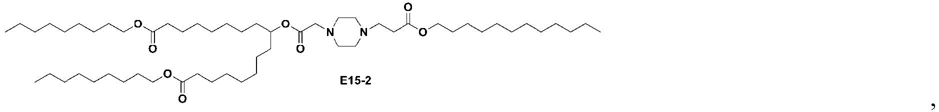 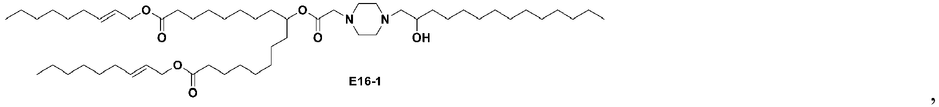 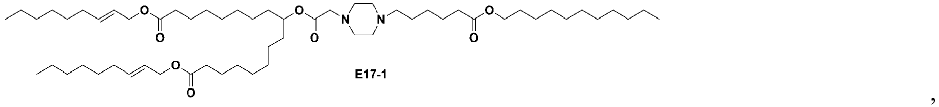 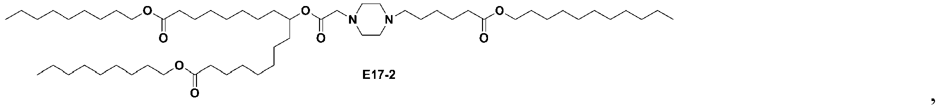 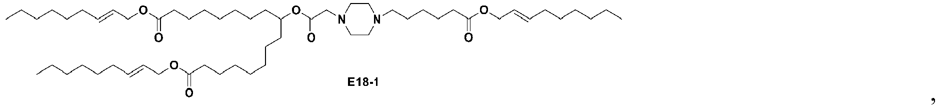 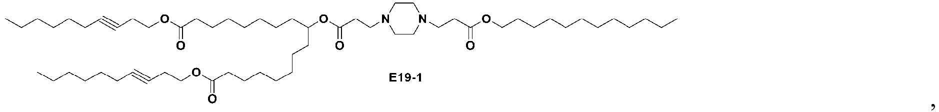 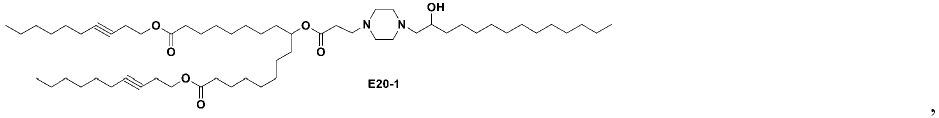 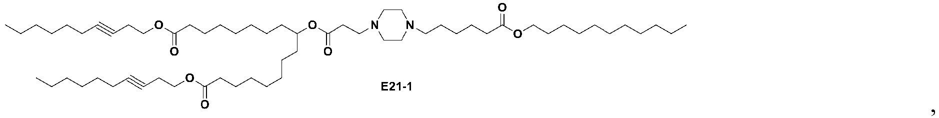 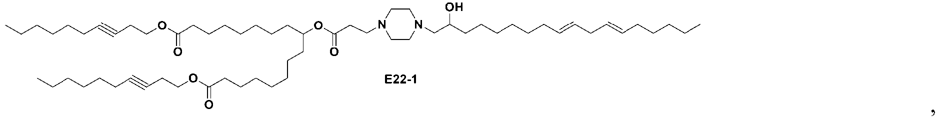 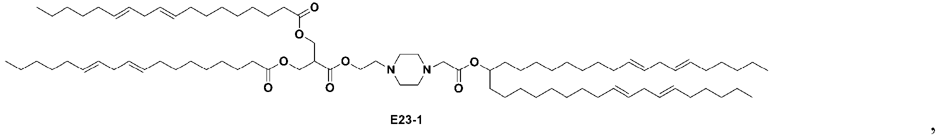 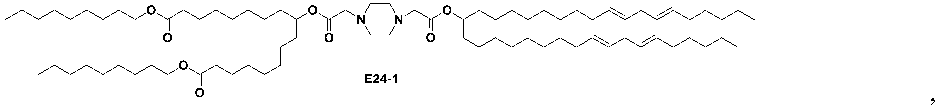 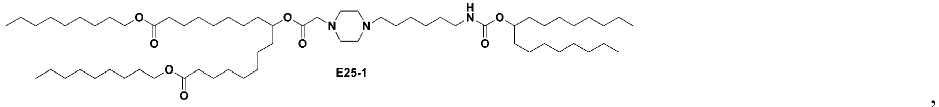 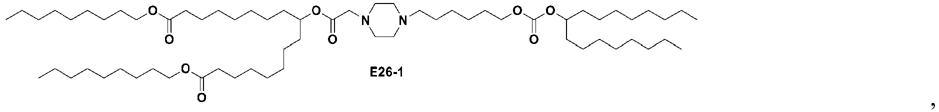 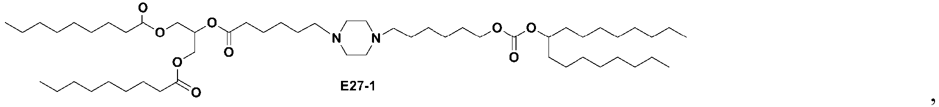 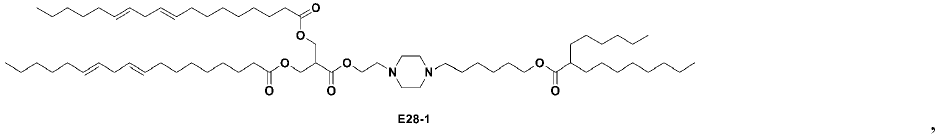 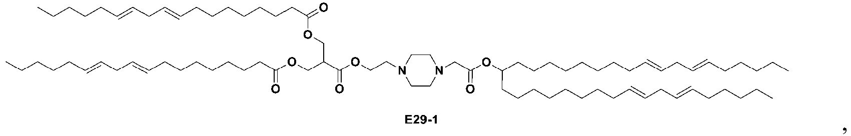 and

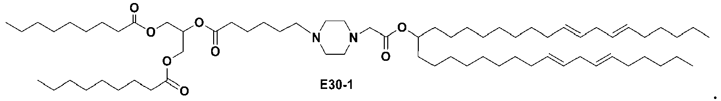

10. A lipid composition comprising a cationic lipid of any one of claims **1** to **9.**

11. The lipid composition according to claim **10,** wherein the lipid composition comprises one or more types of lipids selected from the group consisting of phospholipid, steroid lipid, and PEGylated lipid; the lipid composition is selected from any of the following cases:
Case (1): the lipid composition further comprises a phospholipid;
Case (2): the lipid composition further comprises a steroid lipid;
Case (3): the lipid composition further comprises a PEGylated lipid;
Case (4): the lipid composition further comprises a phospholipid and a steroid lipid;
Case (5): the lipid composition further comprises a phospholipid and a PEGylated lipid;
Case (6): the lipid composition further comprises a steroid lipid and a PEGylated lipid;
Case (7): the lipid composition further comprises a phospholipid, a steroid lipid, and a PEGylated lipid;
Case (8): the lipid composition further comprises a phospholipid, a steroid lipid, a PEGylated lipid, and another cationic lipid;
Case (9): the lipid composition further comprises a phospholipid, a steroid lipid, a PEGylated lipid, and an anionic lipid;
more preferably, the lipid composition further comprises three types of lipids, simultaneously, which are a phospholipid lipid, a steroid lipid, and a PEGylated lipid.

12. The lipid composition according to claim **11,** wherein the phospholipid in the lipid composition is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-*O*-octadecenyl-sn-glycero-3-phosphatidylcholine, 1-oleoyl-2-cholesterylhemisuccinyl-sn-glycero-3-phosphocholine, 1-*O*-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof;
or the steroid lipid in the lipid composition is selected from the group consisting of cholesterol, fecal sterol, sitosterol, ergosterol, campesterol, stigmasterol, rapeseed sterol, lycopene, ursolic acid, α-tocopherol, and combinations thereof;
or the PEGylated lipid in the lipid composition is selected from the group consisting of 1,2-dimyristoyl-sn-glycerol methoxypolyethylene glycol, 1,2-distearoyl-sn-glycero-3-phosphoethanolamine-*N*-[amino(polyethylene glycol)], PEG-cholesterol, PEG-diacylglycamide, and PEG-dialkyloxypropyl, specifically including PEG500-dipalmitoylphosphatidylcholine, PEG2000-dipalmitoylphosphatidylcholine, PEG500-stearylphosphatidylethanolamine, PEG2000-distearylphosphatidylethanolamine, PEG500-1,2-oleoylphosphatidylethanolamine, PEG2000-1,2-oleoylphosphatidylethanolamine, PEG2000-2,3-dimyristoylglycerol, and combinations thereof;
or the PEGylated lipid in the lipid composition is selected from the group consisting of the following structures and combinations thereof: 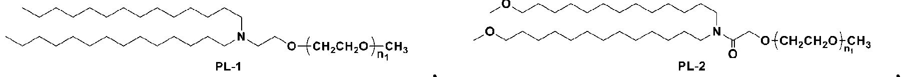 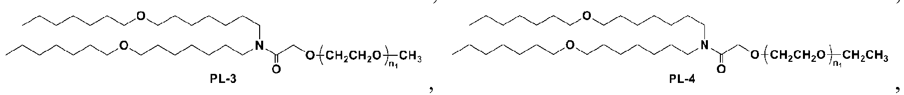 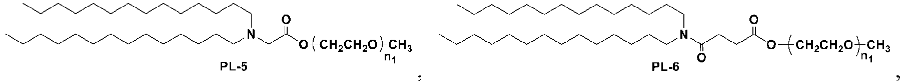 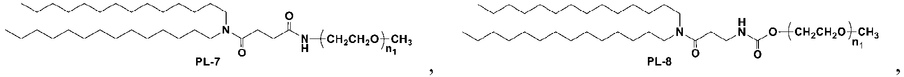 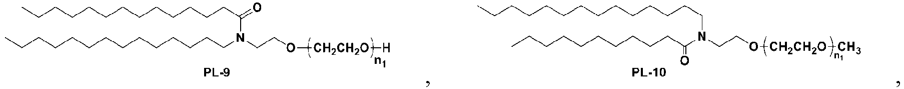 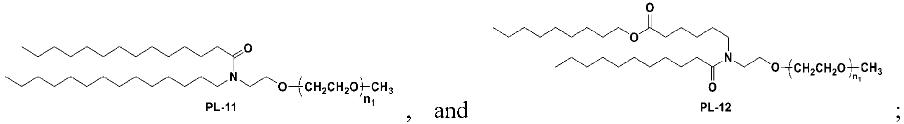 wherein, ni is an integer of 25 to 300, and is more preferably selected from the group consisting of 44, 45, 46, 47, and 48;
or the other cationic lipid in the lipid composition is selected from the group consisting of 1,2-dioleoyl-3-trimethylammonium-propane, 1,2-bisoctadecoxy-3-methylammonium propane chloride, 1-[2-(oleoyloxy)ethyl]-2-oleo-3-(2-hydroxyethyl)imidazoline chloride, 1,2-diolecanol-3-dimethylamino-propane, 2,3-bis(tetradecanoyloxy)propyltrimonium chloride, didecyldimonium chloride, bisdecyldimonium bromide, *N*,*N*-diole-*N*,*N*-dimethylammonium chloride, *N*-(4-carboxybenzyl)-*N,N*-dimethyl-2,3-bis(oleoyloxy)propyl-1-ammonium, 3,6-bis{4-[bis(2-hydroxydodecyl)amino]butyl}piperazine-2,5-dione, 1'-((2-(4-(2-(2-(2-(bis(2-hydroxydodecyl)amino)ethyl)(2-hydroxydodecyl)amino)ethyl)pipera zine-1-yl)ethyl)azanediyl)bis(dodecane-2-ol), 4-(*N,N*-dimethylamino)butyric acid(dilinole)methyl ester, 1,2-dimyristoyl-sn-glycero-3-ethylphosphocholine, ((4-hydroxybutyl) azadiyl)bis(hexane-6,1-diyl)bis(2-hexyldecanoate), 8-[(2-hydroxyethyl)(6-oxo-6-(undecoxy)hexyl)amino]caprylic acid (hepta-9-yl) ester, ((2-(2-hydroxyethoxy)ethyl)azadialkyl)bis(hexane-6,1-diyl)bis(2-hexyldecanoate), and combinations thereof;
or the anionic lipid in the lipid composition is selected from the group consisting of 1,2-dioleoyl-sn-glycerol-3-phosphate sodium salt, 1,2-dimyristoyl-sn-glycerol-3-phosphate sodium salt, bis(monoolein) phosphatidylmonomethylethanolamine, cardiolipin, and combinations thereof.

13. The lipid composition according to any one of claims **11** to **12,** wherein the lipid composition comprises 20-80% cationic lipids, 5-16% phospholipids, 25-55% steroid lipids and 0.5-10% PEGylated lipids; the percentages are molar percentages of each lipid with respect to the total lipids in the solvent-comprising solution.

14. The lipid composition according to claim **13,** wherein the molar percentage of cationic lipid in the total lipids in the solvent-comprising solution is 30-65%, more preferably 35%, 40%, 45%, 46%, 47%, 48%, 49%, 50%, or 55%;
or the molar percentage of phospholipid in the total lipids in the solvent-comprising solution is 7.5-16%, more preferably 8%, 9%, 10%, 11%, 12%, or 16%;
or the molar percentage of steroid lipid in the total lipids in the solvent-comprising solution is 35-50%, more preferably 40%, 41%, 42%, 43%, 44%, 45%, 46%, 47%, 48%, 49%, or 50%;
or the molar percentage of PEGylated lipid in the total lipids in the solvent-comprising solution is 0.5-5%, preferably 1-3%, more preferably 1.5%, 1.6%, 1.7%, 1.8%, 1.9%, 2.0%, or 2.5%.

15. A lipid pharmaceutical composition comprising a lipid composition of any one of claims **10** to **14** and a drug, wherein the drug is selected from the group consisting of a nucleic acid drug, a gene vaccine, an antitumor drug, a small molecule drug, a peptide drug, and a protein drug.

16. The lipid pharmaceutical composition according to claim **15,** wherein the nucleic acid drug is selected from the group consisting of RNA, DNA, antisense nucleic acid, plasmid, interfering nucleic acid, aptamer, antagomir, and ribozyme; the RNA is selected from the group consisting of mRNA, saRNA, circRNA, miRNA, and siRNA; the nucleic acid drug is preferably selected from the group consisting of DNA, mRNA, miRNA, and siRNA.

17. A lipid pharmaceutical composition according to any one of claims **15** to **16,** wherein the lipid pharmaceutical composition is used as a drug, selected from any of the following drugs: an antineoplastic agent, an antiviral agent, an antifungal agent, and a vaccine.

18. A formulation of lipid pharmaceutical composition comprising any lipid pharmaceutical composition of any one of claims **15** to **17** and a pharmaceutically acceptable diluent or excipient; the diluent or excipient is preferably deionized water, ultrapure water, phosphate buffer or normal saline, more preferably phosphate buffer or normal saline, and most preferably normal saline.

19. A liposome or lipid nanoparticle comprising a lipid composition of any one of claims **10** to **14.**

20. The liposome or lipid nanoparticle according to claim **19,** wherein the lipid nanoparticle is an LNP-pharmaceutical composition, an LPP-pharmaceutical composition, or a PNP-pharmaceutical composition, preferably an LNP-pharmaceutical composition, more preferably an LNP-nucleic acid pharmaceutical composition, more preferably an LNP-mRNA pharmaceutical composition.
